(19) Europäisches Patentamt
European Patent Office
Office européen des brevets

(11) EP 2 633 868 A1

(12) EUROPEAN PATENT APPLICATION

(43) Date of publication:
**04.09.2013 Bulletin 2013/36**

(51) Int Cl.:
***A61K 45/06*** *(2006.01)*

(21) Application number: **13167089.5**

(22) Date of filing: **13.02.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **13.02.2008 US 28282**

(62) Document number(s) of the earlier application(s) in accordance with Art. 76 EPC:
**09709577.2 / 2 254 598**

(71) Applicant: **Targacept, Inc.**
**100 North Main Street**
**Suite 1510**
**Winston-Salem, NC 27101 (US)**

(72) Inventors:
- **Bencherif, Merouane**
  **Winston-Salem, NC 27106 (US)**
- **Gatto, Gregory J.**
  **Winston-Salem, NC 27103 (US)**
- **Hauser, Terry**
  **Winston-Salem, NC 27103 (US)**
- **Jordan, Kristen G.**
  **Clemmons, NC 27012 (US)**
- **Letchworth, Sharon R.**
  **Kernersville, NC 27284 (US)**

(74) Representative: **Crowhurst, Charlotte Waveney**
**Potter Clarkson LLP**
**The Belgrave Centre**
**Talbot Street**
**Nottingham NG1 5GG (GB)**

Remarks:
This application was filed on 08-05-2013 as a divisional application to the application mentioned under INID code 62.

# (54) COMBINATION OF ALPHA 7 NICOTINIC AGONISTS AND ANTIPSYCHOTICS

(57) The present invention relates to a synergistic combination of an alpha7 (a7) nicotinic agonist and an antipsychotic agent.

EP 2 633 868 A1

## Description

### FIELD OF THE INVENTION

The present invention relates to a combination of an alpha7 (α7) nicotinic agonist and an antipsychotic agent. The invention further relates to pharmaceutical compositions comprising such a combination and to methods of treating psychiatric disorders, particularly psychotic disorders, by administrating said combination. The invention further relates to a kit comprising the combination and use of said kit in treatment of psychiatric disorders, particularly psychotic disorders.

### BACKGROUND OF THE INVENTION

The neuronal nicotinic receptors (NNRs) characteristic of the central nervous system (CNS) have been shown to occur in several subtypes, the most common of which are the α4β2 and α7 subtypes. See, for example, Schmitt, Current Med. Chem. 7: 749 (2000). Ligands that interact with the α7 NNR subtype have been proposed to be useful in the treatment of a variety of conditions and disorders (see Mazurov et al., Curr. Med. Chem. 13: 1567-1584 (2006) and references therein). Prominent among those conditions and disorders are cognitive impairment, schizophrenia, inflammation, angiogenesis, neuropathic pain and fibromyalgia.

Various compounds have been reported to interact with α7 NNRs and have been proposed as therapies on that basis. See, for instance, PCT WO 99/62505, PCT WO 99/03859, PCT WO 97/30998, PCT WO 01/36417, PCT WO 02/15662, PCT WO 02/16355, PCT WO 02/16356, PCT WO 02/16357, PCT WO 02/16358, PCT WO 02/17358, Stevens et al., Psychopharm. 136: 320 (1998), Dolle et al., J. Labelled Comp. Radiopharm. 44: 785 (2001) and Macor et al., Bioorg. Med. Chem. Lett. 11: 319 (2001) and references therein, each of which is incorporated by reference with regard to such background teaching. Among these compounds, a common structural theme is that of the substituted tertiary bicyclic amine (e.g., quinuclidine). Similar substituted quinuclidine compounds have also been reported to bind at muscarinic receptors. See, for instance, U.S. Patent Nos. 5,712,270 to Sabb and PCTs, WO 02/00652 and WO 02/051841, each of which is incorporated by reference with regard to such teaching.

A limitation of some nicotinic compounds is that they are associated with various undesirable side effects, for example, by stimulating muscle and ganglionic receptors. It would thus be desirable to have compounds, compositions and methods for preventing and/or treating various conditions or disorders (e.g., CNS disorders), including alleviating the symptoms of these disorders, where the compounds exhibit nicotinic pharmacology with a beneficial effect (e.g., upon the functioning of the CNS), but without significant associated side effects. It would further be highly desirable to provide compounds, compositions and methods that affect CNS function without significantly affecting those nicotinic receptor subtypes which have the potential to induce undesirable side effects (e.g., appreciable activity at cardiovascular and skeletal muscle sites). In addition, it would be highly desirable to provide pharmaceutical compositions and methods incorporating a compound which interacts with nicotinic receptors but not muscarinic receptors, as the latter are associated with side effects, such as hypersalivation, sweating, tremors, cardiovascular and gastrointestinal disturbances, related to the function of the parasympathetic nervous system (see Caulfield, Pharmacol. Ther. 58: 319 (1993) and Broadley and Kelly, Molecules 6: 142 (2001), incorporated herein by reference with regard to such teaching). In addition, it would be highly desirable to provide pharmaceutical compositions and methods incorporating a compound which interacts with nicotinic receptors but not 5-hydroxytryptamine ($5HT_3$) receptors, as cross-reactivity between these two receptor types has limited the therapeutic index of some other nicotinic ligands. The present invention provides compositions and methods which incorporate such highly selective nicotinic compounds.

Schizophrenia is an example of a psychotic disorder that is particularly amenable to treatment by modulating the α7 NNR subtype. There are a decreased number of hippocampal NNRs in postmortem brain tissue of schizophrenic patients. Also, there is improved psychological effect in smoking versus non-smoking schizophrenic patients. Nicotine improves sensory gating deficits in animals and schizophrenics. Blockade of the α7 NNR subtype induces a gating deficit similar to that seen in schizophrenia. See, for example, Leonard et al., Schizophrenia Bulletin 22(3): 431 (1996), herein incorporated by reference with regard to such teaching. Biochemical, molecular, and genetic studies of sensory processing, in patients with the P50 auditory-evoked potential gating deficit, suggest that the α7 NNR subtype may function in an inhibitory neuronal pathway. See, for example, Freedman et al., Biological Psychiatry 38(1): 22 (1995), herein incorporated by reference with regard to such teaching.

Antipsychotics have long been used in the treatment of psychotic disorders (schizophrenia, for example), as well as other psychiatric disorders. Examples of conventional antipsychotics include but are not limited to chlorpromazine, haloperidol, flupenthixol, and perphenazine. Examples of atypical antipsychotics include but are not limited to clozapine, risperidone, olanzapine, quetiapine, aripiprazole, ziprasidone, amisulpride, sulpride, zotepine, sertindole, paliperidone, bifeprunox, and asenapine. Atypical antipsychotics offer several clinical benefits over the conventional antipsychotics. Exemplary distinct advantages over traditional antipsychotic medications include greater improvement in negative symptoms, such as social withdrawal, and lower risk of Parkinsonian side effects and tardive dyskinesia.

SUMMARY OF THE INVENTION

The present invention provides a pharmaceutical combination comprising at least one α7 nicotinic agonist and at least one antipsychotic agent, wherein the combination provides synergistic therapy for psychiatric disorders.
In one embodiment, the psychiatric disorder is a psychotic disorder. In a further embodiment, the psychiatric disorder is schizophrenia.
In one embodiment the pharmaceutical combination of the present invention provides synergistic therapy for one or more of attention disorders, information processing, memory disorders, or deficits in executive function.
In one embodiment the pharmaceutical combination of the present invention includes the at least one α7 nicotinic agonist and the at least one antipsychotic agent that are provided simultaneously, sequentially, or separately.
In one embodiment, the at least one α7 nicotinic agonist is a compound of Formula 1:

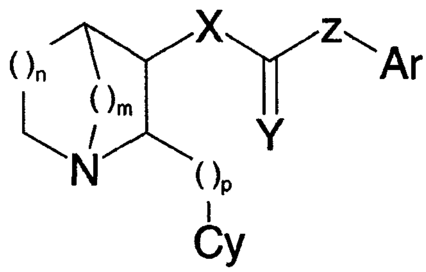

Formula 1

or a pharmaceutically acceptable salt or solvate thereof,
wherein

m is 1 or 2;
n is 1 or 2;
p is 1, 2, 3, or 4;
X is oxygen or -NR'-;
Y is oxygen or sulfur;
Z is -NR'-, a covalent bond, or a linker species, A;
A is -CR'R"-, -CR'R"-CR'R"-, -CR'=CR'-, or -C≡C-,
wherein each R' and R" individually are hydrogen, $C_1$-$C_8$ alkyl, $C_3$-$C_8$ cycloalkyl, heterocyclyl, aryl, or arylalkyl, or $R^I$ and $R^{II}$ may combine with the atom to which they are attached to form a 3 to 8 membered ring, which may contain additional heteroatoms;
when Z is a covalent bond or A, then X must be nitrogen;
Ar is an optionally substituted aryl group; and
Cy is an optionally substituted 5- or 6-membered heteroaromatic ring,
where each of Ar and Cy may be substituted with one or more of $C_1$-$C_8$ alkyl, $C_2$-$C_8$ alkenyl, heterocyclyl, $C_3$-$C_8$ cycloalkyl, aryl, substituted aryl, arylalkyl, substituted arylalkyl, halogen, -OR', -NR'R", haloalkyl, -CN, $-NO_2$, -C≡CR', -SR', $-N_3$, -C(=O)NR'R", -NR'C(=O) R", -C(=O)R', -C(=O)OR', -OC(=O)R', $-O(CR'R")_rC(=O)R'$, $-O(CR'R")_rNR"C(=O)R'$, - $O(CR'R")_rNR"SO_2R'$, -OC(=O)NR'R", -NR'C(=O)O R", $-SO_2R'$, $-SO_2NR'R"$, or $-NR'SO_2R"$,
where each of R' and R" are as defined, and
r is an integer from 1 to 6,
wherein substituted aryl and substituted arylalkyl groups have one or more of $C_1$-$C_8$ alkyl, $C_2$-$C_8$ alkenyl, heterocyclyl, $C_3$-$C_8$ cycloalkyl, aryl, arylalkyl, halogen, -OR', -NR'R", haloalkyl, -CN, $-NO_2$, -C≡CR', -SR', $-N_3$, -C(=O)NR'R", -NR'C(=O) R", -C(=O)R', -C(=O)OR', -OC(=O)R', $-O(CR'R")_rC(=O)R'$, $-O(CR'R")_rNR"C(=O)R'$, $-O(CR'R")_rNR"SO_2R'$, -OC(=O)NR'R", -NR'C(=O)O R", $-SO_2R'$, $-SO_2NR'R"$, or $-NR'SO_2R"$,
where each of R', R", and r are as defined.

In one embodiment, p is 1, Cy is 3-pyridinyl or 5-pyrimidinyl, each of X and Y are oxygen, and Z is -NR'-.
In one embodiment, p is 1, Cy is 3-pyridinyl or 5-pyrimidinyl, X and Z are $-NR^I-$, and Y is oxygen.
In one embodiment, p is 1, Cy is 3-pyridinyl or 5-pyrimidinyl, X is $-NR^I-$, Y is oxygen, and Z is a covalent bond.
In one embodiment, p is 1, Cy is 3-pyridinyl or 5-pyrimidinyl, X is $-NR^I-$, Y is oxygen, and Z is A.
In one embodiment, the compound of Formula 1 is:

2-((3-pyridinyl)methyl)-1-azabicyclo[2.2.2]oct-3-yl N-phenylcarbamate,
2-((3-pyridinyl)methyl)-1-azabicyclo[2.2.2]oct-3-yl N-(4-fluorophenyl)carbamate,
2-((3-pyridinyl)methyl)-1-azabicyclo[2.2.2]oct-3-yl N-(4-chlorophenyl)carbamate,
2-((3-pyridinyl)methyl)-1-azabicyclo[2.2.2]oct-3-yl N-(4-bromophenyl)carbamate,
2-((3-pyridinyl)methyl)-1-azabicyclo[2.2.2]oct-3-yl N-(3-fluorophenyl)carbamate,
2-((3-pyridinyl)methyl)-1-azabicyclo[2.2.2]oct-3-yl N-(3-chlorophenyl)carbamate,
2-((3-pyridinyl)methyl)-1-azabicyclo[2.2.2]oct-3-yl N-(3-bromophenyl)carbamate,
2-((3-pyridinyl)methyl)-1-azabicyclo[2.2.2]oct-3-yl N-(2-fluorophenyl)carbamate,
2-((3-pyridinyl)methyl)-1-azabicyclo[2.2.2]oct-3-yl N-(2-chlorophenyl)carbamate,
2-((3-pyridinyl)methyl)-1-azabicyclo[2.2.2]oct-3-yl N-(2-bromophenyl)carbamate,
2-((3-pyridinyl)methyl)-1-azabicyclo[2.2.2]oct-3-yl N-(3,4-dichlorophenyl)carbamate,
2-((3-pyridinyl)methyl)-1-azabicyclo[2.2.2]oct-3-yl N-(2-methylphenyl)carbamate,
2-((3-pyridinyl)methyl)-1-azabicyclo[2.2.2]oct-3-yl N-(2-biphenyl)carbamate,
2-((3-pyridinyl)methyl)-1-azabicyclo[2.2.2]oct-3-yl N-(3-methylphenyl)carbamate,
2-((3-pyridinyl)methyl)-1-azablcyclo[2.2.2]oct-3-yl N-(3-biphenyl)carbamate,
2-((3-pyridinyl)methyl)-1-azabicyclo[2.2.2]oct-3-yl N-(4-methylphenyl)carbamate,
2-((3-pyridinyi)methyl)-1-azabicyclo[2.2.2]oct-3-yl N-(4-biphenyl)carbamate,
2-((3-pyridinyl)methyl)-1-azabicyclo[2.2.2]oct-3-yl N-(2-cyanophenyl)carbamate,
2-((3-pyridinyl)methyl)-1-azabicyclo[2.2.2]oct-3-yl N-(3-cyanophenyl)carbamate,
2-((3-pyridinyl)methyl)-1-azabicyclo[2.2.2]oct-3-yl N-(4-cyanophenyl)carbamate,
2-((3-pyridinyl)methyl)-1-azabicyclo[2.2.2]oct-3-yl N-(3-trifluoromethylphenyl)carbamate,
2-((3-pyridinyl)methyl)-1-azabicyclo[2.2.2]oct-3-yl N-(4-dimethylaminophenyl)carbamate,
2-((3-pyridinyl)methyl)-1-azabicyclo[2.2.2]oct-3-yl N-(2-methoxyphenyl)carbamate,
2-((3-pyridinyl)methyl)-1-azabicyclo[2.2.2]oct-3-yl N-(2-phenoxyphenyl)carbamate,
2-((3-pyridinyl)methyl)-1-azabicyclo[2.2.2]oct-3-yl N-(2-methylthiophenyl)carbamate,
2-((3-pyridinyl)methyl)-1-azabicyclo[2.2.2]oct-3-yl N-(2-phenylthiophenyl)carbamate,
2-((3-pyridinyl)methyl)-1-azabicyclo[2.2.2]oct-3-yl N-(3-methoxyphenyl)carbamate,
2-((3-pyridinyl)methyl)-1-azabicyclo[2.2.2]oct-3-yl N-(3-phenoxyphenyl)carbamate,
2-((3-pyridinyl)methyl)-1-azabicyclo[2.2.2]oct-3-yl N-(3-methylthiophenyl)carbamate,
2-((3-pyridinyl)methyl)-1-azabicyclo[2.2.2]oct-3-yl N-(3-phenylthiophenyl)carbamate,
2-((3-pyridinyl)methyl)-1-azabicyclo[2.2.2]oct-3-yl N-(4-methoxyphenyl)carbamate,
2-((3-pyridinyl)methyl)-1-azabicyclo[2.2.2]oct-3-yl N-(4-phenoxyphenyl)carbamate,
2-((3-pyridinyl)methyl)-1-azabicyclo[2.2.2]oct-3-yl N-(4-methylthiophenyl)carbamate,
2-((3-pyridinyl)methyl)-1-azabicyclo[2.2.2]oct-3-yl N-(4-phenylthiophenyl)carbamate,
2-((3-pyridinyl)methyl)-1-azabicyclo[2.2.2]oct-3-yl N-(2,4-dimethoxyphenyl)carbamate,
2-((3-pyridinyl)methyl)-1-azabicyclo[2.2.2]oct-3-yl N-(2-thienyl)carbamate,
2-((3-pyridinyl)methyl)-1-azabicyclo[2.2.2]oct-3-yl N-(3-thienyl)carbamate,
2-((3-pyridinyl)methyl)-1-azabicyclo[2.2.2]oct-3-yl N-(3-benzothienyl)carbamate,
2-((3-pyridinyl)methyl)-1-azabicyclo[2.2.2]oct-3-yl N-(1-naphthyl)carbamate,
2-((3-pyridinyl)methyl)-1-azabicyclo[2.2.2]oct-3-yl N-(2-naphthyl)carbamate,
N-phenyl-N'-(2-((3-pyridinyl)methyl)-1-azabicyclo[2.2.2]oct-3-yl)urea,
N-(4-fluorophenyl)-N'-(2-((3-pyridinyl)methyl)-1-azabicyclo[2.2.2]oct-3-yl)urea,
N-(4-chlorophenyl)-N'-(2-((3-pyridinyl)methyl)-1-azabicyclo[2.2.2]oct-3-yl)urea
N-(4-bromophenyl)-N'-(2-((3-pyridinyl)methyl)-1-azabicyclo[2.2.2]oct-3-yl)urea,
N-(3-fluorophenyl)-N'-(2-((3-pyridinyl)methyl)-1-azabicyclo[2.2.2]oct-3-yl)urea,
N-(3-chlorophenyl)-N'-(2-((3-pyridinyl)methyl)-1-azabicyclo[2.2.2]oct-3-yl)urea,
N-(3-bromophenyl)-N'-(2-((3-pyridinyl)methyl)-1-azabicyclo[2.2.2]oct-3-yl)urea,
N-(2-fluorophenyl)-N'-(2-((3-pyridinyl)methyl)-1-azabicyclo[2.2.2]oct-3-yl)urea,
N-(2-chlorophenyl)-N'-(2-((3-pyridinyl)methyl)-1-azabicyclo[2.2.2]oct-3-yl)urea,
N-(2-bromophenyl)-N'-(2-((3-pyridinyl)methyl)-1-azabicyclo[2.2.2]oct-3-yl)urea,
N-(3,4-dichlorophenyl)-N'-(2-((3-pyridinyl)methyl)-1-azabicyclo[2.2.2]oct-3-yl)urea,
N-(2-methylphenyl)-N'-(2-((3-pyridinyl)methyl)-1-azabicyclo[2.2.2]oct-3-yl)urea,
N-(2-biphenyl)-N'-(2-((3-pyridinyl)methyl)-1-azabicyclo[2.2.2]oct-3-yl)urea,
N-(3-methylphenyl)-N'-(2-((3-pyridinyl)methyl)-1-azabicyclo[2.2.2]oct-3-yl)urea,
N-(3-biphenyl)-N'-(2-((3-pyridinyl)methyl)-1-azabicyclo[2.2.2]oct-3-yl)urea,
N-(4-methylphenyl)-N'-(2-((3-pyridinyl)methyl)-1-azabicyclo[2.2.2]oct-3-yl)urea,
N-(4-biphenyl)-N'-(2-((3-pyridinyl)methyl)-1-azabicyclo[2.2.2]oct-3-yl)urea,
N-(2-cyanophenyl)-N'-(2-((3-pyridinyl)methyl)-1-azabicyclo[2.2.2]oct-3-yl)urea,

N-(3-cyanophenyl)-N'-(2-((3-pyridinyl)methyl)-1-azabicyclo[2.2.2]oct-3-yl)urea,
N-(4-cyanophenyl)-N'-(2-((3-pyridinyl)methyl)-1-azabicyclo[2.2.2]oct-3-yl)urea,
N-(3-trifluoromethylphenyl)-N'-(2-((3-pyridinyl)methyl)-1-azabicyclo[2.2.2]oct-3-yl)urea,
N-(4-dimethylaminophenyl)-N'-(2-((3-pyridinyl)methyl)-1-azabicyclo[2.2.2]oct-3-yl)urea,
N-(2-methoxyphenyl)-N'-(2-((3-pyridinyl)methyl)-1-azabicyclo[2.2.2]oct-3-yl)urea,
N-(2-phenoxyphenyl)-N'-(2-((3-pyridinyl)methyl)-1-azabicyclo[2.2.2]oct-3-yl)urea,
N-(2-methylthiophenyl)-N'-(2-((3-pyridinyl)methyl)-1-azabicyclo[2.2.2]oct-3-yl)urea,
N-(2-phenylthiophenyl)-N'-(2-((3-pyridinyl)methyl)-1-azabicyclo[2.2.2]oct-3-yl)urea,
N-(3-methoxyphenyl)-N'-(2-((3-pyridinyl)methyl)-1-azabicyclo[2.2.2]oct-3-yl)urea,
N-(3-phenoxyphenyl)-N'-(2-((3-pyridinyl)methyl)-1-azabicyclo[2.2.2]oct-3-yl)urea,
N-(3-methylthiophenyl)-N'-(2-((3-pyridinyl)methyl)-1-azabicyclo[2.2.2]oct-3-yl)urea,
N-(3-phenylthiophenyl)-N'-(2-((3-pyridinyl)methyl)-1-azabicyclo[22.2]oct-3-yl)urea,
N-(4-methoxyphenyl)-N'-(2-((3-pyridinyl)methyl)-1-azabicyclo[2.2.2]oct-3-yl)urea,
N-(4-phenoxyphenyl)-N'-(2-((3-pyridinyl)methyl)-1-azabicyclo[2.2.2]oct-3-yl)urea,
N-(4-methylthiophenyl)-N'-(2-((3-pyridinyl)methyl)-1-azabicyclo[2.2.2]oct-3-yl)urea,
N-(4-phenylthiophenyl)-N'-(2-((3-pyridinyl)methyl)-1-azabicyclo[2.2.2]oct-3-yl)urea,
N-(2,4-dimethoxyphenyl)-N'-(2-((3-pyridinyl)methyl)-1-azabicyclo[2.2.2]oct-3-yl)urea,
N-(2-thienyl)-N'-(2-((3-pyridinyl)methyl)-1-azabicyclo[2.2.2]oct-3-yl)urea,
N-(3-thienyl)-N'-(2-((3-pyridinyl)methyl)-1-azabicyclo[2.2.2]oct-3-yl)urea,
N-(3-benzothienyl)-N'-(2-((3-pyridinyl)methyl)-1-azabicyclo[2.2.2]oct-3-yl)urea,
N-(1-naphthyl)-N'-(2-((3-pyridinyl)methyl)-1-azabicyclo[2.2.2]oct-3-yl)urea,
N-(2-naphthyl)-N'-(2-((3-pyridinyl)methyl)-1-azabicyclo[2.2.2]oct-3-yl)urea,
N-(2-((3-pyridinyl)methyl)-1-azabicyclo[2.2.2]oct-3-yl)benzamide,
N-(2-((3-pyridinyl)methyl)-1-azabicyclo[2.2.2]oct-3-yl)-2-fluorobenzamide,
N-(2-((3-pyridinyl)methyl)-1-azabicyclo[2.22]oct-3-yl)-3-fluorobenzamide,
N-(2-((3-pyridinyl)methyl)-1-azabicyclo[22.2]oct-3-yl)-4-fluorobenzamide,
N-(2-((3-pyridinyl)methyl)-1-azabicyclo[2.2.2]oct-3-yl)-2-chlorobenzamide,
N-(2-((3-pyridinyl)methyl)-1-azabicyclo[2.2.2]oct-3-yl)-3-chlorobenzamide,
N-(2-((3-pyridinyl)methyl)-1-azabicyclo[2.2.2]oct-3-yl)-4-chlorobenzamide,
N-(2-((3-pyridinyl)methyl)-1-azabicyclo[2.2.2]oct-3-yl)-2-bromobenzamide,
N-(2-((3-pyridinyl)methyl)-1-azabicyclo[2.2.2]oct-3-yl)-3-bromobenzamide,
N-(2-((3-pyridinyl)methyl)-1-azabicyclo[2.2.2]oct-3-yl)-4-bromobenzamide,
N-(2-((3-pyridinyl)methyl)-1-azabicyclo[2.2.2]oct-3-yl)-3,4-dichlorobenzamide,
N-(2-((3-pyridinyl)methyl)-1-azabicyclo[2.2.2]oct-3-yl)-2-methylbenzamide,
N-(2-((3-pyridinyl)methy))-1-azabicyclo[2.2.2]oct-3-yl)-3-methylbenzamide,
N-(2-((3-pyridinyl)methyl)-1-azabicyclo[2.2.2]oct-3-yl)-4-methylbenzamide,
N-(2-((3-pyridinyl)methyl)-1-azabicyclo[2.2.2]oct-3-yl)-2-phenylbenzamide,
N-(2-((3-pyridinyl)methyl)-1-azabicycto[2.2.2]oct-3-yl)-3-phenylbenzamide,
N-(2-((3-pyridinyl)methyl)-1-azabicyclo[2.2.2]oct-3-yl)-4-phenylbenzamide,
N-(2-((3-pyridinyl)methyl)-1-azabicyclo[2.2.2]oct-3-yl)-2-cyanobenzamide,
N-(2-((3-pyridinyl)methyl)-1-azabicyclo[2.2.2]oct-3-yl)-3-cyanobenzamide,
N-(2-((3-pyridinyl)methyl)-1-azabicyclo[2.2.2]oct-3-yl)-4-cyanobenzamide,
N-(2-((3-pyridinyl)methyl)-1-azabicyclo[2.2.2]oct-3-yl)-3-trifluoromethylbenzamide,
N-(2-((3-pyridinyl)methyl)-1-azabicyclo[2.2.2]oct-3-yl)-4-dimethylaminobenzamide,
N-(2-((3-pyridinyl)methyl)-1-azabicyclo[2.2.2]oct-3-yl)-2-methoxybenzamide,
N-(2-((3-pyridinyl)methyl)-1-azabicyclo[2.2.2]oct-3-yl)-3-methoxybenzamide,
N-(2-((3-pyridinyl)methyl)-1-azabicyclo[2.2.2]oct-3-yl)-4-methoxybenzamide,
N-(2-((3-pyridinyl)methyl)-1-azabicyclo[2.2.2]oct-3-yl)-2-phenoxybenzamide,
N-(2-((3-pyridinyl)methyl)-1-azabicyclo[2.2.2]oct-3-yl)-3-phenoxybenzamide,
N-(2-((3-pyridinyl)methyl)-1-azabicyclo[2.2.2]oct-3-yl)-4-phenoxybenzamide,
N-(2-((3-pyridinyl)methyl)-1-azabicyclo[2.2.2]oct-3-yl)-2-methylthiobenzamide,
N-(2-((3-pyridinyl)methyl)-1-azabicyclo[2.2.2]oct-3-yl)-3-methylthiobenzamide,
N-(2-((3-pyridinyl)methyl)-1-azabicyclo[2.2.2]oct-3-yl)-4-methylthiobenzamide,
N-(2-((3-pyridinyl)methyl)-1-azabicyclo[2.2.2]oct-3-yl)-2-phenylthiobenzamide,
N-(2-((3-pyridinyl)methyl)-1-azabicyclo[2.2.2]oct-3-yl)-3-phenylthiobenzamide,
N-(2-((3-pyridinyl)methyl)-1-azabicyclo[2.2.2]oct-3-yl)-4-phenylthiobenzamide,
N-(2-((3-pyridinyl)methyl)-1-azabicyclo[2.2.2]oct-3-yl)-2,4-dimethoxybenzamide,
N-(2-((3-pyridinyl)methyl)-1-azabicyclo[2.2.2]oct-3-yl)-5-bromonicotinamide,

N-(2-((3-pyridinyl)methyl)-1-azabicyclo[2.2.2]oct-3-yl)-6-chloronicotinamide,
N-(2-((3-pyridinyl)methyl)-1-azabicyclo[2.2.2]oct-3-yl)-5-phenylnicotinamide,
N-(2-((3-pyridinyl)methyl)-1-azabicyclo[2.2.2]oct-3-yl)furan-2-carboxamide,
N-(2-((3-pyridinyl)methyl)-1-azabicyclo[2.2.2]oct-3-yl)furan-3-carboxamide,
N-(2-((3-pyridinyl)methyl)-1-azabicyclo[2.2.2]oct-3-yl)thiophene-2-carboxamide,
N-(2-((3-pyridinyl)methyl)-1-azabicyclo[2.2.2]oct-3-yl)-5-bromothiophene-2-carboxamide,
N-(2-((3-pyridinyl)methyl)-1-azabicyclo[2.2.2]oct-3-yl)-5-methylthiothiophene-2-carboxamide,
N-(2-((3-pyridinyl)methyl)-1-azabicyclo[2.22]oct-3-yl)-5-phenylthiothiophene-2-carboxamide,
N-(2-((3-pyridinyl)methyl)-1-azabicyclo[2.2.2]oct-3-yl)-5-methylthiophene-2-carboxamide,
N-(2-((3-pyridinyl)methyl)-1-azabicyclo[2.2.2]oct-3-yl)-3-methylthiophene-2-carboxamide,
N-(2-((3-pyridinyl)methyl)-1-azabicyclo[2.2.2]oct-3-yl)-3-bromothiophene-2-carboxamide,
N-(2-((3-pyridinyl)methyl)-1-azabicyclo[2.2.2]oct-3-yi)-3-chlorothiophene-2-carboxamide,
N-(2-((3-pyridinyl)methyl)-1-azabicyclo[2.2.2]oct-3-yl)-5-(2-pyridinyl)thiophene-2-carboxamide,
N-(2-((3-pyridinyl)methyl)-1-azabicyclo[2.2.2]oct-3-yl)-5-acetylthiophene-2-carboxamide,
N-(2-((3-pyridinyl)methyl)-1-azabicyclo[2.2.2]oct-3-yl)-3-ethoxythiophene-2-carboxamide,
N-(2-((3-pyridinyl)methyl)-1-azabicyclo[2.2.2]oct-3-yl)-3-methoxythiophene-2-carboxamide,
N-(2-((3-pyridinyl)methyl)-1-azabicyclo[2.2.2]oct-3-yl)-4-acetyl-3-methyl-5-methylthiothiophene-2-carboxamide,
N-(2-((3-pyridinyl)methyl)-1-azabicyclo[2.2.2]oct-3-yl)thiophene-3-carboxamide,
N-(2-((3-pyridinyl)methyl)-1-azabicyclo[2.2.2]oct-3-yl)-1-methylpyrrole-2-carboxamide,
N-(2-((3-pyridinyl)methyl)-1-azabicyclo[2.2.2]oct-3-yl)pyrrole-3-carboxamide,
N-(2-((3-pyridinyl)methyl)-1-azabicyclo[2.2.2]oct-3-yl)indole-2-carboxamide,
N-(2-((3-pyridinyl)methyl)-1-azabicyclo[2.2.2]oct-3-yl)indole-3-carboxamide,
N-(2-((3-pyridinyl)methyl)-1-azabicyclo[2.2.2]oct-3-yl)-1-methylindole-3-carboxamide,
N-(2-((3-pyridinyl)methyl)-1-azabicyclo[2.2.2]oct-3-yl)-1-benzylindole-3-carboxamide,
N-(2-((3-pyridinyl)methyl)-1-azabicyclo[2.2.2]oct-3-yl)-1H-benzimidazole-2-carboxamide,
N-(2-((3-pyridinyl)methyl)-1-azabicyclo[2.2.2]oct-3-yl)-1-isopropyl-2-trifluoromethyl-1 H-benzimidazole-5-carboxamide,
N-(2-((3-pyridinyl)methyl)-1-azabicyclo[222]oct-3-yl)-1-isopropyl-1 H-benzotriazole-5-carboxamide,
N-(2-((3-pyridinyl)methyl)-1-azabicyclo[2.2.2]oct-3-yl)benzo[b]thiophene-2-carboxamide,
N-(2-((3-pyndiny))methyt)-1-azabicyclo[2.2.2]oct-3-yl)benzo[b]thiophene-3-carboxamide,
N-(2-((3-pyridinyl)methyl)-1-azabicyclo[2.2.2]oct-3-yl)benzofuran-2-carboxamide,
N-(2-((3-pyridinyl)methyl)-1-azabicyclo[2.2.2]oct-3-yl)benzofuran-3-carboxamide,
N-(2-((3-pyridinyl)methyl-1-azabicyclo[2.2.2]oct-3-yl)-3-methylbenzofuran-2-carboxamide,
N-(2-((3-pyridinyl)methyl-1-azabicyclo[2.2.2]oct-3-yl)-5-nitrobenzofuran-2-carboxamide,
N-(2-((3-pyridinyl)methyl-1-azabicyclo[2.2.2]oct-3-yl)-5-methoxybenzofuran-2-carboxamide,
N-(2-((3-pyridinyl)methyl-1-azabicyclo[2.2.2]oct-3-yl)-7-methoxybenzofuran-2-carboxamide,
N-(2-((3-pyridinyl)methyl-1-azabicyclo[2.2.2]oct-3-yl)-7-ethoxybenzofuran-2-carboxamide,
N-(2-((3-pyridinyl)methyl-1-azabicyclo[2.2.2]oct-3-yl)-3-methyl-5-chlorobenzofuran-2-carboxamide,
N-(2-((3-pyridinyl)methyl-1-azabicyclo[2.2.2]oct-3-yl)-6-bromobenzofuran-2-carboxamide,
N-(2-((3-pyridinyl)methyl-1-azabicyclo[2.2.2]oct-3-yl)-4-acetyl-7-methoxybenzofuran-2-carboxamide,
N-(2-((3-pyridinyl)methyl-1-azabicyclo[2.2.2]oct-3-yl)-2-methylbenzofuran-4-carboxamide,
N-(2-((3-pyridinyl)methyl-1-azabicyclo[2.2.2]oct-3-yl)naphtho[2,1-b]furan-2-carboxamide,
N-(2-((3-pyridinyl)methyl)-1-azabicyclo[2.2.2]oct-3-yl)naphthalene-1-carboxamide,
N-(2-((3-pyridinyl)methyl)-1-azabicyclo[2.2.2]oct-3-yl)naphthalene-2-carboxamide,
N-(2-((3-pyridinyl)methyl)-1-azabicyclo[2.2.2]oct-3-yl)-6-aminonaphthalene-2-carboxamide,
N-(2-((3-pyridinyl)methyl-1-azabicyclo[2.2.2]oct-3-yl)-3-methoxynaphthalene-2-carboxamide,
N-(2-((3-pyridinyl)methyl-1-azabicyclo[2.2.2]oct-3-yl)-6-methoxynaphthalene-2-carboxamide,
N-(2-((3-pyridinyl)methyl-1-azabicyclo[2.2.2]oct-3-yl)-1-hydroxynaphthalene-2-carboxamide,
N-(2-((3-pyridinyl)methyl-1-azabicyclo[2.2.2]oct-3-yl)-6-hydroxynaphthalene-2-carboxamide,
N-(2-((3-pyridinyl)methyl-1-azabicyclo[2.2.2]oct-3-yl)-6-acetoxynaphthalene-2-carboxamide,
N-(2-((3-pyridinyl)methyl)-1-azabicyclo[2.2.2]oct-3-yl)3-phenylprop-2-enamide,
N-(2-((3-pyridinyl)methyl)-1-azabicyclo[2.2.2]oct-3-yl)-3-(3-fluorophenyl)prop-2-enamide,
N-(2-((3-pyridinyl)methyl)-1-azabicyclo[2.2.2]oct-3-yl)-3-(4-methoxyphenyl)prop-2-enamide,
N-(2-((3-pyridinyl)methyl)-1-azabicyclo[2.2.2]oct-3-yl)-2-methyl-3-phenylprop-2-enamide,
N-(2-((3-pyridinyl)methyl)-1-azabicyclo[2.2.2]oct-3-yl)-3-(2-fluorophenyl)prop-2-enamide,
N-(2-((3-pyridinyl)methyl)-1-azabicyclo[2.2.2]oct-3-yl)-3-(3-methylphenyl)prop-2-enamide,
N-(2-((3-pyridinyl)methyl)-1-azabicyclo[2.2.2]oct-3-yl)-3-(4-fluorophenyl)prop-2-enamide,
N-(2-((3-pyridinyl)methyl)-1-azabicyclo[2.2.2]oct-3-yl)-3-(4-methylphenyl)prop-2-enamide,

N-(2-((3-pyridinyl)methyl)-1-azabicyclo[2.2.2]oct-3-yl)-3-(2-furyl)prop-2-enamide,
N-(2-((3-pyridinyl)methyl)-1-azabicycb[2.2.2]oct-3-yl)-3-(2-methoxyphenyl)prop-2-enamide,
N-(2-((3-pyridinyl)methyl)-1-azabicyclo[2.2.2]oct-3-yl)-3-(3-bromophenyl)prop-2-enamide,
N-(2-((3-pyridinyl)methyl)-1-azabicyclo[2.2.2]oct-3-yl)-3-(3-methoxyphenyl)prop-2-enamide,
N-(2-((3-pyridinyl)methyl)-1-azabicyclo[2.2.2]oct-3-yl)-3-(3-hydroxyphenyl)prop-2-enamide,
N-(2-((3-pyridinyl)methyl)-1-azabicyclo[2.2.2]oct-3-yl)-3-(4-bromophenyl)prop-2-enamide,
N-(2-((3-pyridinyl)methyl)-1-azabicyclo[2.2.2]oct-3-yl)-3-(4-chlorophenyl)prop-2-enamide,
N-(2-((3-pyridinyl)methyl)-1-azabicyclo[2.2.2]oct-3-yl)-3-(4-hydroxyphenyl)prop-2-enamide,
N-(2-((3-pyridinyl)methyl)-1-azabicyclo[2.2.2]oct-3-yl)-3-(4-hydroxy-3-methoxyphenyl)prop-2-enamide,
N-(2-((3-pyridinyl)methyl)-1-azabicyclo[2.2.2]oct-3-yl)-3-(2-thienyl)prop-2-enamide,
N-(2-((3-pyridinyl)methyl)-1-azabicyclo[2.2.2]oct-3-yl)-3-(3-pyridinyl)prop-2-enamide,
N-(2-((3-pyridinyl)methyl)-1-azabicyclo[2.2.2]oct-3-yl)-3-(4-biphenyl)prop-2-enamide,
N-(2-((3-pyridinyl)methyl)-1-azabicyclo[2.2.2]oct-3-yl)-3-(1-naphthyl)prop-2-enamide,
N-(2-((3-pyridinyl)methyl)-1-azabicyclo[22.2]oct-3-yl)-3-(3-thienyl)prop-2-enamide,
N-(2-((3-pyridinyl)methyl)-1-azabicyclo[2.2.2]oct-3-yl)-3-(4-isopropylphenyl)prop-2-enamide,
N-(2-((3-pyridinyl)methyl)-1-azabicyclo[2.2.2]oct-3-yl)-3-methyl-3-phenylprop-2-enamide,
N-(2-((3-pyridinyl)methyl)-1-azabicyclo[2.2.2]oct-3-yl)-3-(3-furyl)prop-2-enamide,
N-(2-((3-pyridinyl)methyl)-l -azabicyclo[2.2.2]oct-3-yl)-2-ethyl-3-phenylprop-2-enamide,
N-(2-((3-pyridinyl)methyl)-1-azabicyclo[2.2.2]oct-3-yl)-3-(2-pyridinyl)prop-2-enamide,
N-(2-((3-pyridinyl)methyl)-1-azabicyclo[2.2.2]oct-3-yl)-3-(3,4-dimethylthieno[2,3-b]thiophen-2-yl)prop-2-enamide,
N-(2-((3-pyridinyl)methyl)-1-azabicyclo[2.2.2]oct-3-yl)-3-(3-methylthien-2-yl)prop-2-enamide,
N-(2-((3-pyridinyl)methyl)-1-azabicyclo[2.2.2]oct-3-yl)-3-(2-naphthyl)prop-2-enamide,
N-(2-((3-pyridinyl)methyl)-1-azabicyclo[2.2.2]oct-3-yl)-3-(4-methylthiophenyl)prop-2-enamide,

or a pharmaceutically acceptable salt or solvate thereof.

In one embodiment, the compound of Formula 1 is (2S,3R)-N-(2-((3-pyridinyl)methyl)-1-azabicyclo[2.2.2]oct-3-yl)benzofuran-2-carboxamide or a pharmaceutically acceptable salt of solvate thereof, also referred to herein as Compound A.

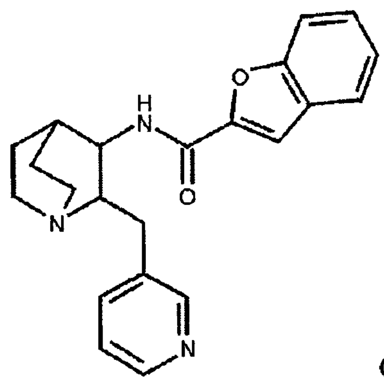

Compound A

In a further embodiment, the compound of Formula 1 is (2S,3R)-N-(2-((3-pyridinyl)methyl)-1-azabicyclo[2.2.2]oct-3-yl) benzofuran-2-carboxamide hydrochloric acid, phosphoric acid, maleic acid, or p-toluenesulfonic acid salt or a solvate thereof.

In one embodiment, the compound of Formula 1 is (2S,3R)-N-(2-((3-pyridinyl)methyl)-1-azabicyclo[2.2.2]oct-3-yl)-5-methylthiophene-2-carboxamide or a pharmaceutically acceptable salt thereof, also referred to herein as Compound B. In a further embodiment, the compound of Formula 1 is (2S,3R)-N-(2-((3-pyridinyl)methyl)-1-azabicyclo[2.2.2]oct-3-yl)-5-methylthiophene-2-carboxamide hydrochloric acid, phosphoric acid, or p-toluenesulfonic acid salt or a solvate thereof.

In one embodiment, the antipsychotic agent is either a conventional or atypical antipsychotic. In those embodiments where the antipsychotic is a conventional antipsychotic, the agent is selected from chlorpromazine, haloperidol, flupenthixol, or perphenazine, or a metabolite, salt, or solvate thereof. In those embodiments where the antipsychotic is an atypical antipsychotic, the agent is selected from clozapine, risperidone, otanzapine, quetiapine, aripiprazole, ziprasidone, amisulpride, sulpride, zotepine, sertindole, paliperidone, bifeprunox, or asenapine, or a metabolite, salt, or solvate thereof. In one embodiment, the at least one antipsychotic agent is clozapine or quetiapine, or a metabolite, salt, or solvate thereof.

The present invention includes a method for the treatment of prevention of a psychiatric disorder comprising the administration of a pharmaceutical combination according to the present invention. In one embodiment, the psychiatric disorder is a psychotic disorder. In a further embodiment, the psychiatric disorder is schizophrenia, schizophreniform disorder, schizoaffective disorder, delusional disorder, brief psychotic disorder, shared psychotic disorder, treatment-resistant psychotic disorder, a psychotic disorders due to a general medical conditions, or a psychotic disorder that is not otherwise specified. In still further an embodiment, the psychiatric disorder is schizophrenia.

The present invention includes a kit for the treatment or prevention of psychiatric disorders comprising a package containing a synergistic combination of one or more α7 nicotinic agonist and one or more antipsychotic agent.

BRIEF DESCRIPTION OF THE FIGURES

Figure 1 illustrates the ability of an α-7 nicotinic receptor agonist, specifically Compound B, to reverse deficit in sensory gaining in th(tk-)/th(tk-) mice (n =15). Compound B had no effect on control (n = 8).

Figure 2a illustrates that there was no significant main effect of Compound A in control mice, indicating that the drug had no effect on PPI in control mice. As illustrated in Figure 2b, however, Compound A did have an effect on transgenic [th(tk-)/th(tk-)] mice, thereby demonstrating the ability of Compound A to improve PPI in transgenic mice (n = 8).

Figure 3 illustrates the therapeutic effects of α7 nicotinic agonists on sensory gating. Transgenic mice had a higher startle response than control mice (n = 8).

Figure 4 illustrates the therapeutic effects on clozapine to correct sensory gaining in transgenic mice.

Figure 5 illustrates a synergistic interaction between clozapine and Compound A in transgenic mice (PPI) (n = 8).

Figure 6 illustrates a synergistic interaction between clozapine and Compound A in transgenic mice (startle response) (n = 8).

Figures 7a and 7b illustrate the ability of quetiapine to correct sensory gating in transgenic mice, PPI and startle response respectively.

Figure 8 illustrates a synergistic interaction between Compound A and quetiapine in transgenic mice (PPI and startle response).

Figure 9A and 9B are graphic illustrations of the effect of Compound A on α7 receptors expressed in *Xenopus* oocytes. Figure 9A: Dose-response for Compound A-evoked currents in human α7 receptors expressed in *Xenopus* oocytes. Figure 9B: Control ACh-evoked responses of human α7 receptors after the application of Compound A at the indicated concentrations. Data were normalized to the net charge of control 300 μM ACh responses obtained 5 min before the experimental agonist-evoked responses. Each point represents the Mean ± S EM of the normalized responses of at least 4 oocytes.

Figures 10A and 10B are graphic representations of data showing that Compound A has no effect on locomotor activity in the Open-field Test and Elevated Plus Maze. Figure 10A: The time Control or th(tk-)/th(tk-) (TK-) mice spent in the periphery or center zone of the open field. Although TK- mice spent significantly more time in the center (and consequently significantly less time in the periphery) than Controls, there was no effect of Compound A (0.3mg/kg). * = significantly different from controls ($p < 0.05$). Figure 10B. Compound A had no effect on time spent in the open and the closed arms of the elevated plus maze. Subjects of both genotypes spent significantly more time in the closed arms than in the open arms. TK- mice spent significantly more time in the open arms than controls. There was no effect of drug. * = significantly different from controls ($p < 0.05$).

Figure 11 is a graphic representation of data showing that Compound A reverses apomorphine-induced impairment of pre-pulse inhibition. To examine pre-pulse (PP) inhibition, the PP trials involved either a pre-pulse of 75 dB (=10 dB over background) or 85 dB (=20 dB over background) of 20 msec duration with onset 100 msec prior to a 120 dB pulse of 40 msec duration. The average inter-trial interval was set to 40 sec with a range of 20-60 sec, and the inter-trial interval length was randomized. The startle response was measured for 100 ms from the onset of the 120 dB pulse presentation. The magnitude of the "flinch" of a startled rat was measured. In an overall comparison among drug treatment factors (alone), administration of saline plus (-)-apomorphine (1.0 mg/kg; s.c.) significantly reduced %PPI compared with saline + vehicle-treated animals (+ $p<0.001$). Compound A (0.3 mg/kg; s.c.) significantly (**$p<0.001$) reversed apomorphine-induced PPI deficits (%PPI) following 0.3 Compound A + 1.0 Apo when compared to saline plus (-)-apomorphine (1.0 mg/kg; s.c.). The typical antipsychotic haloperidol (0.3 mg/kg; i.p.) significantly reversed (**$p<0.001$) the PPI deficits induced by apomorphine (%PPI) following 0.3 HAL + 1.0 Apo when compared to saline plus (-)-apomorphine (1.0 mg/kg; s.c). Data are expressed as mean ± SEM. (-)-Apomorphine was obtained from Sigma Chemical Co. (St. Louis, MO). (-)-Apomorphine was dissolved in saline containing 0.1 % (w/v) ascorbic acid (Sigma) and refrigerated in the dark to protect against oxidative degradation.

Figures 12A and 12B are graphic representations of data showing dose-response effects of Compound A on cognition

in a Novel Object Recognition paradigm. Figure 12A (left): Compound A was administered p.o. (0.3 - 10 mg/kg) and the effects on cognition were determined in a novel object recognition (NOR) paradigm, as described in 'Methods'. Results are expressed as the time spent exploring the novel and familiar objects (Mean ± SEM). **$p<0.02$ vs. vehicle controls. Figure 12A (right): Compound A was administered p.o. (0.3 mg/kg) and the exploration times were determined at 0.5, 2, 6, 18 and 24 h post-administration. Data are expressed as Mean ± SEM. * $p<0.05$ vs. vehicle controls. Figure 12B: A % recognition index was calculated (%RI=[(time investigating novel object)/(total time investigating both novel + familiar objects)]) at various times following administration of Compound A (0.3 mg/kg p.o.). Results represent the Recognition Index (RI) as a function of time following administration of Compound A and are expressed as Mean ± SEM. * $p<0.05$ vs. vehicle controls.

Figure 13 is a graphic representation showing the effects of Compound B on plasma glucose in obese db/db mice.

Figure 14 is a graphic representation showing the effects of Compound B on body weight in obese db/db mice.

DETAILED DESCRIPTION OF THE INVENTION

The combinations described herein are contemplated to provide synergistic effects in treating psychiatric disorders, particularly, psychotic disorders. The described combinations are contemplated to provide symptomatic relief of psychiatric disorders, particularly psychotic disorders, are contemplated to have fewer side effects, are contemplated to permit a reduction in use of these agents as compared to independent administration, are contemplated to complement sedatives and mood stabilizers such as lithium, and are contemplated to prophylactically address progression of psychotic conditions.

Exemplary psychotic disorders include, but are not limited to, schizophrenia, schizophreniform disorder, schizoaffective disorder, delusional disorder, brief psychotic disorder, shared psychotic disorder, treatment-resistant psychotic disorder and psychotic disorders due to a general medical conditions. The above conditions and disorders are defined for example in the American Psychiatric Association: Diagnostic and Statistical Manual of Mental Disorders, Fourth Edition, Text Revision, Washington, DC, American Psychiatric Association, 2000, herein incorporated by reference with regard to such definitions.

Compositions and methods described herein are contemplated to offer advantages over previous methods for treating neuropsychiatric disorders. The combination of the present invention provides enhanced efficacy of an antipsychotic when taken in combination with a nicotinic agonist and therefore permits reduced quantities of these agents to be used, as well as permits improved management of disease symptoms and disease-related side effects. A further advantage of this synergistic effect may be a quicker onset of the therapeutic effect than that of the individual compounds.

The nicotinic agonists of the present invention are those compounds having agonist or partial agonist activity at the $\alpha7$ NNR receptor subtype ($\alpha7$ nicotinic agonist). Particular nicotinic agonists useful in the combination of the present invention are those described in US Patent No. 6,953,855 and US application serial nos. 11/157,119, 11/458,231 and 60/971,654, each of which are hereby incorporated by reference.

Particular nicotinic agonists are the stereoisomeric forms of N-(2-((3-pyridinyl)methyl)-1-azabicyclo[2.2.2]oct-3-yl)benzofuran-2-carboxamide and metabolites or prodrugs and pharmaceutically-acceptable salts or solvates thereof.

An exemplary nicotinic agonist is (2S,3R)-N-(2-((3-pyridinyl)methyl)-1-azabicyclo[2.2.2]oct-3-yl)benzofuran-2-carboxamide, which is also referred to herein as Compound A. Compound A and other compounds of Formula 1 modulate nicotinic receptors in a subject's brain. As such, the compounds have the ability to express nicotinic pharmacology, and in particular, to act as nicotinic agonists. The preparation of these compounds is described in the above-referenced and incorporated patents and applications.

The following definitions are meant to clarify, but not limit, the terms defined. If a particular term used herein is not specifically defined, such term should not be considered indefinite. Rather, terms are used within their accepted meanings.

As used herein the term "alkyl" refers to a straight or branched chain hydrocarbon having one to twelve carbon atoms, preferably one to eight, more preferably one to six, which may be optionally substituted as herein further described, with multiple degrees of substitution being allowed. Examples of "alkyl" as used herein include, but are not limited to, methyl, ethyl, propyl, isopropyl, isobutyl, n-butyl, tert-butyl, isopentyl, and n-pentyl.

As used herein, the term "cycloalkyl" refers to a saturated optionally substituted nonaromatic, three- to twelve-membered, preferably three- to eight-membered, monocyclic, bicyclic, or bridged hydrocarbon ring, with multiple degrees of substitution being allowed. Exemplary "cycloalkyl" groups as used herein include, but are not limited to, cyclopropyl, cyclobutyl, cyclopentyl, cyclohexyl, and cycloheptyl, as well as rings containing one or more degrees of unsaturation but short of aromatic, such as cyclopropenyl, cyclobutenyl, cyclopentenyl, cyclohexenyl, and cycloheptenyl.

As used herein, the term "heterocycle" or "heterocyclyl" refers to an optionally substituted mono- or polycyclic ring system, optionally containing one or more degrees of unsaturation and also containing one or more heteroatoms, which may be optionally substituted as herein further described, with multiple degrees of substitution being allowed. Exemplary heteroatoms include nitrogen, oxygen, or sulfur atoms, including N-oxides, sulfur oxides, and dioxides. Preferably, the ring is three to twelve-membered and is either fully saturated or has one or more degrees of unsaturation. Such rings may be optionally fused to one or more of another heterocyclic ring(s) or cycloalkyl ring(s). Examples of "heterocyclic" groups as used herein include, but are not limited to, tetrahydrofuran, pyran, 1,4-dioxane, 1,3-dioxane, piperidine, pyrrolidine, morpholine, tetrahydrothiopyran, and tetrahydrothiophene.

As used herein, the term "aryl" refers to a univalent benzene ring or fused benzene ring system, which may be optionally substituted as herein further described, with multiple degrees of substitution being allowed. The term "aryl" also refers to a monocyclic five to seven membered aromatic ring, or to a fused bicyclic aromatic ring system comprising two of such aromatic rings, which may be optionally substituted as herein further described, with multiple degrees of substitution being allowed, which rings may contain one or more nitrogen, sulfur, and/or oxygen atoms (such as in 5- and 6-membered heteroaromatic rings), where N-oxides, sulfur oxides, and dioxides are permissible heteroatom substitutions. Examples of "aryl" groups as used include, but are not limited to, phenyl, 2-naphthyl, 1-naphthyl, anthracene, and phenanthrene. Further examples of "aryl" groups as used herein include, but should not be limited to, furan, thiophene, pyrrole, imidazole, pyrazole, triazole, tetrazole, thiazole, oxazole, isoxazole, oxadiazole, thiadiazole, isothiazole, pyridine, pyridazine, pyrazine, pyrimidine, quinoline, isoquinoline, benzofuran, benzothiophene, indole, indazole, benzimidizolyl, imidazopyridinyl, pyrazolopyridinyl, and pyrazolopyrimidinyl.

As used herein, the term "aralkyl" refers to an "aryl" group as herein defined attached through an alkylene linker, including but not limited to benzyl.

Compounds included within the scope of the present invention may form acid addition salts. Examples of suitable pharmaceutically acceptable salts include inorganic acid addition salts such as chloride, bromide, sulfate, phosphate, and nitrate; organic acid addition salts such as acetate, galactarate, propionate, succinate, lactate, glycolate, malate, tartrate, citrate, maleate, fumarate, methanesulfonate, p-toluenesulfonate, and ascorbate; salts with acidic amino acid such as aspartate and glutamate. Representative salts are provided as described in U.S. Patent Nos. 5,597,919 to Dull et al., 5,616,716 to Dull et al. and 5,663,356 to Ruecroft et al, each of which is herein incorporated by reference with regard to such teaching.

The pharmaceutically acceptable salts of Formula 1 may be of several different stoichiometries. Thus, in some cases the mole ratio of acid to base is 1:1; in other cases the mole ratio of acid to base is 1:2; and in yet other cases, the mole ratio of acid to base is 2:1. Other stoichiometries are also possible.

The pharmaceutically acceptable salts may be, in some cases, hydrates or ethanol solvates, which are also useful according to the present invention. Thus, as used herein, the term "solvate" includes solvates of compounds and solvates of salts of compounds.

Metabolites and pro-drugs of compounds that are herein described are also useful according to the present invention. Any reference to a compound should include an appreciation that a metabolite or a pro-drug of such compound is included, as well.

The term "therapeutically-effective amount" as used herein refers to a sufficient amount of the compound to treat psychiatric disorders, particularly psychotic disorders or conditions, at a reasonable risk-to-benefit ratio applicable to any medical treatment.

As used herein, the terms "prevention" or "prophylaxis" include any degree of reducing the progression of or delaying the onset of a disease, disorder, or condition. The term includes providing protective effects against a particular disease, disorder, or condition as well as amelioration of the recurrence of the disease, disorder, or condition. Thus, as one example, the invention provides a method for treating a subject having or at risk of developing or experiencing a recurrence of an NNR or nAChR mediated disorder. The compounds and pharmaceutical compositions of the invention may be used to achieve a beneficial therapeutic or prophylactic effect, for example, in a subject with a CNS dysfunction.

The term "disorder," unless stated otherwise, has the same meaning as the terms "condition" and "disease" and are used interchangeably throughout the specification and claims.

The term "synergy" means that the effect of the combination is greater than the sum of the two individual agents.

Disclosed herein are combinations comprising (a) an amount of a first therapeutic agent, which is an $\alpha7$ nicotinic agonist and (b) an amount of a second therapeutic agent, which is an antipsychotic.

Also disclosed herein are combinations of an $\alpha7$ nicotinic agonist and antipsychotic that are useful for the simultaneous, sequential, or separate treatment of psychotic disorders, particularly schizophrenia. Particularly, compositions comprising a pharmaceutical combination of an $\alpha7$ nicotinic agonist and atypical antipsychotic are described as useful for the simultaneous, sequential, or separate treatment of said disorders.

The invention also relates to a combination where the $\alpha7$ nicotinic agonist is (2S,3R)-N-(2-((3-pyridinyl)methyl)-1-azabicyclo[2.2.2]oct-3-yl)benzofuran-2-carboxamide and the atypical antipsychotic is either clozapine or quetiapine, including pharmaceutically-acceptable salts or solvates of any of these agents.

A second aspect of the invention relates to pharmaceutical compositions comprising a combination of (a) an amount of a first therapeutic agent, which is an $\alpha7$ nicotinic agonist and (b) an amount of a second therapeutic agent, which is an antipsychotic, particularly an atypical antipsychotic, together with a pharmaceutically acceptable carrier.

A third aspect of the invention relates to a kit comprising a dosage unit of mixture of a first therapeutic agent,

which is an α7 nicotinic agonist, and a second therapeutic agent, which is an antipsychotic, optionally with instructions for use.

A fourth aspect of the invention relates to a method for treating psychiatric disorders, particularly psychotic disorders, such as schizophrenia, in a subject in need thereof, comprising administering simultaneously, sequentially or separately to said subject (a) an amount of a first therapeutic agent, which is an α7 nicotinic agonist; and (b) an amount of a second therapeutic agent, which is an antipsychotic, wherein the amounts of (a) and (b) are together synergistically effective in the treatment.

Another aspect relates to said method wherein (a) an amount of a first therapeutic agent, which is an α7 nicotinic agonist and (b) an amount of a second therapeutic agent, which is an antipsychotic, are administered simultaneously, sequentially or separately, to the subject in a pharmaceutical composition additionally comprising a pharmaceutically acceptable carrier, by a method selected from the group consisting of oral, transmucosal, transdermal, nasal, pulmonary, buccal, parenteral rectal, and sublingual administration.

A further aspect relates to said method wherein (a) an amount of a first therapeutic agent, which is an α7 nicotinic agonist, and (b) an amount of a second therapeutic agent, which is an antipsychotic, are administered simultaneously, sequentially, or separately, to a subject in a pharmaceutical composition additionally comprising a pharmaceutically acceptable carrier, by a method selected from the group consisting of orally, parenterally, transmucosally, namely, sublingually or via buccal administration, topically, transdermally, rectally, or via inhalation, namely, nasal or deep lung inhalation. Parenteral administration includes, but is not limited to intravenous, intraarterial, intraperitoneal, subcutaneous, intradermal, intramuscular, intrathecal or via a high pressure technique.

Another aspect relates to said methods mentioned above wherein the α7 nicotinic agonist is (2S,3R)-N-(2-((3-pyridinyl)methyl)-1-azabicyclo[2.2.2]oct-3-yl)benzofuran-2-carboxamide and the atypical antipsychotic is either clozapine or quetiapine, including pharmaceutically-acceptable salts or solvates of any of these agents.

Another aspect relates to compositions and methods including α7 NNR agonists to address high blood sugar, diabetes, weight gain, and/or dyslipidemia resulting from antipsychotic administration. In particular embodiments, the compositions and methods include typical or atypical antipsychotics.

The association between antipsychotic use and high blood sugar, weight gain, dyslipidemia and diabetes has been well documented (American Diabetes Association et al. (2004); Henderson, DC et al. (2005a); Koller and Doraiswamy (2002); Sernyak, MJ (2002)). Lifestyle changes can be recommended to manage blood sugar, weight and diabetes, but lack of compliance is often a problem in the psychiatric population. Switching to a different antipsychotic is sometimes attempted, but the advantages must be weighed with the need for effective treatment of the psychiatric illness. Amantadine, metformin, sibutramine and topiramate are sometimes prescribed to offset the effects of antipsychotics on blood sugar and weight gain (Canitano, R (2005); Graham, KA et al. (2005); Henderson, DC et al. (2005b); Klein, DJ et al. (2006)), but side effects have been reported. The use of an NNR α7 agonist as adjunctive therapy to antipsychotic treatment may address the issues of high blood sugar, weight gain, dyslipidemia and/or diabetes with an improved side effect profile.

There is substantial evidence that administration of typical or atypical antipsychotics can lead to high blood sugar and diabetes (American Diabetes Association et al. (2004); Henderson, DC et al. (2005a); Koller and Doraiswamy (2002);Sernyak, MJ (2002)). The inventors' studies show that NNR α7 agonists (e.g., Compound B as described herein) are effective in diabetes models. Therefore, one aspect of the invention provides an advantage of administering an NNR α7 agonist in a clinical setting to counteract antipsychotic-induced high blood sugar, diabetes, weight gain, dyslipidemia, diabetes-related symptoms, complications of diabetes or complications of weight gain.

One aspect of the present invention involves concurrent administration of an NNR α7 agonist with an antipsychotic in a patient who is exhibiting high blood sugar, diabetes, weight gain, dyslipidemia, diabetes-related symptoms, complications of diabetes or complications of weight gain, or the administration of an NNR α7 agonist for the prevention of such in a patient who is taking an antipsychotic.

Compound B, an NNR a7 agonist, reduces blood sugar and weight gain in db/db mice, a model of diabetes (see Figures 13 and 14).

ALPHA 7 NICOTINIC AGONISTS

Compounds useful according to the present invention are α7 NNR selective ligands and include those compounds exemplified herein. For reference, Compound A is (2S,3R)-N-(2-((3-pyridinyl)methyl)-1-azabicyclo[2.2.2]oct-3-yl)benzofuran-2-carboxamide and is described in US Patent 6,953,855 and US applications 11/157,119,11/458,231, and 60/971,654, each of which is incorporated herein by reference for its disclosure relevant to compounds and their synthesis whose structures that are represented by Formula 1.

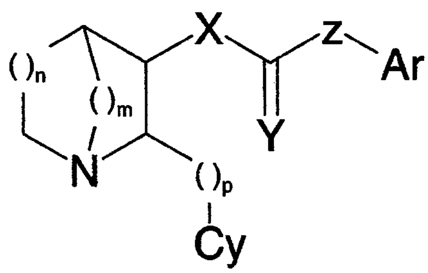

Formula 1

In Formula 1, m and n individually can have a value of 1 or 2, and p can have a value of 1, 2, 3 or 4. In the Formula, X is either oxygen or nitrogen (i.e., NR'), Y is either oxygen or sulfur, and Z is either nitrogen (i.e., NR'), a covalent bond or a linker species, A. A is selected from the group -CR' R"-, -CR' R"- CR' R"-, -CR'= CR'-, and -$C_2$-, wherein R' and R" are as hereinafter defined. When Z is a covalent bond or A, X must be nitrogen. Ar is an aryl group, either carbocyclic or heterocyclic, either monocyclic or fused polycyclic, unsubstituted or substituted; and Cy is a 5- or 6-membered heteroaromatic ring, unsubstituted or substituted. Thus, the invention includes compounds in which Ar is linked to the azabicycle by a carbonyl group-containing functionality, such as an amide, carbamate, urea, thioamide, thiocarbamate or thiourea functionality. In addition, in the case of the amide and thioamide functionalities, Ar may be bonded directly to the carbonyl (or thiocarbonyl) group or may be linked to the carbonyl (or thiocarbonyl) group through linker A. Furthermore, the invention includes compounds that contain a 1-azabicycle, containing either a 5-, 6-, or 7-membered ring and having a total of 7, 8 or 9 ring atoms (e.g., 1-azabicyclo[2.2.1]heptane, 1-azabicyclo[3.2.1]octane, 1-azabicyclo[2.2.2]octane, and 1-azabicyclo[3.2.2]nonane).

In one embodiment, the value of p is 1, Cy is 3-pyridinyl or 5-pyrimidinyl, X and Y are oxygen, and Z is nitrogen. In another embodiment, the value of p is 1, Cy is 3-pyridinyl or 5-pyrimidinyl, X and Z are nitrogen, and Y is oxygen. In a third embodiment, the value of p is 1, Cy is 3-pyridinyl or 5-pyrimidinyl, X is nitrogen, Y is oxygen, and Z is a covalent bond (between the carbonyl and Ar). In a fourth embodiment, the value of p is 1, Cy is 3-pyridinyl or 5-pyrimidinyl, X is nitrogen, Y is oxygen, Z is A (a linker species between the carbonyl and Ar).

The compounds of Formula 1 have one or more asymmetric carbons and can therefore exist in the form of racemic mixtures, enantiomers and diastereomers. The wavy lines indicate that both relative and absolute stereochemistry at those sites are variable (e.g., cis or *trans,* R or S). In addition, some of the compounds exist as E and Z isomers about a carbon-carbon double bond. All these individual isomeric compounds and their mixtures are also intended to be within the scope of Formula 1.

As used in Formula 1, Ar ("aryl") includes both carbocyclic and heterocyclic aromatic rings, both monocyclic and fused polycyclic, where the aromatic rings can be 5- or 6-membered rings. Representative monocyclic aryl groups include, but are not limited to, phenyl, furanyl, pyrrolyl, thienyl, pyridinyl, pyrimidinyl, oxazolyl, isoxazolyl, pyrazolyl, imidazolyl, thiazolyl, isothiazolyl and the like. Fused polycyclic aryl groups are those aromatic groups that include a 5- or 6-membered aromatic or heteroaromatic ring as one or more rings in a fused ring system. Representative fused polycyclic aryl groups include naphthalene, anthracene, indolizine, indole, isoindole, benzofuran, benzothiophene, indazole, benzimidazole, benzthiazole, purine, quinoline, isoquinoline, cinnoline, phthalazine, quinazoline, quinoxaline, 1,8-naphthyridine, pteridine, carbazole, acridine, phenazine, phenothiazine, phenoxazine, and azulene.

As used in Formula 1, "Cy" groups are 5- and 6-membered ring heteroaromatic groups. Representative Cy groups include pyridinyl, pyrimidinyl, furanyl, pyrrolyl, thienyl, oxazolyl, isoxazolyl, pyrazolyl, imidazolyl, thiazolyl, isothiazolyl and the like, where pyridinyl is preferred.

Individually, Ar and Cy can be unsubstituted or can be substituted with 1, 2, or 3 substituents, such as alkyl, alkenyl, heterocyclyl, cycloalkyl, aryl, substituted aryl, arylalkyl, substituted arylalkyl, halo (e.g., F, Cl, Br, or I), -OR', -NR'R", -$CF_3$, -CN, -$NO_2$, -$C_2$R', -SR', -$N_3$, -C(=O)NR'R", -NR'C(=O) R", -C(=O)R', -C(=O)OR', -OC(=O)R', -O(CR'R")$_r$C (=O)R', -O(CR'R")$_r$NR"C(=O)R', -O(CR'R")$_r$NR"$SO_2$R', -OC(=O)NR'R", -NR'C(=O)O R", -$SO_2$R', -$SO_2$NR'R", and -NR'$SO_2$R", where R' and R" are individually hydrogen, alkyl, preferably $C_1$-$C_8$ alkyl, preferably $C_1$-$C_5$, cycloalkyl, preferably $C_{3-8}$, heterocyclyl, aryl, or arylalkyl, preferably benzyl.

Representative compounds of Formula 1 include particular compounds described herein.

With regard to the above-referenced listing of representative compounds, salts or solvates of such compounds are included.

A preferred embodiment of Formula 1 is (2S,3R)-N-(2-((3-pyridinyl)methyl)-1-azabicyclo[2.2.2]oct-3-yl)benzofuran-2-carboxamide. Preferred salt forms of (2S,3R)-N-(2-((3-pyridinyl)methyl)-1-azabicyclo[2.2.2]oct-3-yl)benzofuran-2-carboxamide are described in US application 60/971,654 and include the hydrochloric acid, phosphoric acid, maleic acid and p-toluenesulfonic acid salts, as well as solvates of such salts.

Those skilled in the art will recognize that, in light of this disclosure, any nicotinic agonists having binding action at α7 NNRs may be useful in the combinations, pharmaceutical compositions, methods and kits described herein.

ANTIPSYCHOTIC AGENTS

Compounds useful according to the present invention are antipsychotics, both conventional and atypical.
Examples of conventional antipsychotics include, but are not limited to, chlorpromazine, haloperidol, flupenthixol, and perphenazine, as well as salts or solvates thereof.
Examples of atypical antipsychotics include, but are not limited to, clozapine, risperidone, olanzapine, quetiapine, aripiprazole, ziprasidone, amisulpride, sulpride, zotepine, sertindole, paliperidone, bifeprunox, and asenapine, as well as salts or solvates thereof.
Preferred antipsychotics are clozapine and quetiapine, as well as salts or solvates thereof.
Suitable pharmaceutically acceptable salts of the antipsychotic compounds described herein include acid addition salts which may, for example, be formed by mixing a solution of the compound according to the invention with a solution of a pharmaceutically-acceptable acid. Furthermore, where the compounds carry an acidic moiety, suitable pharmaceutically-acceptable salts thereof may include alkali metal salts, such as sodium or potassium salts, alkaline earth metal salts, such as calcium or magnesium salts, and salts formed with suitable organic bases, such as quaternary ammonium salts.

PHARMACEUTICAL COMPOSITIONS

The pharmaceutical compositions of the present invention comprise a combination of (a) an amount of a first therapeutic agent, which is an α7 nicotinic agonist and (b) an amount of a second therapeutic agent, which is an antipsychotic, together with a pharmaceutically-acceptable vehicle, carrier or diluent.
In one embodiment, the α7 nicotinic agonist is a compound of Formula 1, or a pharmaceutically acceptable salt or solvate thereof, and antipsychotic is an atypical antipsychotic agent.
In another embodiment, the α7 nicotinic agonist is a compound of Formula 1, or a pharmaceutically acceptable salt or solvate thereof, and the antipsychotic is either clozapine or quetiapine or a pharmaceutically acceptable salt or solvate thereof.
In another embodiment, the α7 nicotinic agonist is (2S,3R)-N-(2-((3-pyridinyl)methyl)-1-azabicyclo[2.2.2]oct-3-yl)benzofuran-2-carboxamide, or a pharmaceutically acceptable salt or solvate thereof, and the antipsychotic is an atypical antipsychotic.
In another embodiment, the α7 nicotinic agonist is (2S,3R)-N-(2-((3-pyridinyl)methyl)-1-azabicyclo[2.2.2]oct-3-yl)benzofuran-2-carboxamide, or a pharmaceutically acceptable salt or solvate thereof, and the antipsychotic is either clozapine or quetiapine, or a pharmaceutically acceptable salt or solvate thereof.
The active ingredients of the composition described herein can be co-administered simultaneously or may be administered separately or sequentially in any order, or as a single pharmaceutical composition.
The combinations described herein can be administered in a standard manner for the treatment of psychiatric disorders, particularly psychotic disorders, such as orally, parenterally, transmucosally such as sublingually or via buccal administration, topically, transdermally, rectally, or via inhalation such as nasal or deep lung inhalation. Parenteral administration includes, but is not limited to intravenous, intraarterial, intraperitoneal, subcutaneous, intramuscular, intrathecal or via a high pressure technique.
For buccal administration, the composition can be in the form of tablets or lozenges formulated in conventional manner. For example, tablets and capsules for oral administration can contain one or more conventional carriers such as binding agents, such as syrup, acacia, gelatin, sorbitol, tragacanth, mucilage of starch or polyvinylpyrrolidone, fillers, such as, lactose, sugar, microcrystalline cellulose, maize-starch, calcium phosphate or sorbitol, lubricants, such as magnesium stearate, stearic acid, talc, polyethylene glycol, or silica, disintegrants, such as potato starch or sodium starch glycollate, or wetting agents, such as sodium lauryl sulfate. Tablets may be coated according to methods known in the art.
Such preparations can also be formulated as suppositories for rectal administration with one or more carrier, namely, containing conventional suppository bases, such as cocoa butter or other glycerides.
Compositions for inhalation typically can be provided in the form of a solution, suspension, or emulsion that can be administered as a dry powder or in the form of an aerosol using a conventional carrier such as a propellant, such as dichlorodifluoromethane or trichlorofluoromethane.
Typical topical and transdermal compositions may comprise conventional aqueous or nonaqueous carriers, such as eye drops, creams, ointments, lotions, and pastes, or may be in the form of a medicated plaster, patch, or membrane.
Additionally, compositions described herein can be formulated for parenteral administration by injection or

continuous infusion. Compositions for injection can be in the form of suspensions, solutions, or emulsions in oily or aqueous carriers, and can contain composition agents, such as suspending, stabilizing, and/or dispersing agents. Alternatively, the active ingredient can be in powder form for constitution with a suitable carrier (e.g., sterile, pyrogen-free water) before use.

A composition in accordance with the present invention also can be formulated as a depot preparation. Such long acting compositions can be administered by implantation (e.g., subcutaneously or intramuscularly) or by intramuscular injection. Accordingly, the compounds of the invention can be formulated with suitable polymeric or hydrophobic materials (e.g., an emulsion in an acceptable oil), ion exchange resins, or as sparingly soluble derivatives (e.g., a sparingly soluble salt).

For oral administration a pharmaceutical composition can take the form of solutions, suspensions, tablets, pills, capsules, powders, and the like. Tablets containing various pharmaceutically acceptable carriers, namely, excipients such as sodium citrate, calcium carbonate and calcium phosphate are employed along with various disintegrants such as starch, for example potato or tapioca starch, and certain complex silicates, together with binding agents such as polyvinylpyrrolidone, sucrose, gelatin and acacia. Additionally, lubricating agents such as magnesium stearate, sodium lauryl sulfate and talc may be used to form tablets. Solid compositions of a similar type are also employed as fillers in soft and hard-filled gelatin capsules; examples of materials in this connection may also include lactose or milk sugar as well as high molecular weight polyethylene glycols.

Alternatively, the composition described herein can be incorporated into oral liquid preparations such as aqueous or oily suspensions, solutions, emulsions, syrups, or elixirs, for example. Moreover, compositions containing these compounds can be presented as a dry product for constitution with water or other suitable vehicle before use. Such liquid preparations can contain conventional carriers, such as suspending agents, for example sorbitol syrup, synthetic and natural gums such as tragacanth, acacia, alginate, dextran, sodium carboxymethylcellulose, methylcellulose, polyvinyl-pyrrolidone or gelatin, glucose/sugar syrup, gelatin, hydroxyethylcellulose, hydroxypropylmethylcellulose, aluminum stearate gel, emulsifying agents, such as lecithin, sorbitan monooleate, or acacia; nonaqueous vehicles (which can include edible oils), such as almond oil, fractionated coconut oil, oily esters, propylene glycol, and ethyl alcohol; and preservatives, such as methyl or propyl p-hydroxybenzoate and sorbic acid. The liquid forms in which the compositions described herein may be incorporated for administration orally or by injection include aqueous solutions, suitably flavored syrups, aqueous or oil suspensions, and flavored emulsions with edible oils such as cottonseed oil, sesame oil, coconut oil or peanut oil, as well as elixirs and similar pharmaceutical vehicles.

When aqueous suspensions and elixirs are desired for oral administration, the compounds described herein can be combined with various sweetening agents, flavoring agents, coloring agents, emulsifying agents and/or suspending agents, as well as such diluents as water, ethanol, propylene glycol, glycerin and various like combinations thereof. Suitable dispersing or suspending agents for aqueous suspensions may include synthetic and natural gums such as tragacanth, acacia, alginate, dextran, sodium carboxymethylcellulose, methylcellulose, polyvinyl-pyrrolidone or gelatin.

The combinations described herein can also be administered in a controlled release composition such as a slow release composition, a fast or immediate release composition, or a delayed, controlled, or modified release composition. Such controlled release compositions of the combinations described herein may be prepared using methods known to those skilled in the art. The method of administration will be determined, by the attendant physician or other person skilled in the art after an evaluation of the patient's condition and requirements.

KIT

The kits of the invention comprise a dosage unit of mixture of a first therapeutic agent, which is an $\alpha$7 nicotinic agonist, and a second therapeutic agent, which is an antipsychotic, optionally with instructions for use. In one embodiment, the $\alpha$7 nicotinic agonist is a compound of Formula 1, and or a pharmaceutically acceptable salt or solvate thereof, and antipsychotic is an atypical antipsychotic. In another embodiment, the $\alpha$7 nicotinic agonist is a compound of Formula 1, or a pharmaceutically acceptable salt or solvate thereof, and the antipsychotic is either clozapine or quetiapine, or a pharmaceutically acceptable salt or solvate thereof. In another embodiment, the $\alpha$7 nicotinic agonist is (2S,3R)-N-(2-((3-pyridinyl)methyl)-1-azabicyclo[2.2.2]oct-3-yl)benzofuran-2-carboxamide, or a pharmaceutically acceptable salt or solvate thereof, and the antipsychotic is an atypical antipsychotic. In another embodiment, the $\alpha$7 nicotinic agonist is (2S, 3R)-N-(2-((3-pyridinyl)methyl)-1-azabicyclo[2.2.2]oct-3-yl)benzofuran-2-carboxamide, or a pharmaceutically acceptable salt or solvate thereof, and the antipsychotic is either clozapine or quetiapine, or a pharmaceutically acceptable salt or solvate thereof.

METHOD OF TREATMENT

The invention includes methods for treating psychiatric disorders, particularly psychotic disorders, in a subject in need thereof, comprising administering simultaneously, sequentially or separately, to said subject (a) an amount of

a first therapeutic agent, which is an α7 nicotinic agonist and (b) an amount of a second therapeutic agent, which is an antipsychotic, wherein the amounts of (a) and (b) are together synergistically effective in the treatment.

One embodiment relates to a method for treating psychotic disorders in a subject in need thereof comprising administering simultaneously, sequentially or separately, to said subject (a) an amount of a first therapeutic agent, which is an α7 nicotinic agonist and (b) an amount of a second therapeutic agent, which is an antipsychotic, wherein the amounts of (a) and (b) are together synergistically effective in the treatment.

In one embodiment, the psychotic disorder or condition is selected from the group consisting of schizophrenia, schizophreniform disorder, schizoaffective disorder, delusional disorder, brief psychotic disorder, shared psychotic disorder, treatment-resistant psychotic disorder, psychotic disorders due to a general medical conditions, and psychotic disorder not otherwise specified. In another embodiment, the α7 nicotinic agonist is a compound of Formula 1, or pharmaceutically-acceptable salt, solvate or solvated salt thereof, and the antipsychotic is chosen from a group consisting of clozapine, risperidone, olanzapine, quetiapine, aripiprazole, ziprasidone, amisulpride, sulpride, zotepine, sertindole, paliperidone, bifeprunox and asenapine, or pharmaceutically-acceptable salt, solvate or solvated salt thereof.

Another embodiment relates to a method for treating schizophrenia in a subject in need thereof comprising administering simultaneously, sequentially or separately, to said subject (a) an amount of a first therapeutic agent, which is an α7 nicotinic agonist and (b) an amount of a second therapeutic agent, which is an antipsychotic, wherein the amounts of (a) and (b) are together synergistically effective in the treatment. In another embodiment, the α7 nicotinic agonist is a compound of Formula 1, or pharmaceutically-acceptable salt, solvate or solvated salt thereof, and the antipsychotic is chosen from a group consisting of clozapine, risperidone, olanzapine, quetiapine, aripiprazole, ziprasidone, amisulpride, sulpride, zotepine, sertindole, paliperidone, bifeprunox and asenapine, or pharmaceutically-acceptable salt, solvate or solvated salt thereof. In a preferred embodiment, the α7 nicotinic agonist is (2S,3R)-N-(2-((3-pyridinyl) methyl)-1-azabicyclo[2.2.2]oct-3-yl)benzofuran-2-carboxamide, or a pharmaceutically acceptable salt or solvate thereof, and the antipsychotic is either clozapine or quetiapine, or a pharmaceutically acceptable salt or solvate thereof.

Another embodiment of the invention relates to the use of the combination comprising (a) an amount of a first therapeutic agent, which is an α7 nicotinic agonist and (b) an amount of a second therapeutic agent, which is an antipsychotic, for the manufacturing of a medicament for the treatment, simultaneously, sequentially or separately, of psychiatric disorders, particularly psychotic disorders.

With regard to synergy, as herein described, the present invention is a combination of an α7 nicotinic agonist and an antipsychotic agent. The present combination is believed to provide synergy in the treatment or prophylaxis of cognitive dysfunction. As one example, symptoms associated with schizophrenia are divided into three categories: positive, namely hallucinations, delusions, and thought disorder; negative, namely anhedonia, poverty of speech, and lack of motivation; and cognitive, namely attention, memory, and executive function. Research conducted over the past decade supports the premise that, in addition to α4β2 NNRs, the α7 subtype has great potential as a therapeutic target for cognitive deficits, as well as the positive symptoms of schizophrenia. The origin of this line of investigation is largely attributed to epidemiological reports showing an increased rate of smoking among schizophrenics compared with the general population and suggesting an attempt at self-medication with nicotine. In further support, numerous additional studies have begun to delineate the potential for, and mechanisms underlying, schizophrenia therapeutics involving α7 receptor modulation.

From a physiological perspective, several studies have demonstrated the presence of α7 receptors in various mechanistic pathways known to play a role in schizophrenia etiology and symptomatology. For example, postmortem studies on brain tissue from schizophrenic patients show a marked decrease in the number of α7 NNRs in hippocampus and cortex. These receptors are located in the hippocampus, lateral, and medial geniculate nuclei, and reticular nucleus of the thalamus and are thought to modulate neurotransmitter release, including glutamate, GABA, and dopamine, involved in LTP formation, sensory processing, and neuroprotection. Sensory inhibition deficits and familial schizophrenia have been linked to the 15q13-q14 region on chromosome 15. CHRNA7, the gene for the α7 NNR, is located in this region and polymorphisms in the promoter region of the gene have also been identified. Thus, the potential for α7 mechanisms to play a role in schizophrenia is well supported.

Further investigation into the functional consequences of α7 NNR modulation with *in vivo* animal models has provided additional support for their therapeutic potential in schizophrenia. Most individuals with schizophrenia exhibit cognitive impairment. The cognitive deficits in schizophrenia include attention disorders, slow information processing, working memory disorders and deficits in executive function. Recent consensus meetings (MATRICS/TURNS initiative) identified cognitive deficits in schizophrenia (CDS) as a core feature of the illness that contributes significantly to the lack of functionality of patients. With the need for a therapy to treat cognitive deficits and the evidence of a role for α7 in schizophrenia, the MATRICS initiative also endorsed the α7 subtype as a primary therapeutic target relevant to CDS.

Preclinical testing with multiple α7 ligands has shown the promise of targeting this receptor. For example, GTS-21, a functionally selective α7 agonist, displays effectiveness in several behavioral models of learning and memory including passive avoidance in lesioned rats, active avoidance in the aged rat, eye-blink conditioning in the aged rabbit, and a delayed-matching task in a non-human primate. DBA/2 mice, fimbria-fornix lesioned rats and isolation reared rats

display sensory processing deficits similar to those seen in schizophrenics. GTS-21 improves auditory gating deficits in these animal models and other α7-selective compounds have shown efficacy in sensory gating models. Finally, *in vivo* infusion of MLA, an α7-selective antagonist, in the ventral hippocampus or basolateral amygdala causes significant working memory deficits in rats as observed in radial arm maze tasks, indicating a requirement for α7 receptors in memory processing.

Although treatment of CDS is a clear objective for new antipsychotic therapeutics, the advantage of ligands targeting the α7 NNR is that they also seem to be efficacious in terms of ameliorating some of schizophrenia's positive symptoms. Specifically, the non-selective nicotinic agonist, nicotine, ameliorates defects in schizophrenia such as sensory gating deficit and smooth pursuit eye movement abnormalities. An α7 selective agonist has been shown to reverse PPI deficits in isolation-reared rats, a classic model for antipsychotics. Another example, AR-R17779, another α7 selective agonist, improves scopolamine-induced deficits in social recognition and improves long-term learning and attenuates working memory deficits in rats. The α7 selective agonist SSR180711A has been shown to be effective in object recognition paradigms for long-term and short-term episodic memory and in Morris water maze models for reference and working memory, suggesting that α7-selective compounds could also potentially treat the cognitive deficits of the disease. Recently, a proof-of-concept trial in schizophrenia was undertaken with the α7 nicotinic agonist DMXB-A (also known as GTS-21 referred to herein). Significant improvements in the Repeatable Battery for the Assessment of Neuropsychological Status total scale score and in P50 inhibition were observed. The positive effects of this compound in the clinic support the continued development of selective α7 agonists for cognitive deficits of schizophrenia and potentially for the positive symptoms of the disease.

Reference is made to WONG AHC, VAN TOL HHM: Schizophrenia: from phenomenology to neurobiology. Neurosci. Biobehav. Rev. (2003) 27: 269-306; LEONARD S, ADLER LE, BENHAMMOU K et al.: Smoking and mental illness. Pharmaco/. Biochem. Behav. (2001) 70: 561-570; FREEDMAN F, HALL M, ADLER LE, LEONARD S: Evidence in postmortem brain tissue for decreased numbers of hippocampal nicotinic receptors in schizophrenia Biol. Psychiatry (1995) 38: 22-33; GUAN ZZ, ZHANG X, BLENNW K et al.: Decreased protein level of nicotinic acetylcholine receptor α7 subunit in the frontal cortex from schizophrenic brain. NeuroReport (1999) 10: 1779-1782; BREESE CR, ADAMS C, LOGEL J et al.: Comparison of the regional expression of nicotinic acetylcholine receptor alpha7 mRNA and [1251]-alpha-bungarotoxin binding in human postmortem brain. J. Comp. Neurol. (1997) 387: 385-398; MANSVELDER,HD, MCGEHEE DS: Long-term potentiation of excitatory inputs to brain reward areas by nicotine. Neuron (2000) 27: 349-357; EGEA J, ROSA AO, SOBRADO M, GANDIA L, LOPEZ MG, GARCIA AG: Neuroprotection afforded by nicotine against oxygen and glucose deprivation in hippocampal slices is lost in alpha7 nicotinic receptor knockout mice. Neuroscience (2007) 145: 866-872; LEONARD S, FREEDMAN R: Genetics of Chromosome 15q13-q14 in Schizophrenia. Biol. Psychiatry (2006) 60: 115-122; WALKER E, KESTLER L, BOLLINI A, HOCHMAN KM: Schizophrenia: Etiology and course. Annu. Rev. Psychol. (2004) 55: 401-430; FENTON WS, STOVER EL, INSEL TR: Breaking the log-jam in treatment development for cognition in schizophrenia: NIMH perspective. Psychopharmacology (2003) 169: 365-366; MEYER EM, DE FIEBRE DM, HUNTER BE, SIMPKINS CE, FRAUWORTH N, DE FIEBRE NE: Effects of anabaseine-related analogs on rat brain nicotinic receptor binding and on avoidance behaviors. Drug Dev. Res. (1994) 31:127-134; ARENDASH GW, SENGSTOCK GJ, SANBERG PR, KEM WR: Improved learning and memory in aged rats with chronic administration of nicotinic receptor agonist GTS-21. Brain Res. (1995) 674: 252-259; WOODRUFF-PAK DS, LI YT, KAZMI A, KEM WR: Nicotinic cholinergic system involvement in eyeblink classical conditioning in rabbits. Behav. Neurosci. (1994) 108: 486-493; BRIGGS CA, ANDERSON DJ, BRIONI JD et al.: Functional characterization of a novel neuronal nicotinic acetylcholine receptor ligand GTS-21 in vitro and in vivo. Pharmacol. Biochem. Behav. (1997) 57: 231-241; SIMOSKY JK, STEVENS KE, KEM WR, FREEDMAN R: Intragastric DMXB-A, an alpha7 nicotinic agonist, improves deficient sensory inhibition in DBA/2 mice. Biol. Psychiatry (2001) 50: 493-500; HURST RS, HAJÓS M, RAGGENBASS M et al.: A novel positive allosteric modulator of the α7 neuronal nicotinic acetylcholine receptor: in vitro and in vivo characterization. J. Neurosci. (2005) 25: 4396-4405; HAJÓS M, HURST RS, HOFFMANN WE et al.: The selective α7 nicotinic acetylcholine receptor agonist PNU-282987 [N-[(3R)-1-azabicyclo[2.2.2]oct-3-yl]-4-chlorobenzamide hydrochloride] enhances GABAergic synaptic activity in brain slices and restores auditory gating deficits in anesthetized rats. JPET (2005) 312:1213-1222; BETTANY JH, LEVIN ED: Ventral hippocampal alpha 7 nicotinic receptor blockade and chronic nicotine effects on memory performance in the radial-arm maze. Pharmacol. Biochem. Behav. (2001) 70: 467-474; ADDY NA, NAKIJAMA A, LEVIN ED: Nicotinic mechanisms of memory: effects of acute local DhbetaE and MLA infusions in the basolateral amygdala. Brain Res. Cogn. Brain Res. (2003) 16: 51-57; ADLER LE, OLINCY A, WALDO MC et al.: Schizophrenia, sensory gating and nicotinic receptors. Schizophr. Bull. (1998) 24: 189-202; ROSS RG, OLINCY A, HARRIS JG et al.: Anticipatory saccades during smooth pursuit eye movements and familial transmission of schizophrenia. Biol. Psychiatry (1998) 44: 690-697; CILIA J, CLUDERAY JE, ROBBINS MJ et al.: Reversal of isolation-rearing-induced PPI deficits by an α7 nicotinic receptor agonist. Psychopharmacology (2005) 182: 214-219; VAN KAMPEN M, SELBACH K, SCHNEIDER R, SCHIEGEL E, BOESS F, SCHREIBER R: AR-R 17779 improves social recognition in rats by activation of nicotinic α7 receptors. Psychopharmacology (2004) 172: 375-383; LEVIN ED, BETTEGOWDA C, BLOSSER J, GORDON J: AR-R 17779, an α7 nicotinic agonist, improves learning and memory in rats. Behav. Pharmacol.

(1999) 10: 675-680; PICHAT P, BERGIS OE, TERRANOVA JP et al.: SSR180711A, a novel selective α7 nicotinic receptor partial agonist. III. Effects in models predictive of therapeutic activity on cognitive symptoms of schizophrenia. Soc. Neurosci. Abstr. (2004) 34: 583.3; BERGIS OE, PICHAT P, SANTAMARIA R et al.: SSR180711A, a novel selective α7 nicotinic receptor partial agonist. II. Effects in models predictive of therapeutic activity on cognitive symptoms of Alzheimer's disease. Soc. Neurosci. Abstr. (2004) 34: 583.2; and OLINCY A, HARRIS JG, JOHNSON LL et al.: Proof-of-concept trial of an α7 nicotinic agonist in schizophrenia. Arch. Gen. Psychiatry (2006) 63: 630-638, each of which is herein incorporated by reference with regard to the pharmacology described therein.

DOSAGE

The effective dose of an α7 nicotinic agonist and antipsychotic in the combinations according to the present invention may vary, depending upon factors such as the condition of the patient, the severity of the symptoms of the disorder as well as the potency of the selected specific compound, the mode of administration, the age and weight of the patient, and the like.

Typically, the effective dose of nicotinic agonists generally requires administering the compound in an amount of less than 5 mg/kg of patient weight. Often, the nicotinic agonists are administered in an amount from less than about 1 mg/kg patent weight to less than about 100 μg/kg of patient weight, and occasionally between about 10 μg/kg to less than 100 μg/kg of patient weight. The foregoing effective doses typically represent that amount administered as a single dose, or as one or more doses administered over a 24 hours period.

For human patients, the effective dose of the nicotinic agonists generally requires administering the nicotinic agonist in an amount of at least about 1, often at least about 10, and frequently at least about 25 mg/ 24 hr./ patient. For human patients, the effective dose of the nicotinic agonists requires administering the nicotinic agonist which generally does not exceed about 500, often does not exceed about 400, and frequently does not exceed about 300 mg/ 24 hr./ patient. In addition, administration of the effective dose is such that the concentration of the nicotinic agonist within the plasma of the patient normally does not exceed 500 ng/mL, and frequently does not exceed 100 ng/mL.

The exact composition, route of administration, and dosage can be chosen by the individual physician in view of the patient's condition. Dosage amount and interval can be adjusted individually to provide plasma levels of the active moiety, which are sufficient to maintain therapeutic effects.

The effective dose of the antipsychotic varies with the nature of the antipsychotic. Desirably, when quetiapine is selected as the antipsychotic agent, the daily dose of the combination contains from about 1 mg to about 1200 mg. Preferably, each dose of the first component contains about 25 mg to about 1000 mg of the quetiapine, and even more preferably, each dose contains from about 150 mg to about 800 mg or 300 mg to about 800 mg or 400 mg to about 800 mg of quetiapine. In another embodiment the first component contains about 150-300 or 300-600 mg of the quetiapine. Pediatric dosages may be less such as for example in the range of about 0.5 mg to about 40 mg daily. These dosages may be administered in one, two or more oral doses, for example: quetiapine: from about 1.0 to about 40 mg/kg given once daily or in divided doses.

<u>EXAMPLES</u>

The complex brain pathologies observed in schizophrenics include dopamine (DA) neuron hypoplasia accompanied by hyperactivity of the subcortical DA systems and reduced cortical DA function. A widely accepted formulation of the dopamine hypothesis of schizophrenia (SZ) has the positive symptoms of the disorder (hallucinations, delusions and impaired sensory gating) associated with hyperactivity of the subcortical dopaminergic systems, while the negative symptoms (flattened affect, social withdrawal) are caused by dopamine hypoactivity in the prefrontal cortex. The mechanism by which the subcortical DA pathway is hyperactive and the cortical DA pathway is hypoactive in schizophrenia and how these changes cause the behavioral symptoms is not well understood. Furthermore, the pathogenesis of schizophrenia likely stems from a multifactorial neurodevelopmental derangement involving other neuronal systems (i.e., serotonin neurons, cortical and hippocampal neurons) in addition to the abnormal development and neurotransmission in DA systems. The relationship between DA and other affected systems and their combined contribution to the complex clinical symptoms have not been established in part due to the lack of adequate experimental models that mimic the multifactorial developmental and associated functional deficits as observed in SZ.

Recently multiple genetic links have been identified for this disorder, including factors that influence neural development via cyclic AMP, MAPK signaling pathways, transcription co-activator complexes and epigenetic mechanisms.

Attempts to create animal genetic models, however, by introducing individual mutations into laboratory animals have not to date replicated the complex neuronal and behavioral symptoms typical to SZ.

Recently, changes in FGF-2 and its receptor, FGF Receptor1 (FGFR1), have been described in the brains of SZ. Given the known role of FGF in development, this suggested that impaired FGF signaling may underlie abnormal

brain development and function associated with SZ. The inventors' studies led to the discovery of the Integrative Nuclear FGFR1 Signaling (INFS) locus that integrates several different pathways in which the SZ-linked mutations have been reported. INFS links them to transcription co-activator RNA Pol II activation and to chromatin remodeling. This implicates a disruption in the integrative FGFR signaling as the common pathological mechanism for the diverse SZ-linked genetic defects. A unique animal model has been engineered which shows that such a disruption targeted to developing DA neurons results in a disorder that is comprised of both the neurodevelopmental aspects and the clinical positive and negative symptoms of SZ that may be treated with anti-schizophrenia drugs. In th(tk-)/th(tk-) mice diminished FGFR signaling specifically in DA neurons results in DA neuron hypoplasia which is similar to SZ and is accompanied by hyperactivity of subcortical DA and hypoactivity of cortical DA systems.

Furthermore similar to the human illness, those changes are associated with impaired sensory gating [corrected by typical antipsychotic drugs (TAPD)], which likely underlies the positive SZ symptoms, and with diminished social interactions, which typify the negative symptoms.

Importantly these behavioral deficits appear to develop gradually in early adult life as observed in human SZ. The inventors' studies show that DA neuronal hypoplasia leads to remodeling of other neuronal systems resulting in multitransmitter brain rewiring akin to that proposed in SZ. th(tk-)/th(tk-) mice develop serotonergic hyperinnervation of both substantia nigra and the ventral tegmental area DA neuronal centers. This serotonergic rewiring contributes to the deficits in sensory gating, social interactions and cognition which are corrected by atypical antipsychotic drugs (AAPD) targeting 5HT-2A receptors. Similar to SZ and consistent with the established role of DA neurons in development of other neuronal systems, the th(tk-)/th(tk-) mice show hypoplasia and derangement of both cortical and hippocampal neurons. All the affected systems are known to express alpha7 nicotinic receptors and both the positive and the negative symptoms th(tk-)/th(tk-) mice are corrected by alpha7 nicotinic agonists, a new class of anti-psychotic drugs. In contrast, neither 5HT-2A antagonists nor the nicotinic agonists affect the behavior of normal mice. Hence the "rewired brain" of the th(tk-)/th(tk-) mice offers a unique model for developing and testing antipsychotic agents.

In summary, the th(tk-)/th(tk-) mouse model not only provides an experimental support for the developmental DA hypothesis of SZ, but also links it to alternative hypotheses which focus on other neuronal systems as being important in SZ. The obtained results provide a new insight into the complex multi-neurotransmitter etiology and symptomatology of SZ and suggest new therapeutic targets.

The pathogenesis of schizophrenia SZ likely stems from a multifactorial neurodevelopmental derangement as well an abnormal dopaminergic transmission. How those two deficits may be related has not been established. According to the DA hypothesis of SZ the positive symptoms of schizophrenia (hallucinations, delusions and impaired sensory gating) are associated with hyperactivity of the subcortical dopaminergic system(s) while the negative symptoms (flattened affect, social withdrawal) are caused by DA hypoactivity in the prefrontal cortex. This was corroborated by human PET studies showing that in neuroleptic-naïve SZ patients presynaptic DA synthesis capacity and amphetamine-stimulated DA release in the striatum are enhanced implying a deregulation and hyperresponsiveness of nigro-striatal dopaminergic neurons while the functions of DA neurons that innervate prefrontal cortex appear to be diminished.

Cell bodies of these DA neurons are located in the mesencephalic tegmentum mainly in the Substantia Nigra compacta (SNc; A9 cell group) and in the more medial ventral tegmental area (VTA; A10 cell group). While, A9 SNc to A10 VTA constitute a continuum whose projections overlap in several terminal areas the SNc predominantly innervates the dorsal striatum forming a nigrostriatal system and the VTA neurons project either to the nucleus accumbens (NAc) (mesolimbic system) or to the prefrontal cortex (PFC) (mesocortical system). In human patients with SZ and the related Asperger syndrome, a reduction in the midsagittal diameter of the mesencephalon was found and correlated inversely with the severity of symptoms as well as with exposure to neuroleptics. In SZ patients not subjected to neuroleptic treatments there is a significant decrease in the volume of the SN area (-21%), the nerve cell mean volume was diminished in the SNc (-15%) and VTA (-17%). In contrast, an enlargement of the striatum in SZ appears to be produced by antipsychotic medication. How the hypoplasia of DA neurons could paradoxically lead to DA hypertransmission in subcortical basal ganglia and concurrent hypotransmission in the frontal cortex remains unknown.

Although most, if not all, neuroleptic medications have DA receptor 2 (D2) blocking characteristics, the traditional antipsychotic drugs (TAPD) are not able to treat all clinical SZ defects and cause an unacceptably high incidence of extrapyramidal syndromes (EPS). The more recently introduced atypical antipsychotics (AAPD) often have a higher therapeutic efficacy on a wider range of clinical SZ deficits including positive as well as the negative symptoms and have a much lower propensity for EPS. This may be related to action on both the DA and serotonin (5HT) neurotransmitter systems. Drugs that effectively treat SZ symptoms are thought to reduce DA neurotransmission in brain regions in which it is hyperactive and increase DA neurotransmission in brain regions in which it is hypoactive. However, how 5-HT receptors antagonists exert their therapeutic effects has not been well defined. Furthermore, changes in other systems (cortical and hippocampal neurons) may also contribute to the SZ symptoms and thus could be targeted by therapeutic agents. In addition a new categories of drugs, nicotinic receptors agonists, have been developed that may be the most effective of all in treatment of SZ.

Consistent with high frequency (1%) of SZ in general population, multiple genetic links have been suggested

for this disorder. Of the particular interest are the proteins that may influence neural development via changes in cyclic AMP enzymes, MAPK signaling pathways and transcription co-activator complexes. In addition, epigenetic mechanisms (DNA and chromatin modifications) have been proposed to contribute to the complex patterns of inheritance and etiology of SZ. Recently, changes FGF 2 and its receptors FGFR1 have been described in the brains of SZ and bipolar patients, and the neuroleptic treatments were shown to increase FGF-2 expression suggesting that impaired FGF signaling could underlie abnormal brain development and function associated with these disorders.

The Integrative Nuclear FGF Receptor1 (FGFR1) Signaling, or INFS, integrates several different pathways in which the SZ-linked mutations have been reported, links them to transcription co-activator RNA Pol II activation and to chromatin remodeling, and is important in neural development. One common pathological mechanism for the diverse SZ-linked genetic defects is a disruption in the integrative FGFR signaling.

Th(tk-)/th(tk-) mice were engineered to express a tyrosine kinase deleted FGFR1. The genetic make-up of the animal model employed in the present method is described in Klejbor, et al. (J Neurochem 2006, 97, (5),1243-58, herein incorporated by reference) and is referred to herein as "th(tk-)/th(tk-)" mice. Th(tk)/th(tk-) mice were engineered to express a tyrosine kinase deleted FGFR1.

FGFR1(TK-) blocks the nuclear FGFR1 from activating CBP, RNA Pol II and histone acetylation and prevents activation of genes, neuronal differentiation and growth by cAMP and other signals. In addition, FGFR1(TK-) can dimerize with and inactivate plasma membrane FGF receptors, thereby affecting ERK and Akt signaling also implicated in the SZ25. The expression of the dominant negative FGFR1 (TK-) was targeted to developing postmitotic catecholamine neurons by the rat 4.5 kb tyrosine hydroxylase (TH) gene promoter. The onset of this promoter activity at E17 in differentiating midbrain neurons and its regional brain specificity mimic closely those of the endogenous TH gene. Using standard procedures, mice were obtained which transmit the FGFR1 (TK-) gene to their offspring. FGFR1(TK-) protein was detected in the brain stem and in the dissected SN. Little or no FGFR1 (TK-) was detected in the telencephalon (cortex and striatum) and in other brain regions which express no or low levels of TH (not shown).

There were no significant differences in body and brain weights between control and th(tk)/th(tk-) mice. Furthermore, no apparent changes in gross brain anatomy were found in th(tk-)/th(tk-) mice.

DA neurons:

In th(tk-)lth(tk-) mice diminished FGFR signaling resulted in reduced density and size of SNc DA neurons that form the nigrostriatal dopaminergic projection as well as hypoplasia of VTA DA neurons. This was shown using unbiased stereological counts of the TH-IR neuronal densities in the SNc and VTA nuclei. Statistically significant decreases were found in the density of TH-IR neurons in the SNc (-34%) and in VTA at postnatal day 1 th(tk-)/th(tk-) mice. The changes in SNc but not in VTA were maintained throughout life. Stereological measurements were performed which revealed that the average size of TH-IR somata in newborn (PD 0) th(tk-)/th(tk-) mice were reduced in both the SNc (-37%) and VTA (-20%) as compared to control mice. The TH-IR neurons remained smaller in adult (PD 360) th (tk-)/th(tk-) mice (-15% in SNc and -11 % in VTA).

Reduced density of DA transporter in the striatum further demonstrated an impaired development of the nigrostriatal DA system. Paradoxically, the th(tk-)/th(tk-) mice had increased levels of DA, homovanilic acid and 3-methoxytyramine in the striatum, indicative of excessive DA transmission. These structural and biochemical changes in DA neurons are similar to those reported in human patients with schizophrenia.

Serotonin Neurons:

Although DA appears to be the main neurotransmitter in SF, serotonin may play a significant role in the etiology of this disease. It has been hypothesized that the SF subjects who respond to clozapine may exhibit excessive serotonergic activity, although the nature of such potential changes has been unknown.

In order to examine whether there are alterations of the 5-HT systems in the th(tk-)/th(tk-) transgenic SZ model, brain regions were dissected and analyzed for 5-HT and its metabolite 5hydorxyindoleacetic acid (5-HIAA)s by HPLC-ECD in adult control and homozygous th(tk-)/th(tk) mice. Regions analyzed were those which shared both DA and 5-HT systems, and included both terminal fields (striatum, frontal cortex, nucleus accumbens, hypothalamus) and the somal origin (VTA, SNc, dorsal raphe nucleus) of these two neurotransmitter systems. 5HT levels in the striatum, nucleus accumbens, frontal cortex, and hypothalamus were not significantly different between control and th(tk-)/th(tk-) mice. Nevertheless, in the SN, the 5-HT levels in transgenic mice showed approximately 70%, statistically significant increase compared to control mice. A similar trend was observed in VTA, and was accompanied by a statistically significant increase in 5-HIAA levels. In contrast to the midbrain nuclei, in the pontine raphe region both 5HT and 5-HIAA levels in th(tk-)/th(tk-) mice were reduced significantly compared to controls.

In order to examine cellular mechanisms underlying the observed 5HT/5HIAA changes in the ventral midbrain, an anti-5-HT immunohistochemistry was performed. This revealed positively stained fibers and puncta in the ventral

midbrain in both control and th(tk-)/th(tk-) mice. Examined structures included: the ventral tegmental area (VTA) and substantia nigra (SN) its subdivisions: the interfascicular nucleus (IF), the parabrachial pigmentosus nucleus (PBP), the paranigral nucleus PN, the rostral linear raphe nucleus (RLi); compact and reticular parts of the substantia nigra (SNC and SNR, respectively). In general, all subnuclei of the VTA had 5-HTimmunoreactive fibers, but the appearance and the densities of those fibers differed. In the IF nucleus,a dense network of varicose, short, and small diameter fibers was observed. Stereological analysis revealed no significant differences in the density of the 5-HT-ir fibers in IF between control and th(tk-)/th(tk-) mice.

In the PBP nucleus, a network of 5-HT-ir fibers that followed an irregular course was observed. Some of these fibers were relatively long and running parallel to each other along the nucleus. In addition in the th(tk-)tth(tk-) but not in controls, long, smooth (thin) fibers without varicosities were found. Quantitative analysis showed that the overall density of the 5-HT-ir fibers in PBP nucleus was 2-fold higher in the th(tk-)lth(tk-) mice than in controls ($p<0.00001$).

In the PN, numerous 5-HT-ir puncta and a dense network of 5-HT-ir fibers were present. Long fibers with numerous small varicosities, running along the nucleus were predominant in both tested groups of animals. Moreover, in transgenic mice this nucleus showed group of the fibers which were absent in the control mice. These were long, smooth thin fibers without varicosities and vertically oriented. Quantitative analysis showed that the overall density of the 5HT-ir fibers in PN nucleus was approximately 1.5-fold higher in the th(tk-)/th(tk-) mice than in controls ($p<0.005$).

In RIi single 5-HT-ir fibers following vertical course were found. RIi contained relatively small numbers of the 5-HT-ir puncta. There was no difference in the appearance of these elements between control and th(tk-)/th(tk-) mice. Also qualitative analysis showed no significant differences in fiber densities between the control and transgenic mice.

In the pars compacta region of the substantia nigra both the 5-HT-ir fibers and puncta are visible. There was no significant difference in the 5-HT-ir fiber densities in SNc between control and th(tk-)/th(tk-) mice.

In comparison with the SNc, the SNr displayed significantly higher density of the 5-HT-ir fibers in both groups of mice. These fibers run in diverse directions. In the SNr quantitative analysis showed significant 68% increase in the density of 5-HT-ir fibers in the transgenic animals.

Thus, quantitative anatomical analyses showed that th(tk-)/th(tk-) mice had significantly greater numbers of the 5-HT fibers in PN and PBP nuclei of the VTA which project principally to the prefrontal cortex and nucleus accumbens, as well as within the SNr region, when compared to the control mice. In th(tk-)/th(tk-) mice the hyperinnervating serotonergic axons formed dense networks of the 5-HT-immunoreactive fibers with numerous varicosities, some having different form than observed in the control mice. The invasion of 5-HT terminals was corroborated by increased levels of 5-HT in the ventral midbrain regions of the th(tk-)/th(tk-) mice.

Neither 5HT nor 5HIAA were increased in terminal fields of DA neurons: striatum, nucleus accumbens or frontal cortex. Thus, DA neurons may be affected by an increased 5-HT tone in the midbrain nuclei, rather than indirectly via serotonin control of the telencephalic projections into the ventral tegmentum. Neither the 6-hydroxydopamine induced lesion of DA neurons in adult rat nor the loss of SN DA neurons caused by FGFR1 (TK-) transfection into an adult brain (unpublished observations) led to serotonergic hyperinnervation of striatum or the ventral midbrain, respectively. Thus, the serotonergic hyperinnervation of SN and VTA in th(tk-)/th(tk-) mice may represent a developmental response to the hypoplasia of DA neurons in these brain regions.

<u>Hippocampal and prefrontal/frontal cortical neurons</u>

Cortical layer disruption and reduced size of the cortical and hippocampal neurons have been reported in SZ patients. In agreement with these reports, in th(tk-)/th(tk-) mice double staining with neuron-specific NeuN antibodies and DAPI (DNA) revealed increased density of neuronal somata in the pyramidal and granular hippocampal layers and disruption of cortical layers in the frontal/prefrontal limbic cortex.

The anatomical and neurochemical characteristics of th(tk-)/th(tk-) mice and their correlation with the SZ pathology may be summarized as:

1. Th(tk-)/th(tk-) mice have underdeveloped and hyperfunctional DA neurons innervating in basal ganglia. In contrast DA projection to frontal cortex may be hypofunctional;
2. The underdevelopment of DA neurons has a secondary effect on development of serotonin neurons causing a serotonergic hyperinnervation of the hypoplastic DA neurons;
3. Neuronal malformations in the prefrontal cortex (layer disruption, neuronal displacement and paucity) in th(tk-)/th(tk-) mouse which are similar to the cortical changes observed in human SF. These cortical changes are likely to be secondary to the changes in DA neurons. This is consistent with the role of DA as neurogenesis-controlling factor. It also supports the hypothesis that DA neuronal hypoplasia affects development of other neuronal systems and creates an abnormal brain circuitry as proposed in SF; and
4. Changes in DA transmission develop in adolescence.

Behavioral deficits in mice are akin to the positive, negative and the cognitive deficits in SZ. Positive symptoms of SZ are typified by hallucinations, delusions and associated deficits in sensory gating. Prepulse inhibition (PPI) as a measure of sensorimotor gating and information processing refers to the attenuation of the startle response by a weak stimulus (prepulse) appearing a short time prior to the startle stimulus (Vollenweider F.X., et al., Biol Psychiat 2006,60,597-603, herein incorporated by reference with regard to such teaching).

Deficits in prepulse inhibition (PPI) occur in SZ (31, 32). Both TAPD and AAPD improve PPI (Kumari V, Sharma T; Psychopharmacology 2002, 162,97-101, review, herein incorporated by reference with regard to such teaching). Recent studies suggest that AAPD may be superior to TAPD in normalizing PPI deficits in SZ relative to healthy controls (Kumari et al., 1999 from Vollenweider). Compared to controls, th(tk-)/th(tk-) mice had reduced prepulse inhibition and enhanced startle responses. These changes developed gradually between 1 and 4 months of life and remained stable at least until 14 months of age.

In addition to the impaired sensory gating and associated hallucinations and delusions, SZ is characterized by negative symptoms (flattened affect, social withdrawal) which are typically more resistant to pharmacological treatment. These symptoms have not been successfully modeled in genetically altered animals. To determine whether th(tk-)/th (tk-) mice exhibit negative-like symptoms, their social and non-social investigative behavior was observed.

There was a significant difference in investigatory behavior between control and th(tk-)/th(tk-) subjects. Wild-type controls spent significantly ($p<0.05$) more time investigating a female and a male stimulus animal than did th(tk-) /th(tk-) mice. Other measures of investigatory behavior (e.g. anogenital investigation) showed the same pattern. In contrast there were no differences between control and transgenic mice in a non-social behavior, auto-grooming. In the open-field test, TK- mice traveled a significantly greater distance in both the peripheral and central zones than wild-type mice.

SZ is also characterized by cognitive impairments which are typically resistant to pharmacological treatment. The presence of possible cognitive symptoms in th(tk)/th(tk-) mice was analyzed by radial maze and object recognition tests.

In the radial arm maze experiments, th(tk-)/th(tk-) animals made more errors and took longer to find the food items on testing days 1 and 2 (differences are statistically significant). By testing day 3, there was no genotypic difference. This indicates that there is a deficit in th(tk)/th(tk-) in learning, memory, or both. The deficit is consistent with a problem with working memory.

In the Object Recognition test the th(tk-)/th(tk-) animals behaved differently than WT animals. th(tk-)/th(tk)mice spent a similar amount of time investigating the familiar and novel object whereas WT mice spent significantly more time investigating the novel object.

One interpretation is that the th(tk-)/th(tk-) mice do not remember which object is novel and which is familiar.

Compared to SZ, the th(tk-)/th(tk-) mouse is a unique model that mimics the complex neurodevelopmental, structural, and functional characteristics ("positive", "negative" and "cognitive impairment" symptoms of human SZ and links them to, but elaborates on the "DA Hypothesis of Schizophrenia". This model offers new and unique insights into human diseases and should facilitate new therapeutic strategies.

The reduced prepulse inhibition in th(tk-)/th(tk-) mice was normalized by treatment with the TAPD (DA receptor antagonist) flupenthixol at doses that did not effect startle amplitude. The PPI data were analyzed using a 3 factor mixed ANOVA with group [FGFR1(TK-), Control] as a between subject variable and stimulus intensity (pp4, pp8, & pp16) and drug dose (saline, 0.25, 0.5, & 1.0 mg/kg) as within subject variables. This analysis produced a significant two way interaction between dose and group ($F3, 72=3.11$, $P<0.05$) and a significant three-way interaction between dose, group and stimulus intensity ($F6, 72=2.85$, $P<0.05$). There was a significant interaction between group and stimulus intensity ($F2,14=14.93$, $P<0.01$). Follow up t-tests at each of the three stimulus intensities revealed significant reductions in PPI at each of the three stimulus intensities ($pp4 = P < 0.1$; $pp8 = P<.05$; & $pp16 = P < 0.01$) in the th(tk-)/th(tk-) group. A two factor ANOVA with dose and stimulus intensity as factors revealed a significant effect of dose in the th(tk-)/th(tk-) mice ($F3,42=29.92$, $P<0.01$) but no effect of dose in the control group. All three doses of flupenthixol increased PPI in the th(tk-)/th(tk-) mice at all three stimulus intensities ($P<0.01$).

In startle response, there was a significant effect of both group ($Ft ,11=21.76$, $P<0.01$) and dose ($F3,33=3.99$, $P<0.05$) in the th(tk-)/th(tk-) mice. Follow up t-tests indicated that compared to saline, startle response was decreased by flupenthixol at the 1.0 mg/kg dose of flupenthixol in both the th(tk-)/th(tk-) and control mice.

Although DA appears to be the main neurotransmitter in SZ, serotonin may play a significant role in the etiology of this disease. Clozapine and related atypical antipsychotic drugs (AAPD) have high affinity for 5HT2A29 30 and increase 5HT brain levels. The high 5HT2A occupancy rate by AAPD is associated with their favorable antipsychotic treatment effects. In contrast there was no association between AAPD anti-psychotic efficacy and D2 occupancy.

Schmidt et al (1993) indicated that 5HT2A antagonism may have a corrective effect on impaired sensory gating, and that schizophrenics who respond to clozapine exhibit excessive serotonergic activity. This hypothesis fits with data in patients in which some indices initial of enhanced central serotonin tone were found and appeared related to clinical efficacy of AAPD (Kasper et al., 1999; Martin et al., 1998; Wandenberg et al., 2001; each of which is herein incorporated

by reference). 5-HT neurons originating in Dorsal Raphe nucleus (DR) and in the Main nucleus of Raphe (MnR) innervate DA terminal region (e.g. prefrontal cortex and striatum) as well as the midbrain structures including VTA and SN where they make synaptic contact with DA and non-dopaminergic neurons.

Given the invasion of SN and VTA by serotonergic neuronal terminals in the th(tk-)/th(tk-) mice,the effects of AAPD (clozapine and quetiapine) and a specific 5-HT2A antagonist M100907 were analyzed.

Clozapine at 3 mg/kg had no effect on either PPI or startle response. In contrast, at higher dose (6 mg/kg), clozapine significantly increased PPI in both control and transgenic mice and decreased startle response in transgenic mice. There were no significant genotype x drug interaction found at either dose of clozapine or at any prepulse intensity within each dose indicating treatment with clozapine had a similar effect on PPI in control and th(tk-)/th(tk-) mice, although there was a significant decrease of PPI in transgenic mice as compared to the appropriate control group at each prepulse intensity at each dose. These results were consistent with the human finding that the AAPD clozapine also increased PPI in healthy individuals in a manner comparable with those in SZ patients. The PPI for the transgenic 6 mg/kg clozapine group did not differ from that of the control vehicle group (t-test: pp4; pp8; pp16 = $p > .05$, NS) demonstrating the ability of clozapine to normalize PPI in the transgenic mice. A similar normalization was seen with the startle response

In th(tk-)/th(tk-) mice, quetiapine normalized the reduced PPI at a dose of 7.0 mg/kg ($p < .001$) while lower doses had no effect. In control mice there was no significant main effect of quetiapine at any dose examined. For transgenic mice there was a significant decrease in startle response compared to saline group at all doses of Quetiapine ($p < 0.05$). In contrast, there was no main effect of Quetiapine on startle response at any dose in control mice.

To ascertain that the effects of AAPD may reflect specifically the inhibition of serotonin receptors, a specific 5-HT2A antagonist M100907 was tested. M100907 had no effect at any dose on PPI or startle response in control mice. The transgenic low dose (0.01 mg/kg) M100907 group showed significantly lower PPI than the control low dose group, similar to the difference seen in vehicle treated groups. There was no significant difference between transgenic and control groups at either the middle (0.1 mg/kg) or high dose (1 mg/kg), and at the high dose there was a significant drug x genotype interaction indicating that M100907 at 1.0 mg/kg preferentially improved PPI in the transgenic mice. At the middle and high doses, M100907 resulted in increases of PPI in transgenic mice as compared to the vehicle group. Although there was no significant difference between the control and transgenic groups at any M100907 dose, there was a significant decrease of startle response in the treated transgenic groups, as compared to the vehicle treated group.

Two of the hallmark negative symptoms of SZ are a lack of social interaction and flattened affect. M100907 significantly increased the amount of time TK- mice spent investigating the stimulus animal but had no effect on investigation time in wild-type animals. In contrast to the social withdrawal, the auto-grooming behavior and movement in the open field were unaffected by drug treatment.

These results indicate that th(tk-)th(tk-) mice exhibit behavior that is analogous to the negative symptoms of SZ. Social investigation is reduced in th(tk-)/th(tk-) mice but treatment with M100907 reverses this deficit. M100907 has no effect in wild-type mice, suggesting that the serotonergic system differs functionally between wild-type and th(tk-)/th (tk-) mice. In patients with SZ, deficits in social behavior are not reversed by typical antipsychotics and are resistant to treatment by many atypical antipsychotics. The observation that M100907 reversed the social deficits in th(tk-)lth(tk-) mice without affecting social investigation in wild-type mice suggests that drugs that target the 5-HT2A receptor may ameliorate negative symptoms of SZ patients without affecting social behavior in the non-SZ population. The facilitatory effect of M100907 on social investigation appears relatively specific since non-social behavior such as autogrooming and locomotion in the open-field were unaffected by the drug.

The th(tk-)/th(tk-) mice provide an experimental support for the 5HT hypothesis of SZ which proposes that the good response to AAPD (5HT2A antagonists) depends on an enhanced central serotonergic activity and that the efficacy of those drugs correlates with the degree of the increase in serotonergic tone. Unmedicated paranoid SZcs in the early phase of disease in which they respond well to selective 5HT2A antagonism have an enhanced central serotonin tone suggested by increased fenfluramine-induced prolactine response and by higher SHIAA levels in CSF than controls (Bartfai et al., 1984; Rimon et al., 1971) (Abel et al., 1996; each of which is incorporated by reference with regard to such teaching). In SZcs the best predictor of favorable response to clozapine is a low CSF ration of HVA/5HIAA, a higher 5HT turnover relative to DA (Pickar et al, 1994; Szymanski et al., 1993; each of which is herein incorporated by reference with regard to such teaching). The favorable clozapine response has been consistently predicted by paranoid SZ (Fenton and Lee 1993), a subtype of disease associated with increased brain levels of 5HIAA (Hanson et al., (1994), each of such references herein incorporated by reference with regard to such teaching.

One hypothesis regarding how serotonergic hyperinnervations affects functions of DA neurons and behavior in th(tk-)/th(tk-) mice is that 5HT hyperinnervation stimulates DA neurons in SN and inhibits VA by acting through 5HT2A receptors.

The impaired PPI and increased startle in th(tk-)/th(tk-) mice were shown to reflect hyperactivity of DA neurons. Thus, one mechanism through which 5-HT hyperinnervation of SN and VTA could impair the sensory gating could involve 5-HT overactivation of DA neurons that innervate subcortical targets. In contrast, the negative symptoms may reflect DA hypofunction in the frontal cortex. 5-HT is known to have opposite effects on DA neurons in SN (predominantly

activatory) and in VTA (predominantly inhibitory). Thus blocking 5-HT2A receptors could normalize DA function in both the subcortical and cortical DA systems.

Serotonergic activity can influence the DA neurons activity in both SN and the VTA. In both rats and humans, 5-HT2A receptors have been localized in the SN and VTA, providing a mechanism by which M100907 could affect DA neurons. Since the drug only had an effect in th(tk)/th(tk-) mice, the role of the 5-HT2A receptor in normal behavior may be subtle. However, in a condition in which serotonergic hyperinnervation occurs, as in th(tk-)/th(tk-) mice, the drug is effective at revering the behavioral changes associated with this hyperinnervation. A similar situation likely exists in human SZ brain in which both DA neuronal hypoplasia (which in mice triggers serotonergic hyperinnervation) and overproduction of serotonin are observed.

MATERIAL AND METHODS

Transgenic th(tk-)tth(tk-) mice were described in Klejbor et al., 2006, herein incorporated by reference with regard to such teaching. The mice were generated by introducing a fusion gene consisting of rat TH promoter (4.5 kb) fused to FGFR1 (TK-) arranged in series with FGFR1 (TK-), the SV40 splice donor-acceptor site and the SV40 poly(A) sequentially downstream (total 6.5-kb). The progenies were screened for the presence of the transgene by PCR amplification of tail DNA for 30 cycles. Sense (GCCAAGACAGTGAAGTTCAAATGC) and antisense (GTAATACGACTCACTATAGGGC) PCR primers were complementary to the transgene regions (Klejbor et al., 2006). All transgenic and control mice used for the experiments described below were male and female F2 animals of the mixed genetic background of BCF1 (C57BL/10J/C3H/HeJ). Adult mice (homozygous, heterozygous or wild-type) were housed on a light:dark cycle of 12:12 h (lights of at 1200 h) with free access to food and water. All behavioral and anatomical procedures were carried out in accordance with the NIH Guide for the Care and Use of Laboratory Animals and with approval from the University at Buffalo IACUC. All efforts were made to minimize animal stress and to reduce the number of mice used for the behavioral and anatomical experiments.

Clozapine (RBI/Sigma St. Louis, MO) and Quetiapine (AstraZeneca) were dissolved with 5 μl of 20% acetic acid/ml of 0.9% saline.

M100907 (K. Rice), was dissolved in phosphate buffered 0.9% NaCl. Drug doses were calculated as free base.

Compounds A and B (Targacept Inc; Winston Salem, NC) and Flupentixol (RBI/Sigma St. Louis, MO) were dissolved in phosphate buffered 0.9% NaCl. Drug doses were calculated as free base.

Drugs or vehicle were injected subcutaneously 30 minutes i.p. before the behavioral testing. All injections were given at a volume of 100- 200 μl/ 30 g of body weight.

<u>Behavioral Methods</u>

A. PPI and Startle:

Apparatus: Startle reactivity was measured using two chambers (SR-LAB, San Diego Instruments, San Diego, CA). Each chamber consisted of a clear nonrestrictive Plexiglas cylinder resting on a platform inside a ventilated box. A high-frequency loudspeaker inside the chamber produced both a continuous background noise of 68 dB and the 120 dB startle pulse. Vibrations of the Plexiglas cylinder caused by the whole-body startle response of the animal were transduced into analog signals by a piezoelectric unit attached to the platform.

Prepulse inhibition (PPI) session: All PPI test sessions consisted of startle trials (pulse-alone), prepulse trials (prepulse + pulse), and no-stimulus trials (nostim). The pulse-alone trial consisted of a 40 ms 120 dB pulse of broadband noise. PPI was measured by prepulse + pulse trials that consisted of a 20 msec noise prepulse, 100 msec delay, then a 40 msec 120 dB startle pulse (120 msec onset-to-onset interval). The acoustic prepulse intensities were 4, 8, and 16 dB above the 68 dB background noise (i.e., 72, 76, and 84 dB). The nostim trial consisted of background noise only. The test session began and ended with five presentations of the pulse-alone trial; in between, each acoustic or nostim trial type was presented 10 times in a pseudorandom order. There was an average of 15 sec (range, 12-30 sec) between trials. For the drug studies, the mice were placed into the startle chambers 30 minutes after each injection, and a 68dB background noise level was presented for a 10 min acclimation period and continued throughout the test session.

The amount of PPI was calculated as a percentage score for each acoustic prepulse trial type: % PPI = 100 {[ (startle response for prepulse + pulse)/(startle response for pulse-alone)] x 100}. The magnitude of the acoustic startle response was calculated as the average response to all of the pulse-alone trials, excluding the first and last blocks of five pulse-alone trials presented.

Procedure: In all cohorts, PPI was tested twice per week with at least two days separating testing days for all drug doses. Each week the mice received a vehicle injection before one of the test sessions and drug treatment for the second test session. PPI and startle magnitude on vehicle test days were examined to determine if these measures changed with repeated testing. Since no effect of repeated testing was observed the average of the vehicle and the drug

test sessions (at each dose) were used for analysis. The order of saline/non-injection and drug injections was changed each week. Data for non-injected groups was collected before any treatment was administered. The number of animals tested for each drug varied and is indicated in the Results. For all experiments, mice were tested between 5 and 12 months of age and there was an equal distribution of gender within each genotype.

Statistics: The PPI data were analyzed using a three-factor mixed ANOVA with group [FGFR1 (TK-), control] as a between-subject variable and prepulse stimulus (pp) intensity (pp4, pp8 and pp16) and drug dose as within-subject variables. To determine whether there was a difference between the groups without drug treatment, the non-injection and saline data were analyzed with a mixed, two-factor ANOVA with group and stimulus intensity as factors. Drug doses analyzed were saline vehicle, flupenthixol (0.25 mg/kg, 0.5 mg/kg and 1.0 mg/kg), clozapine (3.0 mg/kg and 6.0 mg/kg), quetiapine (1.0 mg/kg, 2.0 mg/kg, 3.0 mg/kg and 7.0 mg/kg), M100907 (0.1 mg/kg, 0.3 mg/kg and 1.0 mg/kg), Compound B (0.1 mg/kg and 1.0 mg/kg), Compound A (0.1 mg/kg and 0.3 mg/kg) and combined doses of clozapine (3.0 mg/kg) and Compound A (0.1 mg/kg), and quetiapine (3.0 mg/kg) and Compound A (0.1 mg/kg). Follow up t-tests were conducted at each of the three stimulus intensities (pp4, pp8 and pp16) to determine significant difference between groups. To determine whether treatment differentially increased PPI in the th(tk-)/th(tk-) mice, a two-factor ANOVA with dose and stimulus intensity as factors was performed on both the th(tk-)/th(tk-) and control groups separately. Startle response was analyzed with a mixed two-factor ANOVA with group and dose as factors and follow up t-tests were conducted in the same manner as PPI. Statistical significance for all tests was $p < 0.05$.

B. Social behavior, nonsocial autogrooming, and open field activity were measured in wild-type (n=7) and FGFR1(TK-) (n=7) male mice between the ages of 7 and 10 months that had been singly housed for at least four weeks before testing. On testing days, each subject received three tests: a social behavior test with a female, a social behavior test with a male, and an open-field test. There was a 30-minute interval between each test. Social behavior was tested using a variant of the resident-intruder paradigm, in which a stimulus animal was introduced into the subject's homecage for three minutes. Prior to testing the subjects' home cages were not changed for at least four days to allow them to establish the cage as their territory. Female stimulus animals were singly-housed, randomly cycling C57Bl/6Js (Jackson Laboratories, Bar Harbor, ME). Each subject was tested with a different stimulus animal and each stimulus animal was used only once per testing day. Male stimulus animals were singly-housed C57Bl/6Js (Jackson Laboratories, Bar Harbor, ME). Each subject was tested with a different stimulus animal and each stimulus animal was used only once per testing day. A stimulus female was placed into the cage of wild-type (n=8) and tk-/ (n=8) subjects. After three minutes, the stimulus female was removed. Thirty minutes later a stimulus male was placed into the subject's homecage. After three minutes, the stimulus animal was removed. All testing occurred in the dark-phase (the active phase) of the light cycle under red-light illumination. The interaction was videotaped from the side using the NIGHTSHOT feature on a Sony video camera (DRV-120, Sony Corporation). The behavior of the subject was quantified from the videotape using the Observer Mobile (Noldus Information Technologies, Sterling, VA). The number of bouts observed and the amount of time engaged in the following behaviors was measured: general social contact (contact with the stimulus animal, sniffing (both anogenital and non-anogenital contact), and non-social behavior (autogrooming). The person scoring the behavior was blind to the genotype and treatment of the subjects. To test open-field activity, the subjects were removed from their home cage and placed alone into a clean Plexiglas open-field testing arena (45cm x 45 cm x 25 cm) for a ten minute testing session, after which they were returned to their home cage. The test was videotaped from above using a SONY TRV-350 Handycam videocamera using the nightshot feature. Movement was analyzed in detail using the Clever Sys. Inc. system.

For drug testing, the wild-type (n=7) and tk-/- (n=7) were injected i.p. with 1 mg/kg of M1009007 or vehicle. Thirty minutes later, a stimulus female was placed in the subject's homecage. After three minutes, the stimulus female was removed. Thirty minutes later a stimulus male was placed into the subject's homecage. After three minutes, the stimulus animal was removed. After 30 minutes, the subject was tested in the open-field test.

C. Radial Arm Maze: Deficits in working memory are present in human patients with SZ and psychological disorders that are associated with other deficits in social behavior. Briefly, mice are placed in the center of an eight-arm radial maze and allowed to explore the maze freely. Subjects are tested no more than once per day. There are two training phases. Phase 1 - During the first training phase, a palatable food item (such as a piece of a Cheerio) is placed at the end of each of the eight arms. The test continues until the animal has found all the food items or twenty minutes have elapsed. The test necessitates that the subjects have motivation to find food. Therefore, the subjects are food restricted for approximately 12 hours, from about 8pm until the time of testing, about 8am. In the time between the end of testing and 8pm, the subjects have ad libitum access to food. Whenever in their home cages, the subjects always have ad libitum access to water. All subjects are weighed daily. Any subject that loses more than 15% of its initial body weight is removed from the study and provided ad libitum food. Once the animal has learned to retrieve the food from all the arms, the second phase of training will begin.

Phase 2 - In this phase, food is placed in four out of the eight arms. After several training sessions in this condition, the testing phase begins During testing, food is placed in four of the eight arms. The subject is placed in the center of the maze and allowed to explore the maze freely. The test ends when the subject has consumed all the food

items or 20 minutes have passed. The number of entries into empty arms is thought to reflect spatial memory. The number of entries into arms already investigated during the current test is thought to reflect working memory.

D. Object Recognition: Four different objects were used: copper thimbles, ¾ inch steel hex nuts, 25 m L glass flasks and plastic brain jars. For each object, there were three identical copies (i.e. brain jar 1, brain jar 2, and brain jar 3).

Testing: The subjects were tested between 10am and 3pm in the dark under red light illumination. The testing chamber is a large Plexiglas box (40 x 40 x 40) with an opaque floor. All tests were recorded using a Sony Handycam (DRV120, Sony Corporation, Oradell, NJ) camera using the Nightshot feature. The chamber and objects were thoroughly washed with 95% ethanol and allowed to dry for five minutes prior to testing. Testing was divided into two phases, the acclimation phase and object recognition phase.

Phase 1 - The acclimation phase occurred on days one to three of testing. The subject was placed in the center of the open field and allowed to explore freely for 10 minutes. This occurred once daily for three days.

Phase 2 - The object recognition phase occurred on day's four to five of testing. In this phase, two identical copies of object were placed in adjacent corners of the open field (i.e. brain jar 1 and brain jar 2). Consistent placement of the objects was ensured by measuring with a 5x5cm piece of cardboard. The square was placed in the corner and the center of the object then placed underneath the corner closest to the center of the chamber. The subject was placed in the center of the chamber and allowed to explore freely for three minutes. After three minutes, the subject was returned to its home cage for 30 minutes. During this delay, the testing chamber and objects were again cleaned with 95% alcohol. After the delay, one of the original objects was returned to the same location it was initially in. Then a new object replaced the other copy of the original object. For example, if brain jar 1 and brain jar 2 were initially used, one of these was returned (i.e. brain jar 1) and the other was replaced by a new object (i.e. steel nut 1). After these objects were set, the subject was again placed in the center of the chamber and allowed to explore freely. After three minutes, the subject was returned to its home cage for the day. For each phase, the object used or the object returned were randomly determined. This same procedure was used on day five of testing but using the other objects. Therefore, if the brain jar and steel nut were used on day four, the copper thimble and glass flask are used on day five.

4. Immunocytochemistry and stereology

Immunocytochemistry and stereology of TH expressing neurons and DA fibers are from (Klejbor et al. 2006) and are described below.

A. TH neurons - Mice were perfused with PBS and 4% paraformaldehyde and 40 micron cryostat brain sections were prepared and immunostained with rabbit polyclonal TH antibody (1:1000) (Sigma Chem. Co) and Cy3 conjugated anti-rabbit antibody (1:600) as previously described (Fang et al. 2005). Quantitative stereologic analysis was performed using the GASTGrid system (Olympus, Denmark). The system consists of a computer with a graphic interface and a BX-51 microscope (Olympus, Japan). The primary aim of this counting method was to compare neuronal profiles of transgenic and control mice and not to determine the absolute numbers of THIR neurons in the midbrain. Five mice in each group were analyzed. In all cases, immunoreactive neuronal profiles were analyzed within the tested fields using identical protocols consisting of: (1) outlining the nuclei (SNc and VTA) under low magnification; (2) random sampling under 20x magnification of SNc or VTA areas using the same antero-posterior sequence (5 sections from each brain from -4.52 to -5.6 relative to Bregma); (3) determining TH-IR neuronal density within the tested fields of known surface areas (at least 60% of the nuclear surface for SNc and 100% for VTA). The raw data from the individual tested fields were recorded and weighed and the mean density of TH-IR neurons was catculated for each nucleus. The density of TH-IR cells in control and transgenic mice were compared using ANOVA (Kruskal-Wallis test).

In order to estimate the mean TH-IR cell surfaces, the sampling grids that were laid systematically by the computer over the sectional profiles of the SNc and VTA for the cell counting were used to obtain unbiased estimates of the average TH-IR cell surface by means of Olympus Laser Pix version 4.1 (Biorad, Great Britain) software. One-way ANOVA with Newman-Keuls and Mann-Whitney post-hoc tests was applied for statistical significance between groups. All cell density and size measurements were conducted blindly with respect to the genotype.

B. DAT immunostaining and quantitative analysis. 40 micron brain cryostat sections were immunostained with rat anti-DAT antibody (Chemicon, Temecculla, CA) and Alexa488 conjugated anti-rat antibody. Fluorescent microscopic images (12-bit) were acquired with a XILLIX Microimager cooled CCD camera on a Nikon FXA fluorescent microscope. All images acquired were in the linear range of the camera. Images of sections from control and transgenic mice were acquired under identical illumination conditions and identical camera gain, offset, and exposure times. Background images (outside the tissue sections) were taken for each section and were subtracted from the images

of DAT-IR brain sections (striatum and nucleus accumbens). For each brain and brain structure, 3-4 representative sections were analyzed. Images were preprocessed and thresholded identically using ONCOR Image software. The total area of each image was recorded as well as the number of thresholded pixels and their integrated intensity.

C. Immunocytochemistry and stereology of 5-HT expressing fibers:

All adults animals (6 control and 6 transgenic mice) were deeply anesthetized with lethal doses of Nembutal (80 mg/kg of body weight), then perfused transcardially with 0.9% solution of saline (NaCl) with heparin, followed by 4% paraformaldehyde solution in 0.1 M phosphate buffer (pH 7.4). The brains were postfixed in 4% paraformaldehyde fixative for 3-4 hours. Then, they were placed in 15% sucrose (overnight at 4oC) followed by 30% sucrose until sunk. Coronal 40-μm thick sections of the brains were cut on JUNG 1800 cryostat (Leica, Germany). The sections were then stained with use of immunohistochemical method. The free floating sections were blocked with 10% normal goat serum (NGS) containing 0.3% Triton X-100 for 1 hour and then incubated with anti-5-HT rabbit polyclonal primary antibody (Sigma; 1:1000) for 48 hours in 4oC. After multiple rinses in PBS, sections were incubated for 2-3 hours, at room temperature with the Cy3 conjugated goat anti-rabbit (Jackson ImmunoResearch; diluted 1: 600) appropriate secondary antibodies: The chosen set of brain sections of both experimental as well as control groups underwent negative control with omission of primary antibody. For subdivision of the midbrain structures criteria of Paxinos and Watson (lit.) were used. Examined structures included: the ventral tegmental area (VTA) and substantia nigra (SN) its subdivisions: the interfascicular nucleus (IF), the parabrachial pigmentosus nucleus (PBP), the paranigral nucleus PN, the rostral linear raphe nucleus (RLi); compact and reticular parts of the substantia nigra (SNC and SNR, respectively).

The immunohistochemically stained slides were examined by a fluorescent microscope BX-51 (Olympus, Japan) and the confocal system Radiance 2100 (Bio-Rad, UK), equipped with Krypton/Argon laser and mounted on the light microscope Eclipse 600 (Nikon, Japan). The confocal laser scanning microscopy images (CLSM) were obtained using x40 and x60 oil immersion objective lenses of N.A.=1.3 and 1.4, respectively. The optimal iris was used for each magnification. For the reconstruction of the image analysis program Laser Sharp 2000 v.4.0. (Bio-Rad; UK) was used. In each case only sections completely stained with fluorescence were taken into account.

Quantitative stereologic analysis of 5-HT fibers was performed using the C.A.S.T. Grid system (Olympus, Denmark) as described by Gundersen H.J. and West M.J. 1988 (9103, 9085). One-way ANOVA with Newman-Keuls and Mann-Whitney post-hoc tests was applied for statistical significance between groups. All fibers density measurements were conducted blindly with respect to the genotype. Thirty sections per each nucleus of the VTA and SN in both groups control and transgenic 12 months old mice were studied.

These sections were distributed over the range from about -4.8 to -5.6 mm of Bregma.

Analysis of 5-HT, 5-HIAA, DA, DOPAC, HVA, 3-MT (HPLC with electrochemical detection - ESA system).

The mice were sacrificed using CO2, quickly decapitated and the brains were frozen on dry ice and stored at -80oC. The brain anatomical regions were isolated using punching needles as described previously (Bialowas et al. 1979). For each mg of tissue collected, 4 to 20 L of 50 mM perchloric acid containing 100 uM metabisulfite and 500 nM DHBA as an internal standard was added. The analyses were performed as described in (Corso et al. 2005). Briefly, the tissues were sonicated and the homogenate was centrifuged at 11,000 rpm (7500g) for 20 minutes in a microcentrifuge. The supernatant was placed in an ultra-free MC 0.22 m centrifugal filter unit and centrifuged at the same speed until the supernatant had passed through the filter. The samples were injected through a Suppleco Discovery C18 reverse phase 15 cm column with a 2 cm guard column. Detection was done with either BAS LC-4C or an ESA Coulochem II with ESA computer analysis software. Concentrations of the analyzed tissue 5-HT and its metabolite 5-HIAA as well as DA and its metabolites DOPAC and HVA were determined by HPLC analyses of their respective standards.

Statistical analysis: ANOVA followed by LSD.

THERAPEUTIC EFFECTS OF ALPHA7 NICOTINIC AGONISTS IN TH(TK-)/TH(TK-) MICE

Using th(tk-)/th(tk-) mice model described hereinabove, the hypoplastic development of DA neurons (also found in SZ) affects the development of other neuronal systems thereby creating an abnormal brain circuitry with defective sensory-gating, social behavior and cognition as proposed in SZ. The serotonergic hyperinnervation of the hypoplastic DA neurons and 5-HT hyperfunction supports this hypothesis. In addition, in th(tk-)/th(tk-) mice, structural changes in brain cortex and hippocampus that mimic changes reported in SZ were found. Targeting these diverse brain areas with new treatments could be tested as new therapy for SZ.

5HT neurons and neurons in the brain cortex and hippocampus express nicotinic alpha7 receptors. One could test whether stimulation of these receptors may normalize functions of the aberrant brain circuitry and thus be useful as a treatment of SZ. New categories of anti-SZ drugs that target nicotinic alpha7 receptors are being developed.

**Figure 1 illustrates the effects of an** $\alpha 7$ nicotinic receptor agonist (2S,3R)-N-(2-((3-pyridinyl)methyl)-1-azabicyclo[2.2.2]oct-3-yl)-5-methylthiophene-2-carboxamide (Compound B) to reverse deficits in sensory gating (PPI and acoustic startle response) in th(tk-)/th(tk-) mice. Importantly, Compound B had no effect on control (normal mice; n=8).

**Figures 2a and 2b illustrate the effects of an** $\alpha 7$ nicotinic agonist (Compound A) to reverse deficits in PPI in th(tk-)/th(tk-) mice. Compound A had no effect on control (wild type mice) indicating that Compound A corrects specifically the function of abnormal brain circuitry of th(tk-)/th(tk-) mice.

Figure 3 illustrates that Compound A normalizes startle response in th(tk-)/th(tk-) mice.

SYNERGISTIC ACTIONS OF AAPD AND ALPHA7 NICOTINIC AGONISTS

EXAMPLE 1

A pharmaceutical composition is prepared by combining (2S,3R)-N-(2-((3-pyridinyl)methyl)-1-azabicyclo[2.2.2]oct-3-yl)benzofuran-2-carboxamide with clozapine in a pharmaceutically-acceptable carrier. The composition contains respective amounts of (2S,3R)-N-(2-((3-pyridinyl)methyl)-1-azabicyclo[2.2.2]oct-3-yl)benzofuran-2-carboxamide and clozapine to deliver on a daily basis a therapeutically-effective amount of each ingredient. The composition is administered to a patient for the treatment of schizophrenia on a daily, twice daily, three times daily, or four times daily basis.

Clozapine (3.0 mg/kg) and Compound A (0.1 mg/kg) when given individually, had little effect on PPI or startle. See Figures 4, 2a, and 2b. In contrast, for transgenic mice there was a significant main effect of Clozapine (3.0 mg/kg) and Compound A (0.1 mg/kg) combined ($p = 0.006$).

No synergistic effects were observed in control WT mice. See Figures 5 and 6.

EXAMPLE 2

A pharmaceutical composition is prepared by combining (2S,3R)-N-(2-((3-pyridinyl)methyl)-1-azabicyclo[2.2.2]oct-3-yl)benzofuran-2-carboxamide with quetiapine in a pharmaceutically-acceptable carrier. The composition contains respective amounts of (2S,3R)-N-(2-((3-pyridinyl)methyl)-1-azabicyclo[2.2.2]oct-3-yl)benzofuran-2-carboxamide and quetiapine to deliver on a daily basis a therapeutically-effective amount of each ingredient. The composition is administered to a patient for the treatment of schizophrenia on a daily, twice daily, three times daily, or four times daily basis.

In transgenic th(tk-)/th(tk-) mice there was no significant main effect of Quetiapine at 3.0 mg/kg (Figures 7a and 7b) or Compound A at 0.1 mg/kg (Figures 2a and 2b) independently. When used in combination, however, there was a synergistic treatment effect ($p= 0.047$) (Figure 8). The $\alpha 7$ nicotinic agonist and antipsychotic agent work synergistically to improve prepulse inhibition in transgenic mice th(tk-)/th(tk-) mice. For control mice there was no effect of combined doses.

An increasing amount of evidence supports the hypothesis that hypoplastic development and function of dopaminergic neurons are central to the genesis of schizophrenic psychoses and that changes in serotonergic and glutamatergic neurons are important in the pathology (Dean B 2000, Aus. NZ J. Psychiat 34, 560-569; herein incorporated by reference with regard to such teaching). The transgenic th(tk-)/th(tk-) mice support this hypothesis by showing that hypoplastic development of DA neurons in SZ may lead to their hyperfunction as well as affect the development of other neuronal systems. Consequently, an abnormal brain circuitry is created that is responsible for defective sensory-gating, social behavior and cognitive functions. The functioning of this circuitry may be corrected by TAPD, AAPD, and a new class of $\alpha 7$ nicotinic agents.

The th(tk-)/th(tk-) mouse is a developmental model that mimics the multiple structural neuronal, biochemical, and behavioral (positive and negative symptoms) abnormalities found in SZ. TAPD, AAPD and a new classes of antipsychotics ($\alpha$ 7 nicotinic agonists) correct the impaired sensory gating in th(tk-)/th(tk-) transgenics; there is no demonstrated effect on controls. One AAPD, clozapine, affects PPI in control mice, similar to that observed in normal human subjects. Clozapine and Compound A acted synergistically to correct PPI and startle response only in th(tk-)/th(tk-) transgenics; no synergism in controls. Quetiapine and Compound A acted synergistically to correct PPI only in th(tk)/th(tk-) transgenics; no synergism in controls. Negative symptoms (namely impaired social interactions) are corrected by a combination of one or more AAPD and one or more $\alpha$ 7 nicotinic agonists.

Test compounds were employed in free, salt, or solvated form.

The specific pharmacological responses observed may vary according to and depending on the particular active compound selected or whether there are present pharmaceutical carriers, as well as the type of formulation and mode of administration employed, and such expected variations or differences in the results are contemplated in accordance with practice of the present invention.

Example 3

Binding Assays

[$^3$H]-Methyllycaconitine ([$^3$H]-MLA) binding was determined in hippocampal membranes as described previously (Davies et al., 1999). [$^3$H]-Nicotine binding to $\alpha4\beta2$ NNRs in rat cortical membrane preparations or SH-EP1 cells was assayed using standard methods adapted from published procedures (Lippiello and Fernandes, 1986). The $IC_{50}$ (concentration of the compound that produces 50% inhibition of binding) was determined by least squares non-linear regression using GraphPad Prism software (GraphPAD, San Diego, CA). $K_i$ was calculated using the Cheng-Prusoff equation (Cheng and Prusoff, 1973).

Example 4

Elevated Plus Maze in th(tk-)/th(tk-) Transgenic Mice

The subjects were placed in the center of a mouse elevated plus maze (San Diego Instruments, San Diego, CA). The test was videotaped from above using a SONY TRV-350 Handycam video camera using the Nightshot feature. The behavior of the subject was quantified from the videotape using the Observer Mobile (Noldus Information Technologies, Sterling, VA) by an observer unaware of the treatment or genotype of the subjects.

Example 5

Novel Object Recognition in Rats

The dose-response and duration of cognitive-enhancing effects of Compound A following sub-acute 3-day administration were assessed using two variations of a novel object recognition (NOR) task in the rat. The object recognition model is based on a rodent's spontaneous tendency to explore novel aspects of their environment and this exploratory activity can be an index for memory function (Ennaceur and Delacour, 1988). The NOR test measures the capacity to recognize an object presented on two occasions, some time apart. The test arena consisted of a 17.5x17.5"clear Plexiglas™ with walls 12" in height. The arena was enclosed in an opaque, sound-attenuating chamber and the doors (opening to the front side) remained open. Doses of Compound A or vehicle were administered by oral gavage once daily for three days, with an inter-administration interval of 24h. On the first two days of this sub-acute dosing paradigm, administrations were followed 30 minutes later by an exploratory (habituation) trial (6 minutes) on Day 1 (no objects) and object recognition acquisition trial (3 minutes) on Day 2 (2 of the same objects). On the third day the final OR trial, or recall trial (3 minutes; one familiar, one novel object) was started at either 30 min, 2 h, 6 h, 18 h or 24 h after compound administration. For the recall trial, a video camera was positioned approximately 36" from the unshielded side of the arena for videotaping of the animals' behaviors. These behaviors were subsequently hand-scored by a blinded observer assessing the time spent exploring a novel (object B) versus a familiar (object A) object during this recall exposure trial. Absolute exploration time for each object was recorded, and a % recognition index was calculated as follows:

$$\%RI=[(\text{time investigating novel object})/(\text{total time investigating both novel + familiar objects})]$$

Student's t-tests were performed for each treatment group to determine significant differences between exploration time for the familiar versus novel object and 1-WAY ANOVAs (or comparable Kruskall-Wallace ANOVAs for non-parametrically distributed data) were performed to assess significant differences among groups for %RI. Where significant overall effects were found, post-hoc analyses were performed. $P<0.05$ was considered significant.

Example 6

Selectivity of Compound A for the $\alpha7$ NNR

Compound A is a potent inhibitor of [$^3$H]-MLA binding to the $\alpha7$ receptor from rat brain, with a Ki of 1 nM in rat hippocampal membranes (Table 1). A similar binding affinity of 1 nM was obtained in a HEK293 cell line co-expressing human $\alpha7$ and ric3 cDNAs. Compound A has a lower affinity for the $\alpha4\beta2$ receptor subtype. In competition binding studies with [$^3$H]-(S)-nicotine, Compound A displayed a Ki of 2800 nM at human $\alpha4\beta2$ receptors expressed in SH-EP1

cellular membranes and a Ki of 2100 nM at rat α4β2 receptors expressed in rat cortical membranes.

Compound A was also tested in a broad receptor selectivity battery (Novascreen) and minimal interactions with other non-nicotinic receptor classes were found, as defined by inhibition of receptor-selective ligand binding > 50% at 10 μM. Based on this criterion, Compound A showed positive interactions with a non-selective opioid receptor assay (58% inhibition) and with the sigma site 2 (79% inhibition). Dose-response assessments of these interactions showed that the Ki values for the opioid site and for sigma site 2 were both 13 μM, providing a greater than 1000-fold separation from the binding affinity at α7. Due to the close sequence and structural homology between α7 and $5HT_3$ receptors, and previously reported interactions of some nicotinic ligands with both receptors, the affinity of Compound A for $5HT_3$ receptors was examined. Binding of Compound A (10 μM) to $5HT_3$ receptors displayed 59% inhibition of radioligand binding at the mouse receptor and 25% inhibition at the human receptor. Investigation of functional activation at the human $5HT_3$ receptor suggested minimal to no activation; a maximal response of 15% was obtained at 100 μM Compound A.

Example 7

Functional Activation of NNRs by Compound A

Using patch clamp electrophysiological techniques, the functional activity of Compound A at neuronal nicotinic receptors transiently expressed in *Xenopus* oocytes was examined. At human α7 receptors, Compound A displayed an EC50 of 33 nM and an Emax of 100% relative to ACh (Figure 9A and Table 1). There were decreases in subsequent control responses to ACh following the application of Compound A at concentrations greater than 100 nM (IC50 = 200 nM, Figure 9B). In contrast to previously described α7 full agonists (Astles et al., 2002), the separation between EC50 and IC50 values for Compound A indicates that concentrations which produce the half-maximal functional response of alpha7 lead to minimal, rather than full, residual inhibition. There was no detectable activation when Compound A was applied to oocytes expressing the human α4β2 sub-type and no significant decreases in subsequent control responses to ACh, indicating that Compound A is neither an agonist or antagonist at α4β2 (results not shown). Compound A produced very little functional activation of peripheral nicotinic acetylcholine receptors expressed in **appropriate rat and human cell lines (Table 1)**. At 10 and 100 μM, Compound A produced no, or very low, activation of human muscle (5% and 12% of nicotine's Emax, respectively), rat ganglion (11 % and 20% of nicotine's Emax, respectively) or human ganglion (6% and 11% of nicotine's Emax, respectively) receptors. The lack of interaction with muscle and ganglionic-type receptors suggests low potential for nicotinic side effects with Compound A.

Example 8

Compound A - Motor Activity in the Open-field Test

Compound A treatment had no effect on motor activity in the open-field test in either control or th(tk-)/th(tk-) mice (Figure 10A, no main effect of treatment, p>0.05). As previously reported, th(tk-)/th(tk-) spent more time in the center zone of the open-field and moved a greater distance than control mice (Figure 10A, significant main effect of genotype, p<0.05). Also, there was no effect of Compound A on behavior in the elevated plus maze (Figure 10B, no main effect of treatment, p>0.05). The th(tk-)/th(tk-) mice spent significantly more time in the open arm, and consequently less time in the closed arms, than did controls (Figure 10B, significant main effect of genotype, p<0.05).

Example 9

Effectiveness of Compound A in Models of Positive Symptoms of Schizophrenia (Rats)

*Attenuation of Pre-pulse inhibition in Sprague-Dawley Rats*

Prepulse inhibition (PPI) provides an operational measure of sensorimotor gating, a system in the brain that is deficient in schizophrenia. The psychostimulant apomorphine has been shown to impair PPI, and this effect can be reversed by administration of antipsychotic drugs. Compound A (0.3 mg/kg s.c.) significantly reversed PPI deficits induced by administration of apomorphine (Figure 11). These data provide additional evidence that Compound A may be effective in ameliorating the gating deficits associated with schizophrenia.

Example 10

Effects of Compound A on Cognition (Rats)

In the dose-effect assessment of cognition in the novel object recognition test Compound A, at doses of 0.3, 1 and 10 mg/kg (p.o.; 30 minutes after 3rd q.d. administration), significantly increased the time spent investigating the novel object (Fig. 12A, left). In a duration of effect assessment of Compound A (0.3 mg/kg p.o.) in the novel object recognition paradigm, the average time spent exploring object A versus object B by the vehicle-treated group at 30 min, 6 h, or 24 h after the final sub-acute administration trial was not significantly different (p=0.17, p=0.35, and p=0.12, respectively). By comparison, at 30 min, 2 h, 6 h, and 18 h after the final sub-acute (q.d. x 3 days) administration of Compound A (0.3 mg/kg i.p.), subjects spent significantly more time investigating the novel object than the familiar object (Figure 12A, right). Moreover, at 2 h (75%) and 6 h (71%), the recognition Index (RI) was significantly increased in animals treated with 0.3 mg/kg Compound A compared with the RI (54%) of the vehicle-treated group at 30 min after final administration (Figure 12B). The results demonstrate that Compound A facilitates working memory in young rats up to 18 hours after a third sub-acute daily administration.

Table 1: Compound A Binding and Function Parameters at Nicotinic Receptor Subtypes

| NNR Subtype /Parameter | Source | Parameter | Parameter Value (Mean ± S.E.) |
|---|---|---|---|
| α7 Binding | Rat (Hippocampus) Human (HEK Cells) | Ki (nM) | 1.00 ± 0.50<br>1.00 ± 0.04 |
| α7 Function | Oocytes (voltage clamp) | EC50 (nM)<br>Emax (% ACh) | 33 ± 10<br>100 ± 7 |
| α4β2 Binding | Rat Cortex Human (SH-EP1 cells) | Ki (nM) | 2100 ± 400<br>2800 ± 1300 |
| Muscle Function ($Ca^{++}$ flux) | Human (TE-671 cells) | % nicotine @ 10 uM<br>% nicotine @ 100 uM | 5 ±2<br>12 ±7 |
| Ganglion Function ($Ca^{++}$ flux) | Rat (PC-12 Shooter cells)<br><br><br>Human (SH-SY5Y cells) | % nicotine @ 10 uM<br>% nicotine @ 100 uM<br>% nicotine @ 10 uM<br>% nicotine @ 100 uM | 11 ± 6<br>20 ± 8<br>6 ± 2<br>11 ± 1 |

Example 11

Administration of NNR α7 agonists to address high blood sugar, diabetes, weight gain and/or dyslipidemia that can result from antipsychotic (typical or atypical) administration.

***α7 agonist, on Obesity. Animal Models:*** Parental strains of mice used in these studies were the leptin receptor deficient db/db mice on a C57BL6 background obtained from Jackson Laboratories and PTP1B-null mice on a mixed C57BL6/Balb C background from Dr. Michel Tremblay at the Cancer Institute at McGill University in Montreal, Canada. Because obese *db*/*db* mice are infertile, mice were generated as dual heterozygotes, heterozygous for both the mutant leptin receptor and the deleted PTP1B. Dual heterozygotes were interbred, producing 1:4 obese mice and 1:4 PTP-1 B null mice. In this breeding configuration 1:16 were dual KO mice. In the fourth generation, mice heterozygous for both genes were bred to PTP-1 B null mice heterozygous for the mutant *db* allele. In this breeding configuration 1:4 mice were obese and 1:8 were dual KO mice. For reasons of parsimony, heterozygotes were preferred to wild-types over controls. Dual heterozygous littermates were used as lean controls and littermates heterozygous for db were used as lean PTP1B KO controls.

*Mouse genotyping.* At 3 weeks of age, DNA was obtained by tail clip. The genomic DNA from tail clip was used to screen for the presence of the mutant leptin receptor and deletion cassette of PTP-1 B using the Polymerase Chain Reaction. Specific genotypes were determining by resolving PCR products with agarose gel electrophoresis. Deletion of PTP-1 B was verified by Western analysis using an anti-PTP-1 B antibody from Upstate Biotechnology.

*Metabolic Phanotyping.* The effects of the tested compound (for example, Compound A at 1 mg/kg/day via oral gavage) on growth rates and food intake of mice were generated by measuring body weight and food intake bi-weekly for from ages 3 to 10 weeks. In selected cohorts, the α7 antagonist MLA was also given via gavage, concurrently, at 3mg/kg daily. The JAK2 kinase inhibitor (AG-490) was administered intraperitoneally (IP) at 1 mg/kg daily. Fasting

glucose was measured once a week after food withdrawal, with a Precision XL glucometer using tail vein bleeding. HbA1c levels were also measured from these samples with the A1C kit from Metrika, Inc. To assess glucose tolerance, the mice were anesthetized with 2% isoflurane and the left carotid artery and jugular vein cannulated after an overnight fast. A 10 mg bolus of glucose was injected intravenously (iv) via the jugular vein and blood glucose measured every 5 minutes for 40 minutes in a drop of blood from the carotid line. For measurements of blood plasma analytes, a separate group of fasted mice were anesthetized by isoflurane in a rapid induction chamber and swiftly decapitated. Blood was collected in heparin and rapidly centrifuged at 4ºC to remove cells and to obtain plasma, and the samples were frozen for later analyses. Plasma TNF-$\alpha$ concentrations were determined using ELISA assay kits from eBioscience and plasma triglyceride levels were determined using the L-Type TG H test (Wako Diagnostics), an *in vitro* assay for the quantitative determination of triglycerides in serum or plasma. All data are expressed as mean and SEM. Differences among all groups were compared by One Way ANOVA.

*Statistics:* All data are expressed as mean and SEM. Differences among all four genotypes were compared by One Way ANOVA.

Results of administration of Compound B are shown in Figures 13 (blood glucose) and 14 (weight gain). Compound B (1 mg/kg) significantly decreases blood glucose levels (Figure 13) and body weight gain (Figure 14) in db/db mice with fat diet compared to vehicle-treated mice. The effects become apparent after 6 weeks of treatment and become maximal between 8 and 10 weeks post-treatment.

Although specific embodiments of the present invention are herein illustrated and described in detail, the invention is not limited thereto. The above detailed descriptions are provided as exemplary of the present invention and should not be construed as constituting any limitation of the invention. Modifications will be obvious to those skilled in the art, and all modifications that do not depart from the spirit of the invention are intended to be included with the scope of the appended claims.

Reference may be had to the following, each incorporated by reference with regard to such teaching:


Abi-Dargham, A., et al., SPECT imaging of dopamine transporters in human brain with iodine-123-fluoroalkyl analogs of beta-CIT. J Nucl Med, 37(7): 1129-33 (1996);

Abi-Dargham, A., et al., Striatal amphetamine-induced dopamine release in patients with schizotypal personality disorder studied with single photon emission computed tomography and [1231]iodobenzamide. Biol Psychiatry, 55, (10), 1001-6 (2004);

Acker, B.A., et al., Discovery of N-[(3R,5R)-1-azabicyclo[3.2.1]oct-3-yl]furo[2,3-c]pyridine-5-carboxamide as an agonist of the alpha7 nicotinic acetylcholine receptor: in vitro and in vivo activity. Bioorg Med Chem Lett 18: 3611-3615 (2008).

Adler L., et al., Schizophrenia, sensory gating, and nicotinic receptors. Schizophr Bull 24:189-202(1998).

American Diabetes Association, et al., Consensus development conference on antipsychotic drugs and obesity and diabetes. Diabetes Care 27: 596-601 (2004).

Amaiz-Cot JJ, et al., Allosteric modulation of alpha 7 nicotinic receptors selectively depolarizes hippocampal interneurons, enhancing spontaneous GABAergic transmission. Eur J Neurosci. 27: 1097-1110 (2008).

Astles PC, et al., Recent progress in the development of subtype selective nicotinic acetylcholine receptor ligands. Curr Drug Targets CNS Neurol Disord 1: 337-348 (2002).

Avila MT, , et al., Effects of Nicotine on Leading Saccades during Smooth Pursuit Eye Movements in Smokers and Nonsmokers with Schizophrenia. Neuropsychopharmacol 28: 2184-2191 (2003).

Banerjee, S. A., et al., 5' flanking sequences of the rat tyrosine hydroxylase gene target accurate tissue-specific, developmental, and transsynaptic expression in transgenic mice. J Neurosci 12, (11): 4460-7 (2003).

Barnes TRE, et al., Pharmacological strategies for relapse prevention in schizophrenia. Psychiatry 6: 351-356 (2007).

Beckstead, R. M., et al., Efferent connections of the substantia nigra and ventral tegmental area in the rat. Brain Res 175, (2): 191-217 (1979).

Behrendt, RP, Dysregulation of thalamic sensory 'transmission' in schizophrenia: neurochemical vulnerability to hallucinations. J Psychopharmacol 20: 356-72 (2006).

Belluardo N, et al., Neurotrophic effects of central nicotinic receptor activation. J Neural Transm Suppl 60: 227-245 (2000).

Bialowas, J., et al., The relationship between catecholamine levels in the hypothalamus and amygdala under influence of glucose overloading in hungry and sated rats. Pol J Pharmacol Pharm 31, (4): 325-35 (1979).

Bitner RS, et al. Broad-spectrum efficacy across cognitive domains by alpha7 nicotinic acetylcholine receptor agonism correlates with activation of ERK1/2 and CREB phosphorylation pathways. J Neurosci 27: 10578-10587 (2007).

Blumenthal EM, et al., Detection of Functional Nicotinic Receptors Blocked by □-Bungarotoxin on PC12 Cells and Dependence of Their Expression on Post-Translational Events. J Neurosci 17: 6094-6104 (1997).

Boess FG, et al., The novel alpha7 nicotinic acetylcholine receptor agonist N-[(3R)-1-azabicyclo[2.2.2]oct-3-yl]-7-[2-(methoxy)phenyl]-1-benzofuran-2-carboxamide improves working and recognition memory in rodents. J Pharmacol Exp Ther 321: 716-725 (2007).
Bogerts, B., et al., A morphometric study of the dopamine-containing cell groups in the mesencephalon of normals, Parkinson patients, and schizophrenics. Biol Psychiatry 18, (9): 951-69 (1983).
Braff DL, et al., Sensorimotor gating and schizophrenia: human and animal model studies. Arch Gen Psych 47: 181-188 (1990).
Broderick, P.A., et al., I. Serotonin (5-HT) within dopamine reward circuits signals open-field behavior. II. Basis for 5-HT--DA interaction in cocaine dysfunctional behavior. Neurosci Biobehav Rev 21, (3): 227-60 (1997).
Bunney, E. B., et al., Electrophysiological effects of cocaethylene, cocaine, and ethanol on dopaminergic neurons of the ventral tegmental area. J Pharmacol Exp Ther 297, (2): 696-703 (2001).
Cabib, S., et al., Behavioral and mesocorticolimbic dopamine responses to non aggressive social interactions depend on previous social experiences and on the opponent's sex. Behav Brain Res 112, (1-2), 13-22 (2000).
Canitano, R., Clinical experience with Topiramate to counteract neuroleptic induced weight gain in 10 individuals with autistic spectrum disorders. Brain Dev 27: 228-232 (2005).
Carlsson, A., Does dopamine play a role in schizophrenia? Psychol Med 7, (4): 58397 (1977).
Cheng Y, et al., Relationship between inhibition constant (Ki) and concentration of inhibitor which causes 50 per cent inhibition (I50) of an enzymatic-reaction. Biochem Pharmacol 22: 3099-3108 (1973).
Corbett R, et al., Antipsychotic agents antagonize non-competitive N-methyl-D-aspartate antagonist-induced behaviors. Psychopharmacol 120: 67-74 (1995).
Corso, T. D., et al., Transfection of tyrosine kinase deleted FGF receptor-1 into rat brain substantia nigra reduces the number of tyrosine hydroxylase expressing neurons and decreases concentration levels of striatal dopamine. Brain Res Mol Brain Res 139, (2): 361-6 (2005).
Dalack GW, et al., Nicotine dependence in schizophrenia: clinical phenomena and laboratory findings. Am J Psych 155:1490-1501 (1998) .
Dani JA, et al., Nicotinic acetylcholine receptors and nicotinic cholinergic mechanisms of the central nervous system. Annu Rev Pharmacol Toxicol 47:699-729 (2007).
Davies AR, et al., Characterisation of the binding of [3H]methyllycaconitine: a new radioligand for labelling alpha 7-type neuronal nicotinic acetylcholine receptors. Neuropharmacol 38: 679-690 (1999).
Davis, K. L., et al., Dopamine in schizophrenia: a review and reconceptualization. Am J Psychiatry 148, (11): 1474-86 (1991).
De Luca V, et al., Evidence of Association between Smoking and $\alpha$7 Nicotinic Receptor Subunit Gene in Schizophrenia Patients. Neuropsychopharmacol 29: 1522-1526 (2004).
Dickinson JA, et al., Presynaptic alpha 7- and beta 2-containing nicotinic acetylcholine receptors modulate excitatory amino acid release from rat prefrontal cortex nerve terminals via distinct cellular mechanisms. Mol Pharmacol 74: 348-359 (2008).
Doherty, M. D., et al., Ultrastructural localization of the serotonin 2A receptor in dopaminergic neurons in the ventral tegmental area. Brain Res 864, (2): 176-85 (2000).
Durany N, et al., Human post-mortem striatal alpha4beta2 nicotinic acetylcholine receptor density in schizophrenia and Parkinson's syndrome. Neurosci Lett 287: 109-112 (2000).
Ennaceur A, et al., A new one-trial test for neurobiological studies of memory in rats. I. Behavioral data. Behav Brain Res 100: 85-92 (1988).
Fang, X., et al., Control of CREB-binding protein signaling by nuclear fibroblast growth factor receptor-1: a novel mechanism of gene regulation. J Biol Chem 280, (31): 28451-62 (2005).
Farde, L.; et al., Quantitative analysis of D2 dopamine receptor binding in the living human brain by PET. Science 231: 258-261 (1986).
Freedman R, et al., The alpha7-nicotinic acetylcholine receptor and the pathology of hippocampal interneurons in schizophrenia. J Chem Neuroanat 20: 299-306 (2000).
Freedman R, et al., Initial phase 2 trial of a nicotinic agonist in schizophrenia. Am J Psych 165: 1040-1047 (2008).
Gaughran, F., et al., Hippocampal FGF-2 and FGFR1 mRNA expression in major depression, schizophrenia and bipolar disorder. Brain Res Bull 70, (3): 221-7 (2006).
Geyer MA, et al., Measurement of startle response, prepulse inhibition and habituation. Current Protocols Neurosci 8: 7.1-7.15 (1998).
Graham, KA, et al., Double-blind, placebo-controlled investigation of amantadine for weight loss in subjects who gained weight with olanzapine. Am J Psychiatry 162: 1744-1746 (2005).
Hajós M, et al., The selective alpha7 nicotinic acetylcholine receptor agonist PNU-282987 [N-[(3R)-1-Azabicyclo [2.2.2]oct-3-yl]-4-chlorobenzamide hydrochloride] enhances GABAergic synaptic activity in brain slices and restores auditory gating deficits in anesthetized rats. J Pharmacol Exp Ther 312: 1213-1222 (2005).
Harris JG, et al., Effects of nicotine on cognitive deficits in schizophrenia. Neuropsychophannacol 29: 1378-1385 (2004).

Harrison, P. J., The neuropathology of schizophrenia. A critical review of the data and their interpretation. Brain 122 ( Pt 4): 593-624 (1999)

Hashimoto K, et al., α7 Nicotinic Receptor Agonists as Potential Therapeutic Drugs for Schizophrenia. Curr Med Chem - Central Nervous System Agents 5: 171-184 (2005).

Hashimoto, R., et al., Impact of the DISC1 Ser704Cys polymorphism on risk for major depression, brain morphology and ERK signaling. Hum Mol Genet 15, (20): 3024-33 (2006).

Henderson, DC, et al., Glucose metabolism in patients with schizophrenia treated with atypical antipsychotic agents. Arch Gen Psychiatry 62: 19-28 (2005a).

Henderson, DC, et al., A double-blind, placebo-controlled trial of sibutramine for olanzapine-associated weight gain. Am J Psychiatry 162: 954-962 (2005b).

Hietala, J. et al., Depressive symptoms and presynaptic dopamine function in neuroleptic-naive schizophrenia. Schizophr Res 35, (1):41-50 (1999).

Hietala, J., et al., Presynaptic dopamine function in striatum of neuroleptic-naive schizophrenic patients. Lancet 346, (8983): 1130-1 (1995).

Holden C, Deconstructing Schizophrenia: Large-scale family studies and new drug probes focus on cognitive deficits that may lie at the heart of the disease. Science 299: 333-335 (2003).

Horbinski, C., et al., Bone morphogenetic protein-7 stimulates initial dendritic growth in sympathetic neurons through an intracellular fibroblast growth factor signaling pathway. J Neurochem 80, (1): 54-63 (2002).

Hu, Z., et al., Differentiation of the midbrain dopaminergic pathways during mouse development. J Comp Neurol 476, (3): 301-11 (2004).

Hurst RS, et al., A novel positive allosteric modulator of the alpha7 neuronal nicotinic acetylcholine receptor: in vitro and in vivo characterization. J Neurosci 25: 4396-4405 (2005).

Ikemoto, K., et al., Human midbrain dopamine neurons express serotonin 2A receptor: an immunohistochemical demonstration. Brain Res 853, (2): 377-80 (2000).

Kasper, S., et al., Dopamine- and serotonin-receptors in schizophrenia: results of imaging-studies and implications for pharmacotherapy in schizophrenia. EurArch Psychiatry Clin Neurosci 249 Suppl 4: 83-9 (1999).

Kitagawa H, et al., Safety, pharmacokinetics, and effects on cognitive function of multiple doses of GTS-21 in healthy, male volunteers. Neuropsychopharmacol 28: 542-551 (2003).

Klein, DJ, et al., A randomized, double-blind, placebo-controlled trial of metformin treatment of weight gain associated with initiation of atypical antipsychotic therapy in children and adolescents. Am J Psychiatry 163: 2072-2079 (2006).

Klejbor, I., et al., Fibroblast growth factor receptor signaling affects development and function of dopamine neurons - inhibition results in a schizophrenia-like syndrome in transgenic mice. J Neurochem 97, (5): 1243-58 (2006).

Koller et al., Olanzapine-associated diabetes mellitus. Pharmacotherapy 22: 841-852 (2002).

Kwon JS, et al., Gamma frequency-range abnormalities to auditory stimulation in schizophrenia. Arch Gen Psych 56: 1001-1005 (1999).

Laruelle, M., et al., Single photon emission computerized tomography imaging of amphetamine-induced dopamine release in drug-free schizophrenic subjects. Proc Natl Acad Sci U S A 93, (17): 9235-40 (1996).

Lauder, J. M., Neurotransmitters as growth regulatory signals: role of receptors and second messengers. Trends Neurosci 16, (6): 233-40 (1993).

Lena C, et al., Role of Ca2+ ions in nicotinic facilitation of GABA release in mouse thalamus. J Neurosci 17: 576-585 (1997).

Leonard S, et al., Genetics of chromosome 15q13-q14 in schizophrenia. Biol. Psych 60: 115 -122 (2006).

Leonard S, et al., Association of promoter variants in the alpha7 nicotinic acetylcholine receptor subunit gene with an inhibitory deficit found in schizophrenia. Arch Gen Psych 59: 1085-1096 (2002).

Levin ED, et al., AR-R17779, and alpha7 nicotinic agonist, improves learning and memory in rats. Behav Pharmacol 10: 675-680 (1999).

Levin ED, et al., Nicotine-haloperidol interactions and cognitive performance in schizophrenics. Neuropsychopharmacol 15: 429-436 (1996).

Lippiello PM, et al., The binding of L-[3H]nicotine to a single class of high affinity sites in rat brain membranes. Mol Pharmacol 29: 448-454 (1986).

Liu Q, et al., Dissecting the signaling pathway of nicotine-mediated neuroprotection in a mouse Alzheimer disease model. FASEB J21: 61-73 (2007).

Ludewig K, et al., Impaired sensorimotor gating in schizophrenia with deficit and with nondeficit syndrome. Swiss Med Wkly 132: 159-165 (2002).

Mansvelder HD, et al., Long-term potentiation of excitatory inputs to brain reward areas by nicotine. Neuron 27: 349 -357 (2000).

Martin L, et al., Alpha-7 nicotinic receptor agonists: potential new candidates for the treatment of schizophrenia. Psychopharmacol 174: 54-64 (2004).

Meltzer, H. Y., et al., Classification of typical and atypical antipsychotic drugs on the basis of dopamine D-1, D-2 and serotonin2 pKi values. J Pharmacal Exp Ther 251, (1): 238-46 (1989).
Meyer-Lindenberg, A., et al., Reduced prefrontal activity predicts exaggerated striatal dopaminergic function in schizophrenia. Nat Neurosci 5, (3): 267-71 (2002).
Millar, J. K., et al., DISC1 and PDE4B are interacting genetic factors in schizophrenia that regulate cAMP signaling. Science 310, (5751): 1187-91 (2005).
Moffett J, et al., Increased tyrosine phosphorylation and novel cis-acting element mediate activation of the fibroblast growth factor-2 (FGF-2) gene by nicotinic acetylcholine receptor. New mechanism for trans-synaptic regulation of cellular development and plasticity. Brain Res Mol Brain Res 55: 293-305 (1998).
Mudo G, et al., Nicotinic receptor agonists as neuroprotective / neurotrophic drugs. Prog Mol Mechanisms 114: 135-147 (2007b).
Mudo G, et al., Acute intermittent nicotine treatment induces fibroblast growth factor-2 in the subventricular zone of the adult rat brain and enhances neuronal precursor cell proliferation. Neurosci 145:470-483 (2007a).
Murphy PC, et al., Brain-stem modulation of the response properties of cells in the cat's perigeniculate nucleus. Vis Neurosci 11: 781-791 (1994).
Newhouse PA, et al., Effects of nicotinic stimulation on cognitive performance. Curr Opin Pharmacol4: 36-46 (2004).
Nilsson L.K., et al., Subchronic treatment with kynurenine and probenecid: effects on prepulse inhibition and firing of midbrain dopamine neurons.J. Neural Trans. 113, 557-571, 2006.
Ohtani, N., et al., Dopamine modulates cell cycle in the lateral ganglionic eminence. J Neurosci 23, (7): 2840-50 (2003).
O'Neill HC, et al., DMXB, an alpha7 nicotinic agonist, normalizes auditory gating in isolation-reared rats. Psychopharmacol 169: 332-339 (2003).
Ovalle, S., et al., Fibroblast growth factor-2 is selectively modulated in the rat brain by E-5842, a preferential sigma-1 receptor ligand and putative atypical antipsychotic. Eur J Neurosci 13, (5): 909-15 (2001).
Papke RL, et al., Comparative pharmacology of rat and human alpha7 nAChR conducted with net charge analysis. Br J Pharmacol 137: 49-61 (2002).
Petronis, A., The origin of schizophrenia: genetic thesis, epigenetic antithesis, and resolving synthesis. Biol Psychiatry 55, (10): 965-70 (2004); SA 93, (17): 9235-40 (1996).
Pichat P, et al., SSR180711, a novel selective alpha7 nicotinic receptor partial agonist: (II) efficacy in experimental models predictive of activity against cognitive symptoms of schizophrenia. Neuropsychopharmacol 32: 17-34 (2007).
Quarta D, et al., Drug discrimination and neurochemical studies in alpha7 null mutant mice: tests for the role of nicotinic alpha7 receptors in dopamine release. Psychopharmacol Aug 30 [Epub ahead of print] (2008).
Quik M, et al., Localization of nicotinic receptor subunit mRNAs in monkey brain by in situ hybridization. J Comp Neurol 425: 58-69 (2000).
Schimmel, J. J., et al., 4.5 kb of the rat tyrosine hydroxylase 5' flanking sequence directs tissue specific expression during development and contains consensus sites for multiple transcription factors. Brain Res Mol Brain Res 74, (12): 1-14 (1999).
Schreiber R, et al., Effects of alpha 4/beta 2- and alpha 7-nicotine acetylcholine receptor agonists on prepulse inhibition of the acoustic startle response in rats and mice. Psychopharmacol 159: 248-257 (2002).
Semyak, MJ, Association of diabetes mellitus with use of atypical neuroleptics in the treatment of schizophrenia. Am J Psychiatry 159: 561-566 (2002).
Sharma, R. P., Schizophrenia, epigenetics and ligand-activated nuclear receptors: a framework for chromatin therapeutics. Schizophr Res 72, (2-3): 79-90 (2005).
Sharp FR, et al, Psychosis: pathological activation of limbic thalamocortical circuits by psychomimetics and schizophrenia? Trends Neurosci 24: 330-334 (2001).
Simosky JK, et al., Clozapine improves deficient inhibitory auditory processing in DBA/2 mice, via a nicotinic cholinergic mechanism. Psychopharmacol 165: 386-396 (2003).
Singhal SK, et al., Antipsychotic clozapine inhibits the function of alpha7-nicotinic acetylcholine receptors. Neuropharmacol 52: 387-394 (2007).
Snyder, S. H., The dopamine hypothesis of schizophrenia: focus on the dopamine receptor. Am J Psychiatry 133, (2): 197-202 (1976).
Stachowiak MK, et al., Integrative Nuclear Signaling in Cell Development-A Role for FGF Receptor-1. DNA Cell Biol 26: 811-826 (2007).
Stachowiak, E. K., et al., cAMP-induced differentiation of human neuronal progenitor cells is mediated by nuclear fibroblast growth factor receptor-1 (FGFR1). J Neurochem 84, (6): 1296-312 (2003).
Stachowiak, M. K., et al., Apparent sprouting of striatal serotonergic terminals after dopamine-depleting brain lesions in neonatal rats. Brain Res 291, (1): 164-7 (1984).
Stachowiak, M. K., et al., Integrative Nuclear Signaling in Cell Development-A Role for FGF Receptor-1. DNA Cell

Biol 26, (12): 811-26 (2007).
Stahl SM, Antipsychotic agents. In: Essential Pharmacology: Neuroscientific Basis and Practical Applications. Cambridge University Press, New York, pp 401-458 (2002).
Swerdlow, N. R., et al., Using an animal model of deficient sensorimotor gating to study the pathophysiology and new treatments of schizophrenia. Schizophr Bull 24, (2): 285301 (1998).
Swerdlow, N. R., et al., Assessing the validity of an animal model of deficient sensorimotor gating in schizophrenic patients. Arch Gen Psychiatry 51, (2): 139-54 (1994).
Tietje KR, et al., Preclinical characterization of A-582941: a novel alpha 7 neuronal nicotinic receptor agonist with broad spectrum cognition-enhancing properties. CNS Neurosci Ther 14: 65-82 (2008).
Timmermann DB, et al., An allosteric modulator of the alpha7 nicotinic acetylcholine receptor possessing cognition-enhancing properties in vivo. J Pharmacol Exp Ther 323: 294-307 (2007).
Van Kampen M, et al., AR-R 17779 improves social recognition in rats by activation of nicotinic alpha7 receptors. Psychopharmacol 172: 375-383 (2004).
Waldo MC, et al., Auditory sensory gating, hippocampal volume, and catecholamine metabolism in schizophrenics and their siblings. Schizophrenia Res 12: 93-106 (1994).
Wong AH, et al., Schizophrenia: from phenomenology to neurobiology. Neurosci Biobehav Rev 27: 269-306 (2003).
Wong, A. H., et al., Identification of candidate genes for psychosis in rat models, and possible association between schizophrenia and the 14-3-3eta gene. Mol Psychiatry 8, (2): 156-66 (2003).
Yang, Y. K., et al., Associated alterations of striatal dopamine D2/D3 receptor and transporter binding in drug-naive patients with schizophrenia: a dual-isotope SPECT study. Am J Psychiatry 161, (8): 1496-8 (2004).

## Claims

**1.** A pharmaceutical combination for use in the treatment or prevention of a psychiatric disorder to provide a synergistic therapy for a psychiatric disorder and comprising:

at least one α7 nicotinic agonist; and
at least one antipsychotic agent.

**2.** A pharmaceutical combination according to claim 1, wherein the at least one α7 nicotinic agonist and the at least one antipsychotic agent are for simultaneous, sequential, or separate administration.

**3.** A pharmaceutical combination according to claim 1 or 2, wherein the at least one α7 nicotinic agonist is a compound of Formula 1:

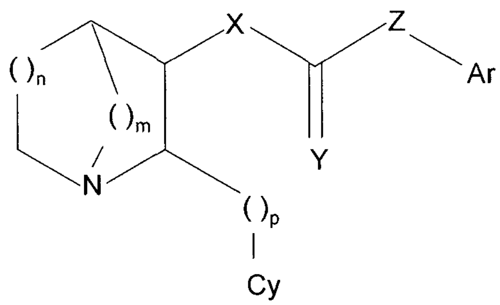

Formula 1

or a pharmaceutically acceptable salt or solvate thereof,

wherein m is 1 or 2;
n is 1 or 2;
p is 1, 2, 3, or 4;
X is oxygen or -NR'-;
Y is oxygen or sulfur;
Z is -NR'-, a covalent bond, or a linker species, A;

A is -CR'R"-, -CR'R"-CR'R"-, -CR'=CR'-, or -C≡C-,
wherein each R' and R" individually are hydrogen, alkyl, cycloalkyl, heterocyclyl, aryl, or arylalkyl, or R' and R" may combine with the atom to which they are attached to form a 3 to 8 membered ring, which may contain additional heteroatoms;
when Z is a covalent bond or A, then X must be nitrogen;
Ar is an optionally substituted aryl group; and
Cy is an optionally substituted 5- or 6-membered heteroaromatic ring,
where each of Ar and Cy may be substituted with one or more of alkyl, alkenyl, heterocyclyl, cycloalkyl, aryl, substituted aryl, arylalkyl, substituted arylalkyl, halogen, -OR', -NR'R", haloalkyl, -CN, $-NO_2$, -C≡CR', -SR', $-N_3$, -C(=O)NR'R", -NR'C(=O)R", -C(=O)R', -C(=O)OR', -OC(=O)R', $-O(CR'R")_rC(=O)R'$, $-O(CR'R")_rNR"C(=O)R'$, $-O(CR'R")_rNR"SO_2R'$, -OC(=O)NR'R", -NR'C(=O)OR", $-SO_2R'$, $-SO_2NR'R"$, or $-NR'SO_2R"$, where each of R' and R" are as defined, and
r is an integer from 1 to 6,
wherein substituted aryl and substituted arylalkyl groups have one or more of alkyl, alkenyl, heterocyclyl, cycloalkyl, aryl, arylalkyl, halogen, -OR', -NR'R", haloalkyl, -CN, $-NO_2$, -C=CR', -SR', $-N_3$, -C(=O)NR'R", -NR'C(=O)R", -C(=O)R', -C(=O)OR', -OC(=O)R', -O(CR'R"),C(=O)R', $-O(CR'R")_rNR"C(=O)R'$, $-O(CR'R")_rNR"SO_2R'$, -OC(=O)NR'R", -NR'C(=O)OR", $-SO_2R"$, $-SO_2NR'R"$, or $-NR'SO_2R"$,
where each of R', R", and r are as defined.

**4.** The pharmaceutical combination of claim 3, wherein

(A) p is 1 ,
Cy is 3-pyridinyl or 5-pyrimidinyl,
each of X and Y are oxygen, and
Z is -NR'-; or
wherein
(B) p is 1 ,
Cy is 3-pyridinyl or 5-pyrimidinyl,
X and Z are -NR'-, and
Y is oxygen; or
wherein
(C) p is 1 ,
Cy is 3-pyridinyl or 5-pyrimidinyl,
X is $-NR^1-$,
Y is oxygen, and
Z is a covalent bond; or
wherein
(D) p is 1,
Cy is 3-pyridinyl or 5-pyrimidinyl,
X is -NR'-,
Y is oxygen, and Z is A.

**5.** A pharmaceutical combination of claim 3, wherein the compound of Formula 1 is:

2-((3-pyridinyl)methyl)-1-azabicyclo[2.2.2]oct-3-yl N-phenylcarbamate,
2-((3-pyridinyl)methyl)-1-azabicyclo[2.2.2]oct-3-yl N-(4-fluorophenyl)carbamate,
2-((3-pyridinyl)methyl)-1-azabicyclo[2.2.2]oct-3-yl N-(4-chlorophenyl)carbamate,
2-((3-pyridinyl)methyl)-1-azabicyclo[2.2.2]oct-3-yl N-(4-bromophenyl)carbamate,
2-((3-pyridinyl)methyl)-1-azabicyclo[2.2.2]oct-3-yl N-(3-fluorophenyl)carbamate,
2-((3-pyridinyl)methyl)-1-azabicyclo[2.2.2]oct-3-yl N-(3-chlorophenyl)carbamate,
2-((3-pyridinyl)methyl)-1-azabicyclo[2.2.2]oct-3-yl N-(3-bromophenyl)carbamate,
2-((3-pyridinyl)methyl)-1-azabicyclo[2.2.2]oct-3-yl N-(2-fluorophenyl)carbamate,
2-((3-pyridinyl)methyl)-1-azabicyclo[2.2.2]oct-3-yl N-(2-chlorophenyl)carbamate,
2-((3-pyridinyl)methyl)-1-azabicyclo[2.2.2]oct-3-yl N-(2-bromophenyl)carbamate,
2-((3-pyridinyl)methyl)-1-azabicyclo[2.2.2]oct-3-yl N-(3,4-dichlorophenyl)carbamate,
2-((3-pyridinyl)methyl)-1-azabicyclo[2.2.2]oct-3-yl N-(2-methylphenyl)carbamate,
2-((3-pyridinyl)methyl)-1-azabicyclo[2.2.2]oct-3-yl N-(2-biphenyl)carbamate,
2-((3-pyridinyl)methyl)-1-azabicyclo[2.2.2]oct-3-yl N-(3-methylphenyl)carbamate,

2-((3-pyridinyl)methyl)-1-azabicyclo[2.2.2]oct-3-yl N-(3-biphenyl)carbamate,
2-((3-pyridinyl)methyl)-1-azabicyclo[2.2.2]oct-3-yl N-(4-methylphenyl)carbamate,
2-((3-pyridinyl)methyl)-1-azabicyclo[2.2.2]oct-3-yl N-(4-biphenyl)carbamate,
2-((3-pyridinyl)methyl)-1-azabicyclo[2.2.2]oct-3-yl N-(2-cyanophenyl)carbamate,
2-((3-pyridinyl)methyl)-1-azabicyclo[2.2.2]oct-3-yl N-(3-cyanophenyl)carbamate,
2-((3-pyridinyl)methyl)-1-azabicyclo[2.2.2]oct-3-yl N-(4-cyanophenyl)carbamate,
2-((3-pyridinyl)methyl)-1-azabicyclo[2.2.2]oct-3-yl N-(3-trifluoromethylphenyl)carbamate,
2-((3-pyridinyl)methyl)-1-azabicyclo[2.2.2]oct-3-yl N-(4-dimethylaminophenyl)carbamate,
2-((3-pyridinyl)methyl)-1-azabicyclo[2.2.2]oct-3-yl N-(2-methoxyphenyl)carbamate,
2-((3-pyridinyl)methyl)-1-azabicyclo[2.2.2]oct-3-yl N-(2-phenoxyphenyl)carbamate,
2-((3-pyridinyl)methyl)-1-azabicyclo[2.2.2]oct-3-yl N-(2-methylthiophenyl)carbamate,
2-((3-pyridinyl)methyl)-1-azabicyclo[2.2.2]oct-3-yl N-(2-phenylthiophenyl)carbamate,
2-((3-pyridinyl)methyl)-1-azabicyclo[2.2.2]oct-3-yl N-(3-methoxyphenyl)carbamate,
2-((3-pyridinyl)methyl)-1-azabicyclo[2.2.2]oct-3-yl N-(3-phenoxyphenyl)carbamate,
2-((3-pyridinyl)methyl)-1-azabicyclo[2.2.2]oct-3-yl N-(3-methylthiophenyl)carbamate,
2-((3-pyridinyl)methyl)-1-azabicyclo[2.2.2]oct-3-yl N-(3-phenylthiophenyl)carbamate,
2-((3-pyridinyl)methyl)-1-azabicyclo[2.2.2]oct-3-yl N-(4-methoxyphenyl)carbamate,
2-((3-pyridinyl)methyl)-1-azabicyclo[2.2.2]oct-3-yl N-(4-phenoxyphenyl)carbamate,
2-((3-pyridinyl)methyl)-1-azabicyclo[2.2.2]oct-3-yl N-(4-methylthiophenyl)carbamate,
2-((3-pyridinyl)methyl)-1-azabicyclo[2.2.2]oct-3-yl N-(4-phenylthiophenyl)carbamate,
2-((3-pyridinyl)methyl)-1-azabicyclo[2.2.2]oct-3-yl N-(2,4-dimethoxyphenyl)carbamate,
2-((3-pyridinyl)methyl)-1-azabicyclo[2.2.2]oct-3-yl N-(2-thienyl)carbamate,
2-((3-pyridinyl)methyl)-1-azabicyclo[2.2.2]oct-3-yl N-(3-thienyl)carbamate,
2-((3-pyridinyl)methyl)-1-azabicyclo[2.2.2]oct-3-yl N-(3-benzothienyl)carbamate,
2-((3-pyridinyl)methyl)-1-azabicyclo[2.2.2]oct-3-yl N-(1-naphthyl)carbamate,
2-((3-pyridinyl)methyl)-1-azabicyclo[2.2.2]oct-3-yl N-(2-naphthyl)carbamate,
N-phenyl-N'-(2-((3-pyridinyl)methyl)-1-azabicyclo[2.2.2]oct-3-yl)urea,
N-(4-fluorophenyl)-N'-(2-((3-pyridinyl)methyl)-1-azabicyclo[2.2.2]oct-3-yl)urea,
N-(4-chlorophenyl)-N'-(2-((3-pyridinyl)methyl)-1-azabicyclo[2.2.2]oct-3-yl)urea,
N-(4-bromophenyl)-N'-(2-((3-pyridinyl)methyl)-1-azabicyclo[2.2.2]oct-3-yl)urea,
N-(3-fluorophenyl)-N'-(2-((3-pyridinyl)methyl)-1-azabicyclo[2.2.2]oct-3-yl)urea,
N-(3-chlorophenyl)-N'-(2-((3-pyridinyl)methyl)-1-azabicyclo[2.2.2]oct-3-yl)urea,
N-(3-bromophenyl)-N'-(2-((3-pyridinyl)methyl)-1-azabicyclo[2.2.2]oct-3-yl)urea,
N-(2-fluorophenyl)-N'-(2-((3-pyridinyl)methyl)-1-azabicyclo[2.2.2]oct-3-yl)urea,
N-(2-chlorophenyl)-N'-(2-((3-pyridinyl)methyl)-1-azabicyclo[2.2.2]oct-3-yl)urea,
N-(2-bromophenyl)-N'-(2-((3-pyridinyl)methyl)-1-azabicyclo[2.2.2]oct-3-yl)urea,
N-(3,4-dichlorophenyl)-N'-(2-((3-pyridinyl)methyl)-1-azabicyclo[2.2.2]oct-3-yl)urea,
N-(2-methylphenyl)-N'-(2-((3-pyridinyl)methyl)-1-azabicyclo[2.2.2]oct-3-yl)urea,
N-(2-biphenyl)-N'-(2-((3-pyridinyl)methyl)-1-azabicyclo[2.2.2]oct-3-yl)urea,
N-(3-methylphenyl)-N'-(2-((3-pyridinyl)methyl)-1-azabicyclo[2.2.2]oct-3-yl)urea,
N-(3-biphenyl)-N'-(2-((3-pyridinyl)methyl)-1-azabicyclo[2.2.2]oct-3-yl)urea,
N-(4-methylphenyl)-N'-(2-((3-pyridinyl)methyl)-1-azabicyclo[2.2.2]oct-3-yl)urea,
N-(4-biphenyl)-N'-(2-((3-pyridinyl)methyl)-1-azabicyclo[2.2.2]oct-3-yl)urea,
N-(2-cyanophenyl)-N'-(2-((3-pyridinyl)methyl)-1-azabicyclo[2.2.2]oct-3-yl)urea,
N-(3-cyanophenyl)-N'-(2-((3-pyridinyl)methyl)-1-azabicyclo[2.2.2]oct-3-yl)urea,
N-(4-cyanophenyl)-N'-(2-((3-pyridinyl)methyl)-1-azabicyclo[2.2.2]oct-3-yl)urea,
N-(3-trifluoromethylphenyl)-N'-(2-((3-pyridinyl)methyl)-1-azabicyclo[2.2.2]oct-3-yl)urea,
N-(4-dimethylaminophenyl)-N'-(2-((3-pyridinyl)methyl)-1-azabicyclo[2.2.2]oct-3-yl)urea,
N-(2-methoxyphenyl)-N'-(2-((3-pyridinyl)methyl)-1-azabicyclo[2.2.2]oct-3-yl)urea,
N-(2-phenoxyphenyl)-N'-(2-((3-pyridinyl)methyl)-1-azabicyclo[2.2.2]oct-3-yl)urea,
N-(2-methylthiophenyl)-N'-(2-((3-pyridinyl)methyl)-1-azabicyclo[2.2.2]oct-3-yl)urea,
N-(2-phenylthiophenyl)-N'-(2-((3-pyridinyl)methyl)-1-azabicyclo[2.2.2]oct-3-yl)urea,
N-(3-methoxyphenyl)-N'-(2-((3-pyridinyl)methyl)-1-azabicyclo[2.2.2]oct-3-yl)urea,
N-(3-phenoxyphenyl)-N'-(2-((3-pyridinyl)methyl)-1-azabicyclo[2.2.2]oct-3-yl)urea,
N-(3-methylthiophenyl)-N'-(2-((3-pyridinyl)methyl)-1-azabicyclo[2.2.2]oct-3-yl)urea,
N-(3-phenylthiophenyl)-N'-(2-((3-pyridinyl)methyl)-1-azabicyclo[2.2.2]oct-3-yl)urea,
N-(4-methoxyphenyl)-N'-(2-((3-pyridinyl)methyl)-1-azabicyclo[2.2.2]oct-3-yl)urea,
N-(4-phenoxyphenyl)-N'-(2-((3-pyridinyl)methyl)-1-azabicyclo[2.2.2]oct-3-yl)urea,

N-(4-methylthiophenyl)-N'-(2-((3-pyridinyl)methyl)-1-azabicyclo[2.2.2]oct-3-yl)urea,
N-(4-phenylthiophenyl)-N'-(2-((3-pyridinyl)methyl)-1-azabicyclo[2.2.2]oct-3-yl)urea,
N-(2,4-dimethoxyphenyl)-N'-(2-((3-pyridinyl)methyl)-1-azabicyclo[2.2.2]oct-3-yl)urea,
N-(2-thienyl)-N'-(2-((3-pyridinyl)methyl)-1-azabicyclo[2.2.2]oct-3-yl)urea,
N-(3-thienyl)-N'-(2-((3-pyridinyl)methyl)-1-azabicyclo[2.2.2]oct-3-yl)urea,
N-(3-benzothienyl)-N'-(2-((3-pyridinyl)methyl)-1-azabicyclo[2.2.2]oct-3-yl)urea,
N-(1-naphthyl)-N'-(2-((3-pyridinyl)methyl)-1-azabicyclo[2.2.2]oct-3-yl)urea,
N-(2-naphthyl)-N'-(2-((3-pyridinyl)methyl)-1-azabicyclo[2.2.2]oct-3-yl)urea,
N-(2-((3-pyridinyl)methyl)-1-azabicyclo[2.2.2]oct-3-yl)benzamide,
N-(2-((3-pyridinyl)methyl)-1-azabicyclo[2.2.2]oct-3-yl)-2-fluorobenzamide,
N-(2-((3-pyridinyl)methyl)-1-azabicyclo[2.2.2]oct-3-yl)-3-fluorobenzamide,
N-(2-((3-pyridinyl)methyl)-1-azabicyclo[2.2.2]oct-3-yl)-4-fluorobenzamide,
N-(2-((3-pyridinyl)methyl)-1-azabicyclo[2.2.2]oct-3-yl)-2-chlorobenzamide,
N-(2-((3-pyridinyl)methyl)-1-azabicyclo[2.2.2]oct-3-yl)-3-chlorobenzamide,
N-(2-((3-pyridinyl)methyl)-1-azabicyclo[2.2.2]oct-3-yl)-4-chlorobenzamide,
N-(2-((3-pyridinyl)methyl)-1-azabicyclo[2.2.2]oct-3-yl)-2-bromobenzamide,
N-(2-((3-pyridinyl)methyl)-1-azabicyclo[2.2.2]oct-3-yl)-3-bromobenzamide,
N-(2-((3-pyridinyl)methyl)-1-azabicyclo[2.2.2]oct-3-yl)-4-bromobenzamide,
N-(2-((3-pyridinyl)methyl)-1-azabicyclo[2.2.2]oct-3-yl)-3,4-dichlorobenzamide,
N-(2-((3-pyridinyl)methyl)-1-azabicyclo[2.2.2]oct-3-yl)-2-methylbenzamide,
N-(2-((3-pyridinyl)methyl)-1-azabicyclo[2.2.2]oct-3-yl)-3-methylbenzamide,
N-(2-((3-pyridinyl)methyl)-1-azabicyclo[2.2.2]oct-3-yl)-4-methylbenzamide,
N-(2-((3-pyridinyl)methyl)-1-azabicyclo[2.2.2]oct-3-yl)-2-phenylbenzamide,
N-(2-((3-pyridinyl)methyl)-1-azabicyclo[2.2.2]oct-3-yl)-3-phenylbenzamide,
N-(2-((3-pyridinyl)methyl)-1-azabicyclo[2.2.2]oct-3-yl)-4-phenylbenzamide,
N-(2-((3-pyridinyl)methyl)-1-azabicyclo[2.2.2]oct-3-yl)-2-cyanobenzamide,
N-(2-((3-pyridinyl)methyl)-1-azabicyclo[2.2.2]oct-3-yl)-3-cyanobenzamide,
N-(2-((3-pyridinyl)methyl)-1-azabicyclo[2.2.2]oct-3-yl)-4-cyanobenzamide,
N-(2-((3-pyridinyl)methyl)-1-azabicyclo[2.2.2]oct-3-yl)-3-trifluoromethylbenzamide,
N-(2-((3-pyridinyl)methyl)-1-azabicyclo[2.2.2]oct-3-yl)-4-dimethylaminobenzamide,
N-(2-((3-pyridinyl)methyl)-1-azabicyclo[2.2.2]oct-3-yl)-2-methoxybenzamide,
N-(2-((3-pyridinyl)methyl)-1-azabicyclo[2.2.2]oct-3-yl)-3-methoxybenzamide,
N-(2-((3-pyridinyl)methyl)-1-azabicyclo[2.2.2]oct-3-yl)-4-methoxybenzamide,
N-(2-((3-pyridinyl)methyl)-1-azabicyclo[2.2.2]oct-3-yl)-2-phenoxybenzamide,
N-(2-((3-pyridinyl)methyl)-1-azabicyclo[2.2.2]oct-3-yl)-3-phenoxybenzamide,
N-(2-((3-pyridinyl)methyl)-1-azabicyclo[2.2.2]oct-3-yl)-4-phenoxybenzamide,
N-(2-((3-pyridinyl)methyl)-1-azabicyclo[2.2.2]oct-3-yl)-2-methylthiobenzamide,
N-(2-((3-pyridinyl)methyl)-1-azabicyclo[2.2.2]oct-3-yl)-3-methylthiobenzamide,
N-(2-((3-pyridinyl)methyl)-1-azabicyclo[2.2.2]oct-3-yl)-4-methylthiobenzamide,
N-(2-((3-pyridinyl)methyl)-1-azabicyclo[2.2.2]oct-3-yl)-2-phenylthiobenzamide,
N-(2-((3-pyridinyl)methyl)-1-azabicyclo[2.2.2]oct-3-yl)-3-phenylthiobenzamide,
N-(2-((3-pyridinyl)methyl)-1-azabicyclo[2.2.2]oct-3-yl)-4-phenylthiobenzamide,
N-(2-((3-pyridinyl)methyl)-1-azabicyclo[2.2.2]oct-3-yl)-2,4-dimethoxybenzamide,
N-(2-((3-pyridinyl)methyl)-1-azabicyclo[2.2.2]oct-3-yl)-5-bromonicotinamide,
N-(2-((3-pyridinyl)methyl)-1-azabicyclo[2.2.2]oct-3-yl)-6-chloronicotinamide,
N-(2-((3-pyridinyl)methyl)-1-azabicyclo[2.2.2]oct-3-yl)-5-phenylnicotinamide,
N-(2-((3-pyridinyl)methyl)-1-azabicyclo[2.2.2]oct-3-yl)furan-2-carboxamide,
N-(2-((3-pyridinyl)methyl)-1-azabicyclo[2.2.2]oct-3-yl)furan-3-carboxamide,
N-(2-((3-pyridinyl)methyl)-1-azabicyclo[2.2.2]oct-3-yl)thiophene-2-carboxamide,
N-(2-((3-pyridinyl)methyl)-1-azabicyclo[2.2.2]oct-3-yl)-5-bromothiophene-2-carboxamide,
N-(2-((3-pyridinyl)methyl)-1-azabicyclo[2.2.2]oct-3-yl)-5-methylthiothiophene-2-carboxamide,
N-(2-((3-pyridinyl)methyl)-1-azabicyclo[2.2.2]oct-3-yl)-5-phenylthiothiophene-2-carboxamide,
N-(2-((3-pyridinyl)methyl)-1-azabicyclo[2.2.2]oct-3-yl)-5-methylthiophene-2-carboxamide,
N-(2-((3-pyridinyl)methyl)-1-azabicyclo[2.2.2]oct-3-yl)-3-methylthiophene-2-carboxamide,
N-(2-((3-pyridinyl)methyl)-1-azabicyclo[2.2.2]oct-3-yl)-3-bromothiophene-2-carboxamide,
N-(2-((3-pyridinyl)methyl)-1-azabicyclo[2.2.2]oct-3-yl)-3-chlorothiophene-2-carboxamide,
N-(2-((3-pyridinyl)methyl)-1-azabicyclo[2.2.2]oct-3-yl)-5-(2-pyridinyl)thiophene-2-carboxamide,
N-(2-((3-pyridinyl)methyl)-1-azabicyclo[2.2.2]oct-3-yl)-5-acetylthiophene-2-carboxamide,

N-(2-((3-pyridinyl)methyl)-1-azabicyclo[2.2.2]oct-3-yl)-3-ethoxythiophene-2-carboxamide,
N-(2-((3-pyridinyl)methyl)-1-azabicyclo[2.2.2]oct-3-yl)-3-methoxythiophene-2-carboxamide,
N-(2-((3-pyridinyl)methyl)-1-azabicyclo[2.2.2]oct-3-yl)-4-acetyl-3-methyl-5-methylthiothiophene-2-carboxamide,
N-(2-((3-pyridinyl)methyl)-1-azabicyclo[2.2.2]oct-3-yl)thiophene-3-carboxamide,
N-(2-((3-pyridinyl)methyl)-1-azabicyclo[2.2.2]oct-3-yl)-1-methylpyrrole-2-carboxamide,
N-(2-((3-pyridinyl)methyl)-1-azabicyclo[2.2.2]oct-3-yl)pyrrole-3-carboxamide,
N-(2-((3-pyridinyl)methyl)-1-azabicyclo[2.2.2]oct-3-yl)indole-2-carboxamide,
N-(2-((3-pyridinyl)methyl)-1-azabicyclo[2.2.2]oct-3-yl)indole-3-carboxamide,
N-(2-((3-pyridinyl)methyl)-1-azabicyclo[2.2.2]oct-3-yl)-1-methylindole-3-carboxamide,
N-(2-((3-pyridinyl)methyl)-1-azabicyclo[2.2.2]oct-3-yl)-1-benzylindole-3-carboxamide,
N-(2-((3-pyridinyl)methyl)-1-azabicyclo[2.2.2]oct-3-yl)-1H-benzimidazole-2-carboxamide,
N-(2-((3-pyridinyl)methyl)-1-azabicyclo[2.2.2]oct-3-yl)-1-isopropyl-2-trifluoromethyl-1H benzimidazole-5-carboxamide,
N-(2-((3-pyridinyl)methyl)-1-azabicyclo[2.2.2]oct-3-yl)-1-isopropyl-1H-benzotriazole-5-carboxamide,
N-(2-((3-pyridinyl)methyl)-1-azabicyclo[2.2.2]oct-3-yl)benzo[b]thiophene-2-carboxamide,
N-(2-((3-pyridinyl)methyl)-1-azabicyclo[2.2.2]oct-3-yl)benzo[b]thiophene-3-carboxamide,
N-(2-((3-pyridinyl)methyl)-1-azabicyclo[2.2.2]oct-3-yl)benzofuran-2-carboxamide,
N-(2-((3-pyridinyl)methyl)-1-azabicyclo[2.2.2]oct-3-yl)benzofuran-3-carboxamide,
N-(2-((3-pyridinyl)methyl-1-azabicyclo[2.2.2]oct-3-yl)-3-methylbenzofuran-2-carboxamide,
N-(2-((3-pyridinyl)methyl-1-azabicyclo[2.2.2]oct-3-yl)-5-nitrobenzofuran-2-carboxamide,
N-(2-((3-pyridinyl)methyl-1-azabicyclo[2.2.2]oct-3-yl)-5-methoxybenzofuran-2-carboxamide,
N-(2-((3-pyridinyl)methyl-1-azabicyclo[2.2.2]oct-3-yl)-7-methoxybenzofuran-2-carboxamide,
N-(2-((3-pyridinyl)methyl-1-azabicyclo[2.2.2]oct-3-yl)-7-ethoxybenzofuran-2-carboxamide,
N-(2-((3-pyridinyl)methyl-1-azabicyclo[2.2.2]oct-3-yl)-3-methyl-5-chlorobenzofuran-2-carboxamide,
N-(2-((3-pyridinyl)methyl-1-azabicyclo[2.2.2]oct-3-yl)-6-bromobenzofuran-2-carboxamide,
N-(2-((3-pyridinyl)methyl-1-azabicyclo[2.2.2]oct-3-yl)-4-acetyl-7-methoxybenzofuran-2-carboxamide,
N-(2-((3-pyridinyl)methyl-1-azabicyclo[2.2.2]oct-3-yl)-2-methylbenzofuran-4-carboxamide,
N-(2-((3-pyridinyl)methyl-1-azabicyclo[2.2.2]oct-3-yl)naphtho[2,1-b]furan-2-carboxamide,
N-(2-((3-pyridinyl)methyl)-1-azabicyclo[2.2.2]oct-3-yl)naphthalene-1-carboxamide,
N-(2-((3-pyridinyl)methyl)-1-azabicyclo[2.2.2]oct-3-yl)naphthalene-2-carboxamide,
N-(2-((3-pyridinyl)methyl)-1-azabicyclo[2.2.2]oct-3-yl)-6-aminonaphthalene-2-carboxamide,
N-(2-((3-pyridinyl)methyl-1-azabicyclo[2.2.2]oct-3-yl)-3-methoxynaphthalene-2-carboxamide,
N-(2-((3-pyridinyl)methyl-1-azabicyclo[2.2.2]oct-3-yl)-6-methoxynaphthalene-2-carboxamide,
N-(2-((3-pyridinyl)methyl-1-azabicyclo[2.2.2]oct-3-yl)-1-hydroxynaphthalene-2-carboxamide,
N-(2-((3-pyridinyl)methyl-1-azabicyclo[2.2.2]oct-3-yl)-6-hydroxynaphthalene-2-carboxamide,
N-(2-((3-pyridinyl)methyl-1-azabicyclo[2.2.2]oct-3-yl)-6-acetoxynaphthalene-2-carboxamide,
N-(2-((3-pyridinyl)methyl)-1-azabicyclo[2.2.2]oct-3-yl)3-phenylprop-2-enamide,
N-(2-((3-pyridinyl)methyl)-1-azabicyclo[2.2.2]oct-3-yl)-3-(3-fluorophenyl)prop-2-enamide,
N-(2-((3-pyridinyl)methyl)-1-azabicyclo[2.2.2]oct-3-yl)-3-(4-methoxyphenyl)prop-2-enamide,
N-(2-((3-pyridinyl)methyl)-1-azabicyclo[2.2.2]oct-3-yl)-2-methyl-3-phenylprop-2-enamide,
N-(2-((3-pyridinyl)methyl)-1-azabicyclo[2.2.2]oct-3-yl)-3-(2-fluorophenyl)prop-2-enamide,
N-(2-((3-pyridinyl)methyl)-1-azabicyclo[2.2.2]oct-3-yl)-3-(3-methylphenyl)prop-2-enamide,
N-(2-((3-pyridinyl)methyl)-1-azabicyclo[2.2.2]oct-3-yl)-3-(4-fluorophenyl)prop-2-enamide,
N-(2-((3-pyridinyl)methyl)-1-azabicyclo[2.2.2]oct-3-yl)-3-(4-methylphenyl)prop-2-enamide,
N-(2-((3-pyridinyl)methyl)-1-azabicyclo[2.2.2]oct-3-yl)-3-(2-furyl)prop-2-enamide,
N-(2-((3-pyridinyl)methyl)-1-azabicyclo[2.2.2]oct-3-yl)-3-(2-methoxyphenyl)prop-2-enamide,
N-(2-((3-pyridinyl)methyl)-1-azabicyclo[2.2.2]oct-3-yl)-3-(3-bromophenyl)prop-2-enamide,
N-(2-((3-pyridinyl)methyl)-1-azabicyclo[2.2.2]oct-3-yl)-3-(3-methoxyphenyl)prop-2-enamide,
N-(2-((3-pyridinyl)methyl)-1-azabicyclo[2.2.2]oct-3-yl)-3-(3-hydroxyphenyl)prop-2-enamide,
N-(2-((3-pyridinyl)methyl)-1-azabicyclo[2.2.2]oct-3-yl)-3-(4-bromophenyl)prop-2-enamide,
N-(2-((3-pyridinyl)methyl)-1-azabicyclo[2.2.2]oct-3-yl)-3-(4-chlorophenyl)prop-2-enamide,
N-(2-((3-pyridinyl)methyl)-1-azabicyclo[2.2.2]oct-3-yl)-3-(4-hydroxyphenyl)prop-2-enamide,
N-(2-((3-pyridinyl)methyl)-1-azabicyclo[2.2.2]oct-3-yl)-3-(4-hydroxy-3-methoxyphenyl)prop-2- enamide,
N-(2-((3-pyridinyl)methyl)-1-azabicyclo[2.2.2]oct-3-yl)-3-(2-thienyl)prop-2-enamide,
N-(2-((3-pyridinyl)methyl)-1-azabicyclo[2.2.2]oct-3-yl)-3-(3-pyridinyl)prop-2-enamide,
N-(2-((3-pyridinyl)methyl)-1-azabicyclo[2.2.2]oct-3-yl)-3-(4-biphenyl)prop-2-enamide,
N-(2-((3-pyridinyl)methyl)-1-azabicyclo[2.2.2]oct-3-yl)-3-(1-naphthyl)prop-2-enamide,

N-(2-((3-pyridinyl)methyl)-1-azabicyclo[2.2.2]oct-3-yl)-3-(3-thienyl)prop-2-enamide,
N-(2-((3-pyridinyl)methyl)-1-azabicyclo[2.2.2]oct-3-yl)-3-(4-isopropylphenyl)prop-2-enamide,
N-(2-((3-pyridinyl)methyl)-1-azabicyclo[2.2.2]oct-3-yl)-3-methyl-3-phenylprop-2-enamide,
N-(2-((3-pyridinyl)methyl)-1-azabicyclo[2.2.2]oct-3-yl)-3-(3-furyl)prop-2-enamide,
N-(2-((3-pyridinyl)methyl)-1-azabicyclo[2.2.2]oct-3-yl)-2-ethyl-3-phenylprop-2-enamide,
N-(2-((3-pyridinyl)methyl)-1-azabicyclo[2.2.2]oct-3-yl)-3-(2-pyridinyl)prop-2-enamide,
N-(2-((3-pyridinyl)methyl)-1-azabicyclo[2.2.2]oct-3-yl)-3-(3,4-dimethylthieno[2,3-b]thiophen-2-yl)prop-2-enamide,
N-(2-((3-pyridinyl)methyl)-1-azabicyclo[2.2.2]oct-3-yl)-3-(3-methylthien-2-yl)prop-2-enamide,
N-(2-((3-pyridinyl)methyl)-1-azabicyclo[2.2.2]oct-3-yl)-3-(2-naphthyl)prop-2-enamide,
N-(2-((3-pyridinyl)methyl)-1-azabicyclo[2.2.2]oct-3-yl)-3-(4-methylthiophenyl)prop-2-enamide, or a pharmaceutically acceptable salt or solvate thereof.

**6.** A pharmaceutical combination according to any one of claims 1 to 5, wherein the at least one antipsychotic agent is either a conventional or atypical antipsychotic.

**7.** A pharmaceutical combination according to any one of claims 1 to 6, wherein the at least one antipsychotic is a conventional antipsychotic, selected from chlorpromazine, haloperidol, flupenthixol, or perphenazine, or a metabolite, salt, or solvate thereof.

**8.** A pharmaceutical combination according to any one of claims 1 to 6, wherein the at least one antipsychotic is an atypical antipsychotic, selected from clozapine, risperidone, olanzapine, quetiapine, aripiprazole, ziprasidone, amisulpride, sulpride, zotepine, sertindole, paliperidone, bifeprunox, or asenapine, or a metabolite, salt, or solvate thereof.

**9.** A pharmaceutical combination of claim 8, wherein the at least one antipsychotic is clozapine or quetiapine, or a metabolite, salt, or solvate thereof.

**10.** Use of a pharmaceutical combination according to any of claims 1 to 9 in the preparation of a medicament for the treatment or prevention of a psychiatric disorder.

**11.** A combination according to any one of claims 1 to 9 or a use according to claim 10, wherein the psychiatric disorder is a psychotic disorder.

**12.** A combination according to any one of claims 1 to 9 or a use according to claim 10 or 11, wherein the psychiatric disorder is schizophrenia, schizophreniform disorder, schizoaffective disorder, delusional disorder, brief psychotic disorder, shared psychotic disorder, treatment-resistant psychotic disorder, a psychotic disorders due to a general medical condition, or a psychotic disorder that is not otherwise specified.

**13.** A combination or use according to claim 12 wherein the psychiatric disorder is schizophrenia.

**14.** A combination according to any one of claims 1 to 9 or a use according to claim 10 or 11, wherein the disorder is one or more of attention disorders, information processing or memory disorders or deficits in executive functions.

**15.** A kit for the treatment or prevention of a psychiatric disorder comprising a package containing a combination as defined in any one of claims 1 to 9 or 11 to 14.

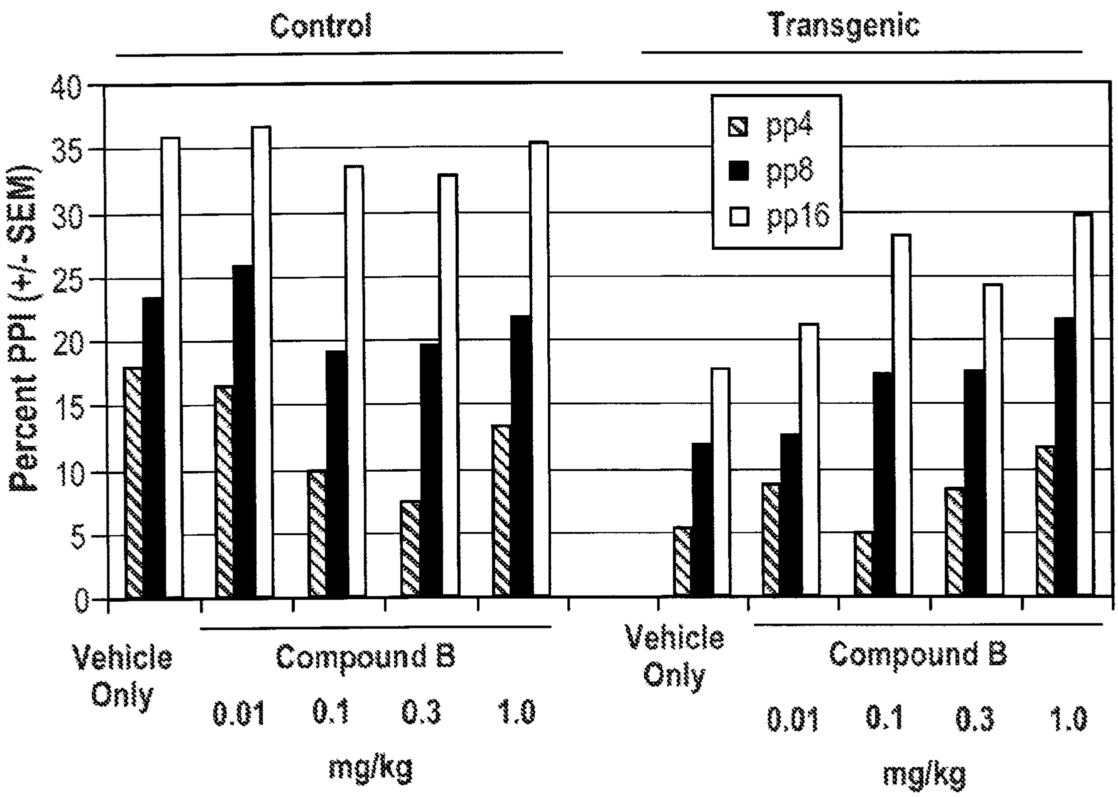
Control
Transgenic
pp4
pp8
pp16
Percent PPI (+/- SEM)
0
5
10
15
20
25
30
35
40
Vehicle Only
Compound B
0.01
0.1
0.3
1.0
mg/kg
Vehicle Only
Compound B
0.01
0.1
0.3
1.0
mg/kg

*Fig. 1*

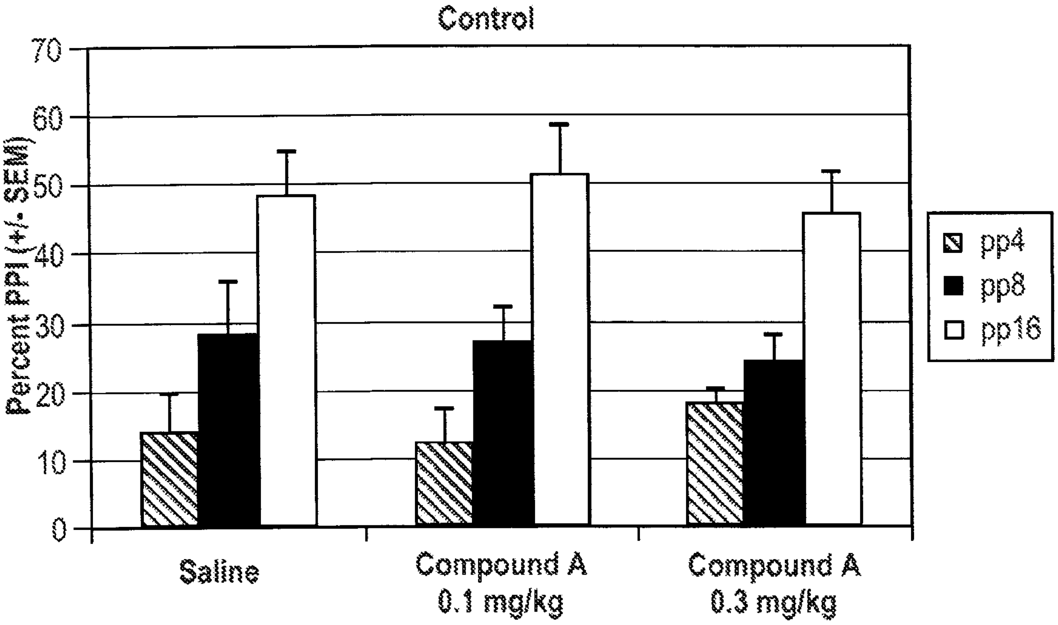
Control
Percent PPI (+/- SEM)
70
60
50
40
30
20
10
0
Saline
Compound A
0.1 mg/kg
Compound A
0.3 mg/kg
pp4
pp8
pp16

*Fig. 2a*

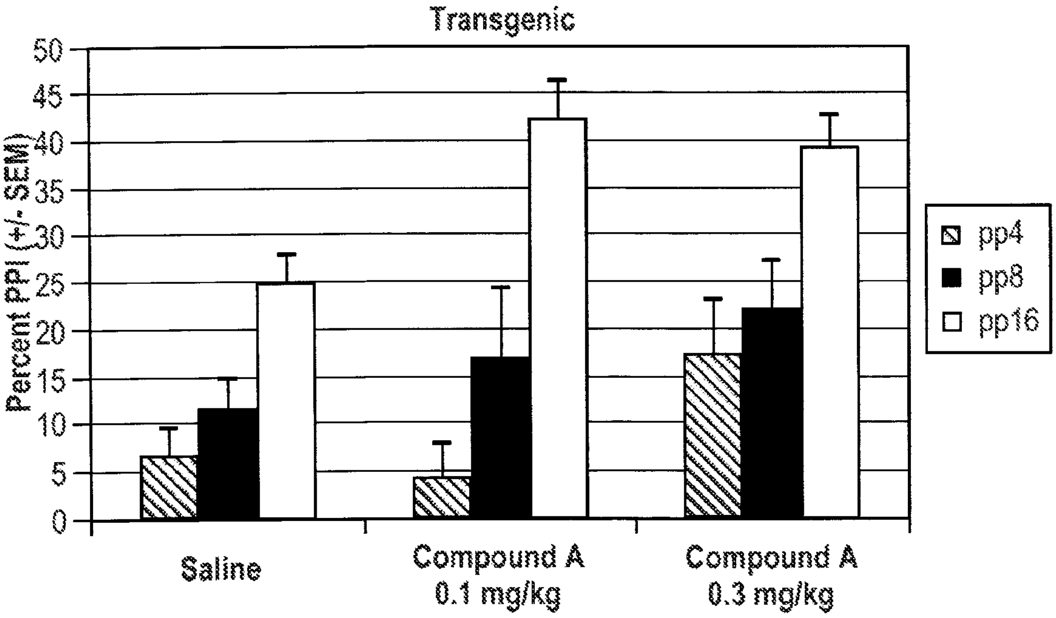
Transgenic
Percent PPI (+/- SEM)
50
45
40
35
30
25
20
15
10
5
0
Saline
Compound A
0.1 mg/kg
Compound A
0.3 mg/kg
pp4
pp8
pp16

*Fig. 2b*

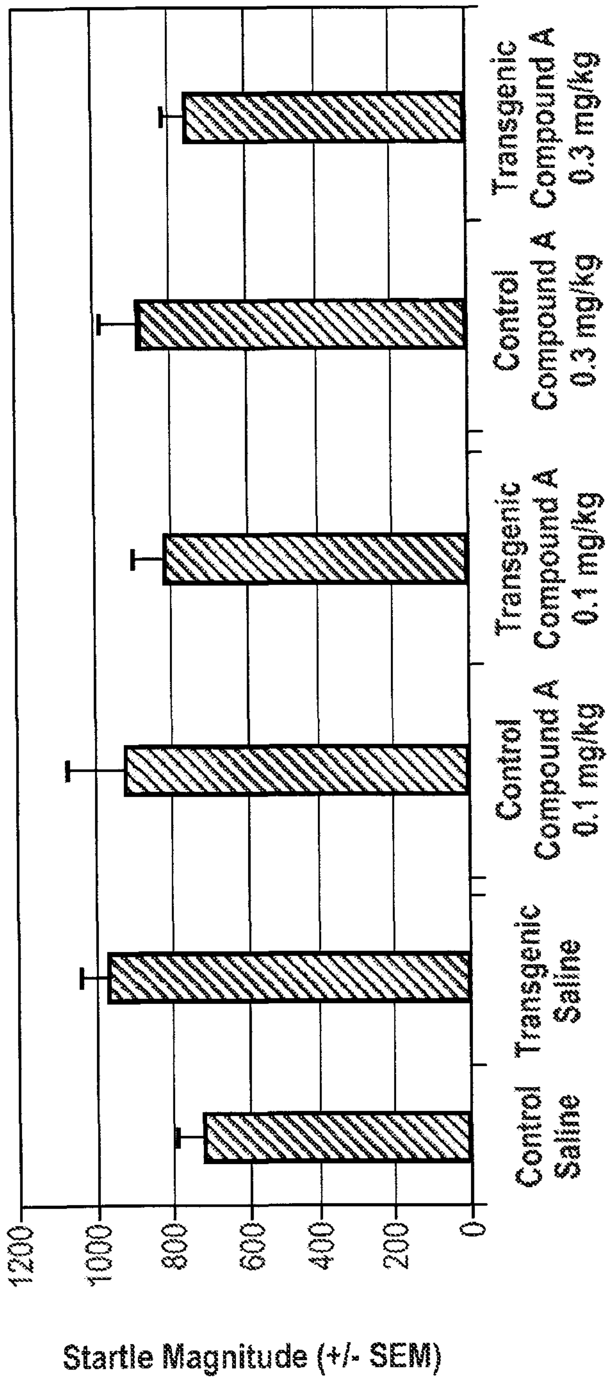
Startle Magnitude (+/- SEM)
0
200
400
600
800
1000
1200
Control Saline
Transgenic Saline
Control Compound A 0.1 mg/kg
Transgenic Compound A 0.1 mg/kg
Control Compound A 0.3 mg/kg
Transgenic Compound A 0.3 mg/kg

Fig. 3

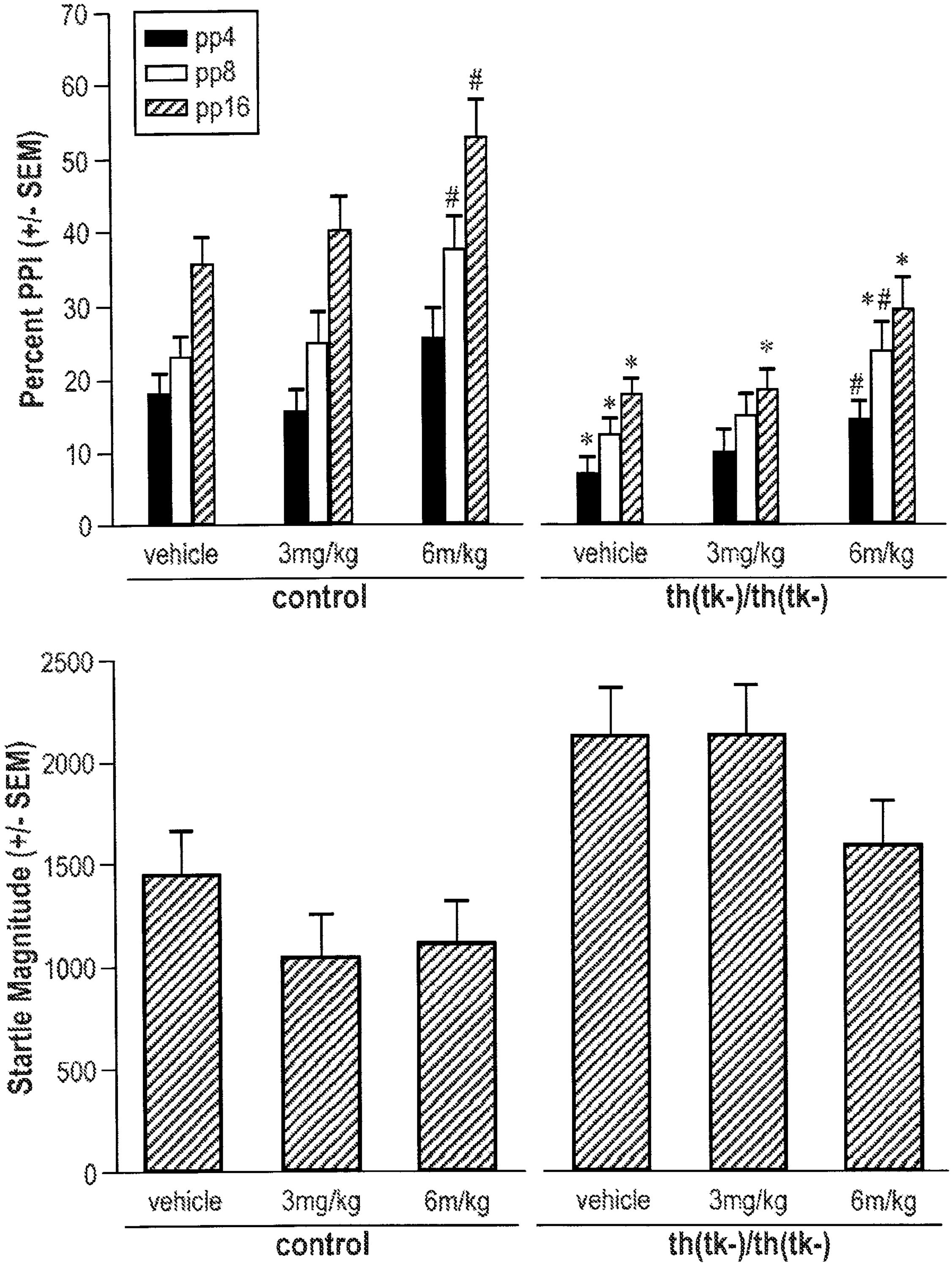
Percent PPI (+/- SEM)
70
60
50
40
30
20
10
0
pp4
pp8
pp16
#
#
*
*#
*
#
*
*
*
vehicle
3mg/kg
6m/kg
vehicle
3mg/kg
6m/kg
control
th(tk-)/th(tk-)
Startle Magnitude (+/- SEM)
2500
2000
1500
1000
500
0
vehicle
3mg/kg
6m/kg
vehicle
3mg/kg
6m/kg
control
th(tk-)/th(tk-)

*Fig. 4*

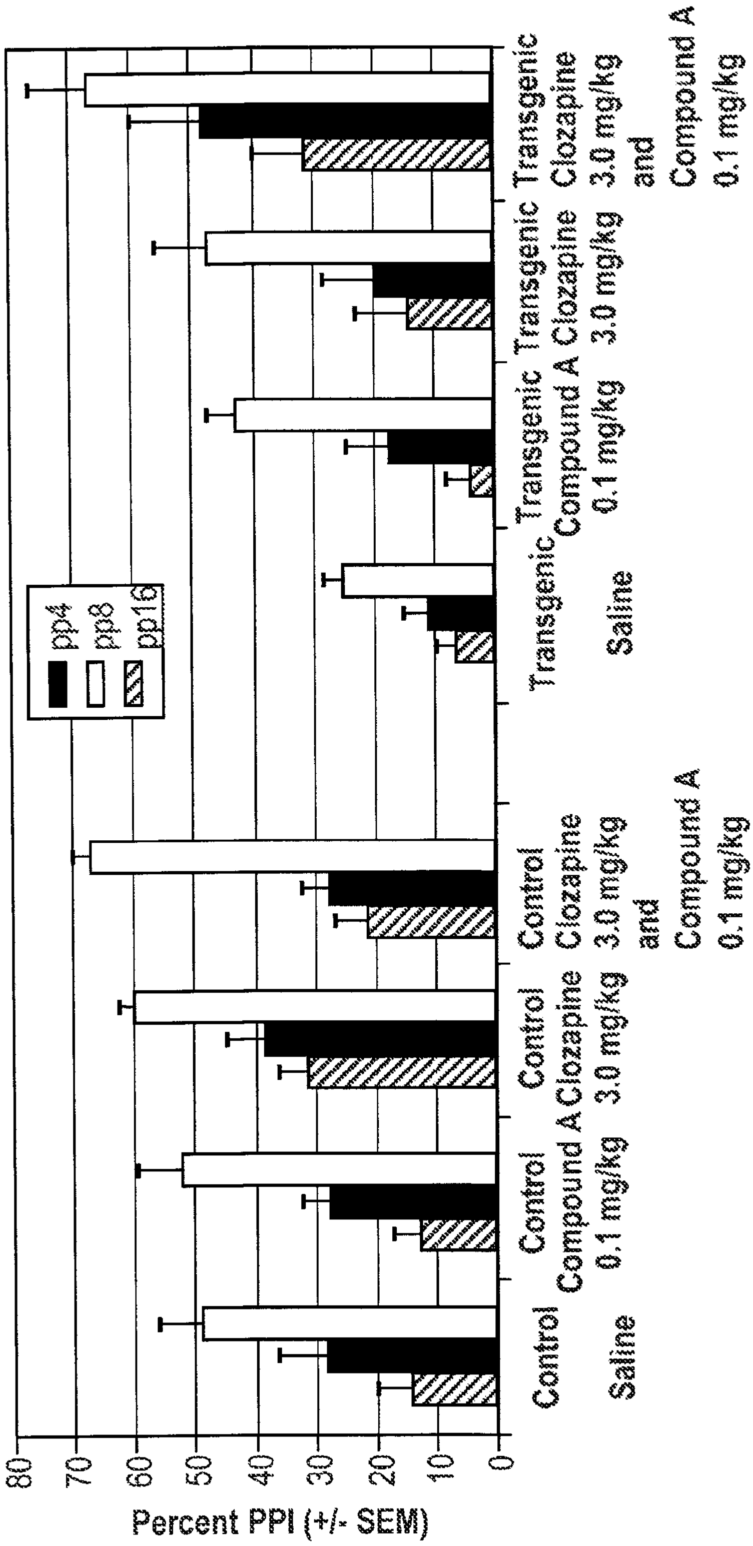

Percent PPI (+/- SEM)
0
10
20
30
40
50
60
70
80
pp4
pp8
pp16
Control Saline
Control Compound A 0.1 mg/kg
Control Clozapine 3.0 mg/kg
Control Clozapine 3.0 mg/kg and Compound A 0.1 mg/kg
Transgenic Saline
Transgenic Compound A 0.1 mg/kg
Transgenic Clozapine 3.0 mg/kg
Transgenic Clozapine 3.0 mg/kg and Compound A 0.1 mg/kg


*Fig. 5*

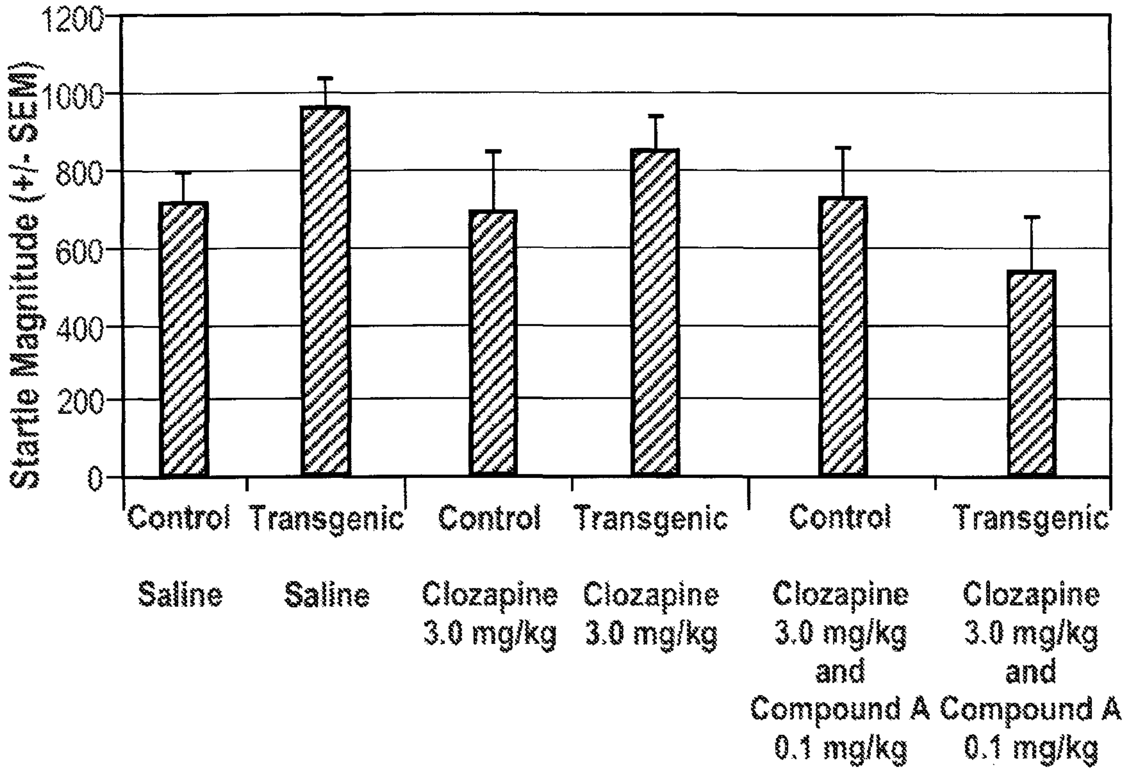
Startle Magnitude (+/- SEM)
1200
1000
800
600
400
200
0
Control
Transgenic
Control
Transgenic
Control
Transgenic
Saline
Saline
Clozapine 3.0 mg/kg
Clozapine 3.0 mg/kg
Clozapine 3.0 mg/kg and Compound A 0.1 mg/kg
Clozapine 3.0 mg/kg and Compound A 0.1 mg/kg

*Fig. 6*

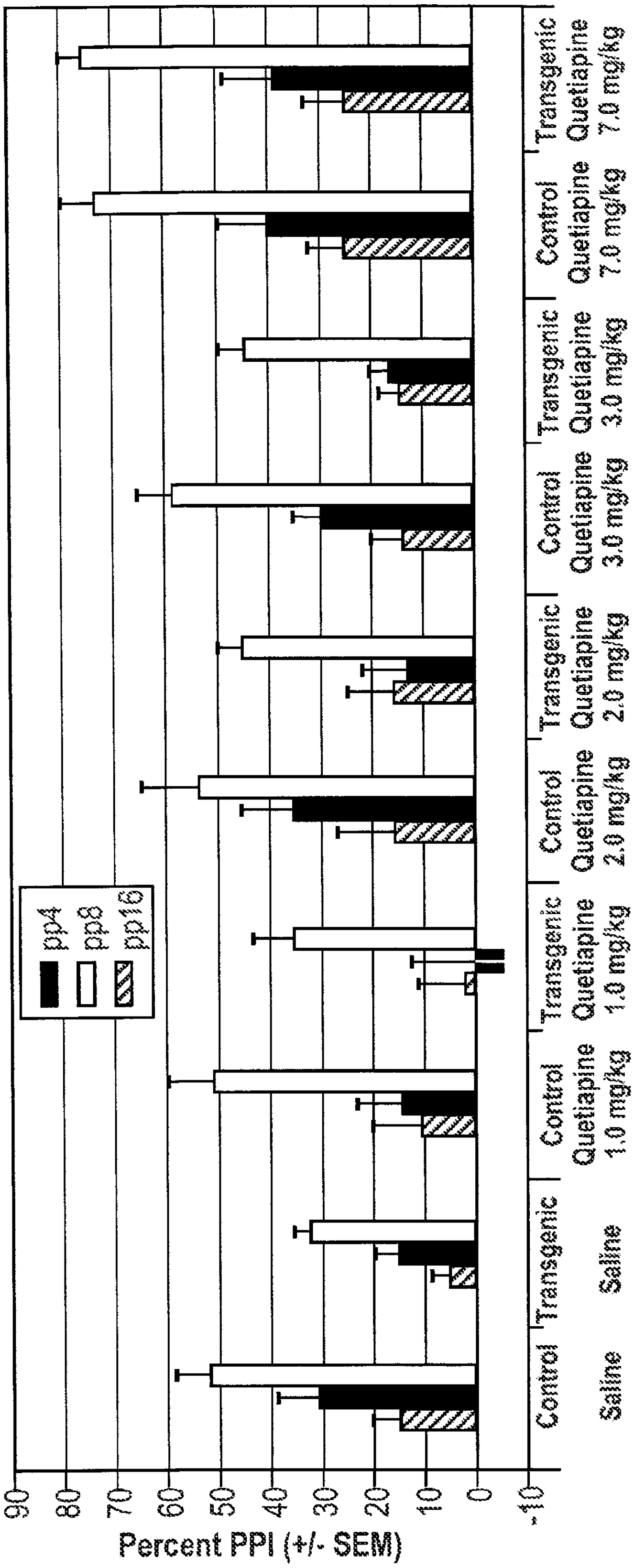

pp4
pp8
pp16
Percent PPI (+/- SEM)
90
80
70
60
50
40
30
20
10
0
-10
Control Saline
Transgenic Saline
Control Quetiapine 1.0 mg/kg
Transgenic Quetiapine 1.0 mg/kg
Control Quetiapine 2.0 mg/kg
Transgenic Quetiapine 2.0 mg/kg
Control Quetiapine 3.0 mg/kg
Transgenic Quetiapine 3.0 mg/kg
Control Quetiapine 7.0 mg/kg
Transgenic Quetiapine 7.0 mg/kg


Fig. 7a

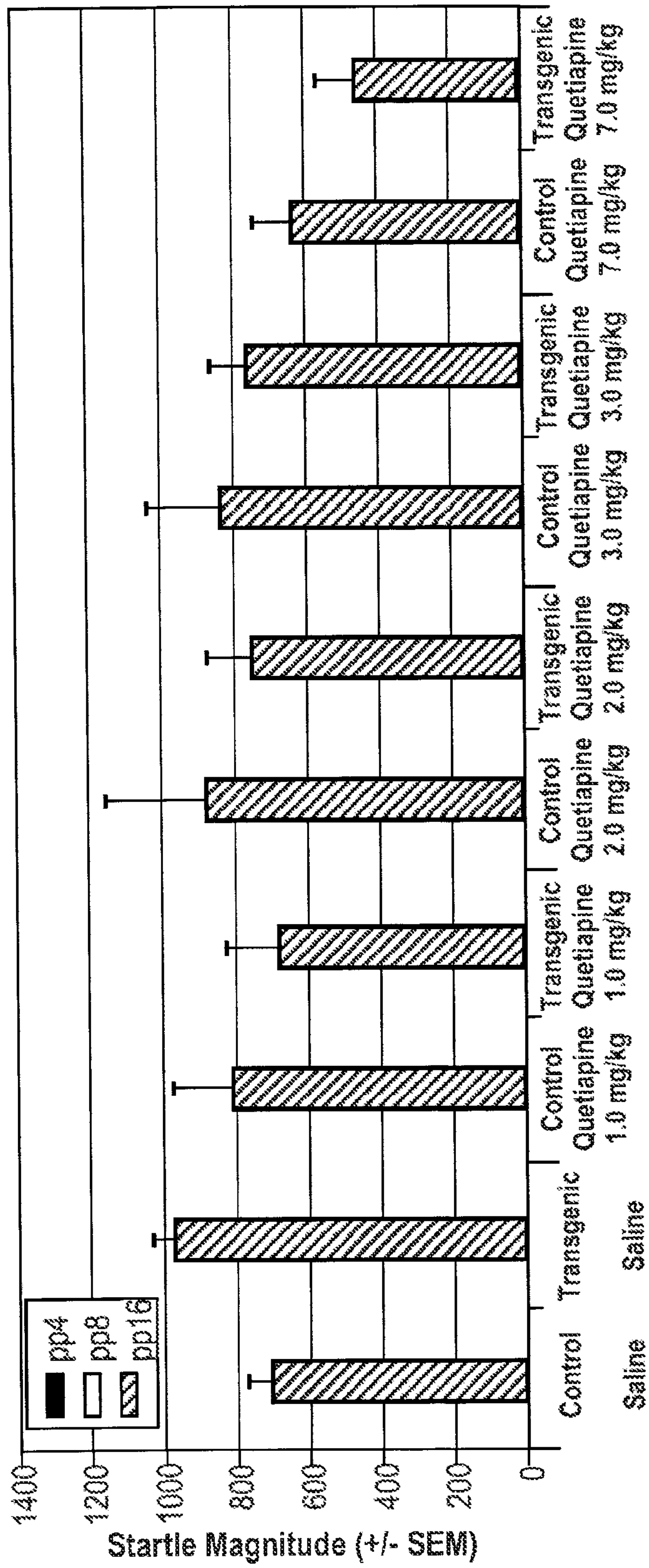
pp4
pp8
pp16
Startle Magnitude (+/- SEM)
1400
1200
1000
800
600
400
200
0
Control Saline
Transgenic Saline
Control Quetiapine 1.0 mg/kg
Transgenic Quetiapine 1.0 mg/kg
Control Quetiapine 2.0 mg/kg
Transgenic Quetiapine 2.0 mg/kg
Control Quetiapine 3.0 mg/kg
Transgenic Quetiapine 3.0 mg/kg
Control Quetiapine 7.0 mg/kg
Transgenic Quetiapine 7.0 mg/kg

*Fig. 7b*

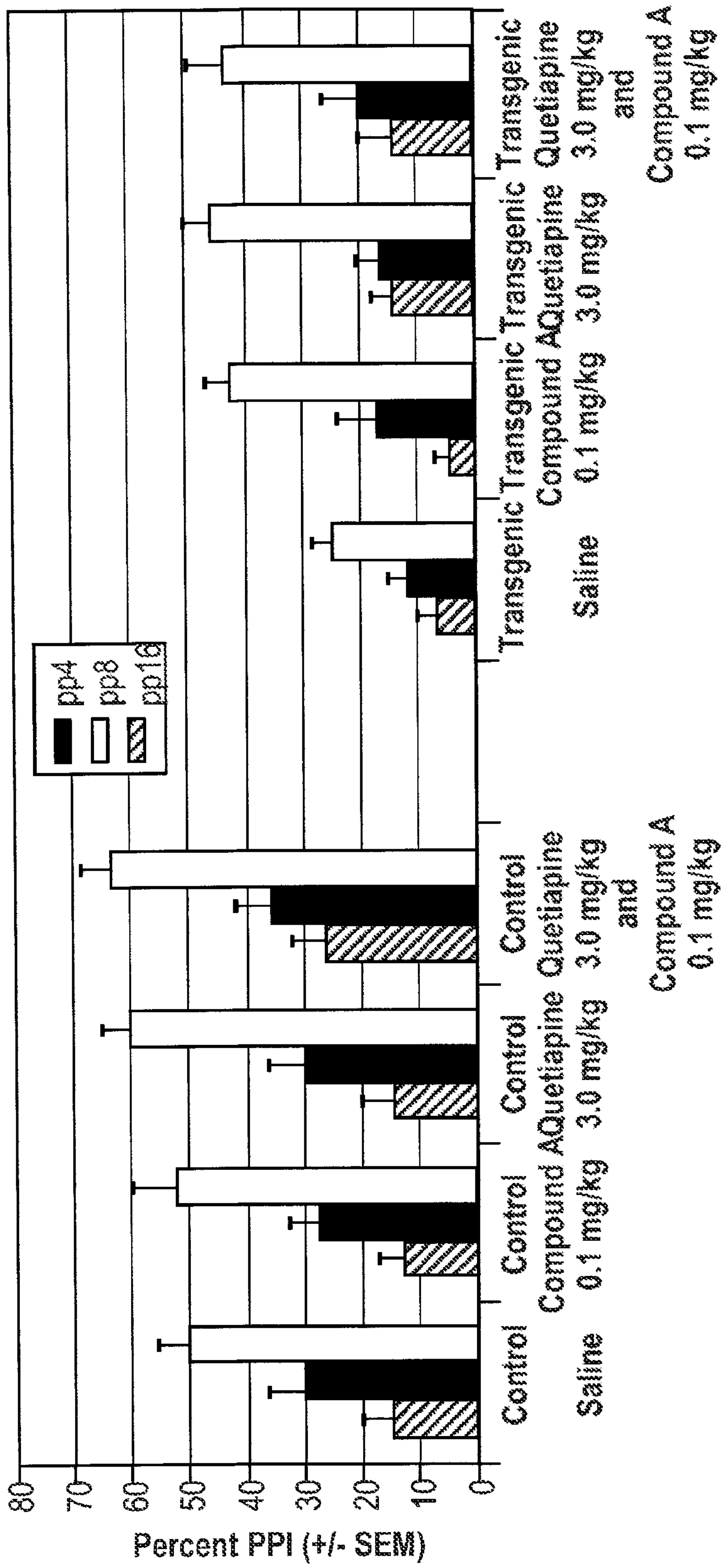
Percent PPI (+/- SEM)
0
10
20
30
40
50
60
70
80
pp4
pp8
pp16
Control Saline
Control Compound A 0.1 mg/kg
Control Quetiapine 3.0 mg/kg
Control Quetiapine 3.0 mg/kg and Compound A 0.1 mg/kg
Transgenic Saline
Transgenic Compound A 0.1 mg/kg
Transgenic Quetiapine 3.0 mg/kg
Transgenic Quetiapine 3.0 mg/kg and Compound A 0.1 mg/kg

*Fig. 8a*

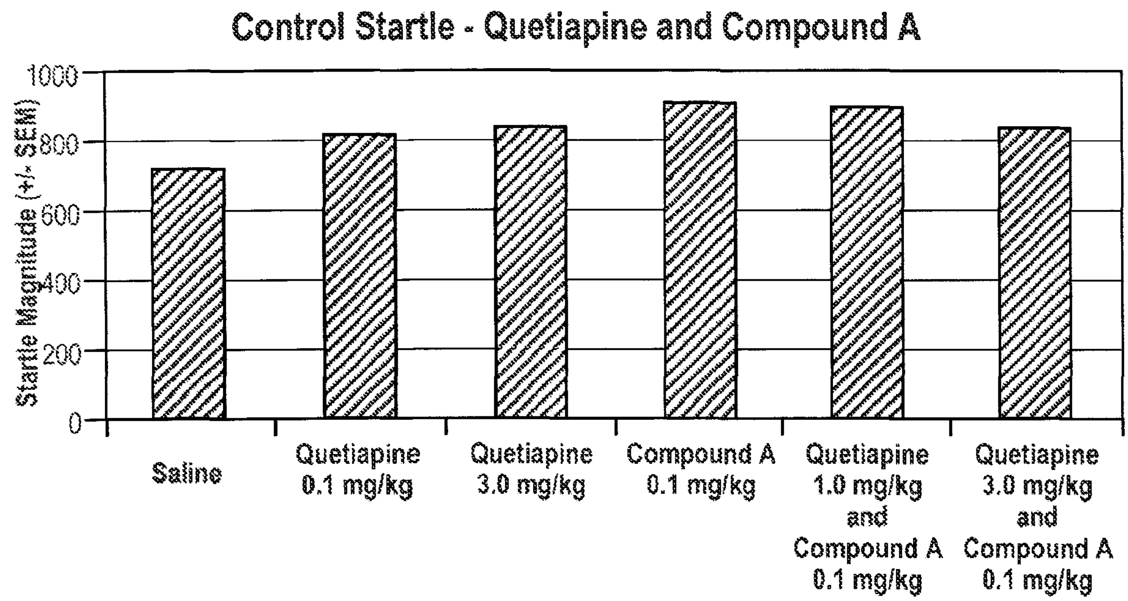
Control Startle - Quetiapine and Compound A
Startle Magnitude (+/- SEM)
1000
800
600
400
200
0
Saline
Quetiapine 0.1 mg/kg
Quetiapine 3.0 mg/kg
Compound A 0.1 mg/kg
Quetiapine 1.0 mg/kg and Compound A 0.1 mg/kg
Quetiapine 3.0 mg/kg and Compound A 0.1 mg/kg

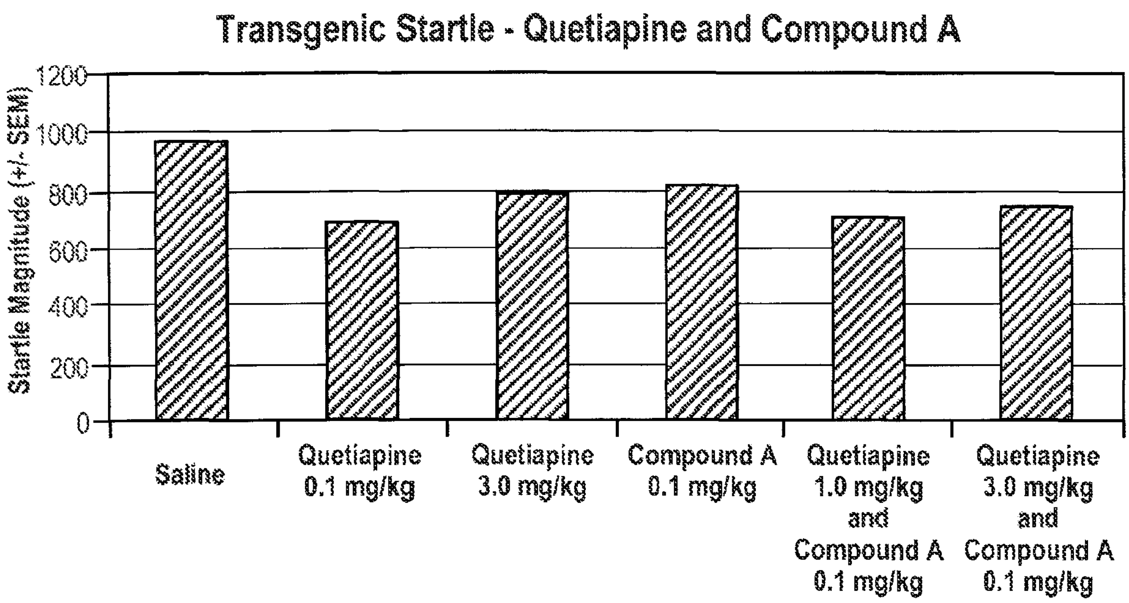
Transgenic Startle - Quetiapine and Compound A
Startle Magnitude (+/- SEM)
1200
1000
800
600
400
200
0
Saline
Quetiapine 0.1 mg/kg
Quetiapine 3.0 mg/kg
Compound A 0.1 mg/kg
Quetiapine 1.0 mg/kg and Compound A 0.1 mg/kg
Quetiapine 3.0 mg/kg and Compound A 0.1 mg/kg

*Fig. 8b*

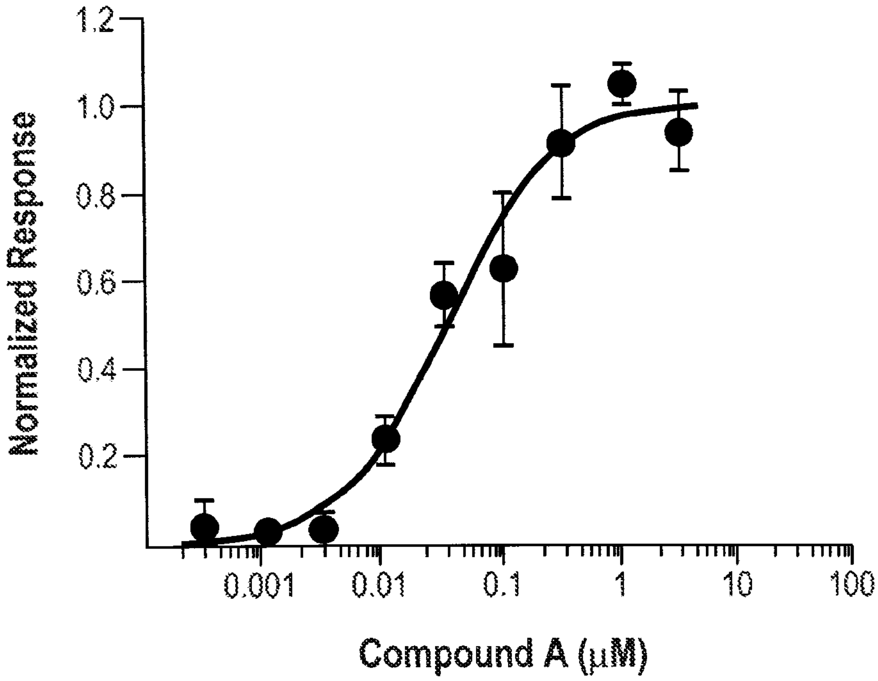
1.2
1.0
0.8
0.6
0.4
0.2
Normalized Response
0.001
0.01
0.1
1
10
100
Compound A (μM)

Fig. 9A

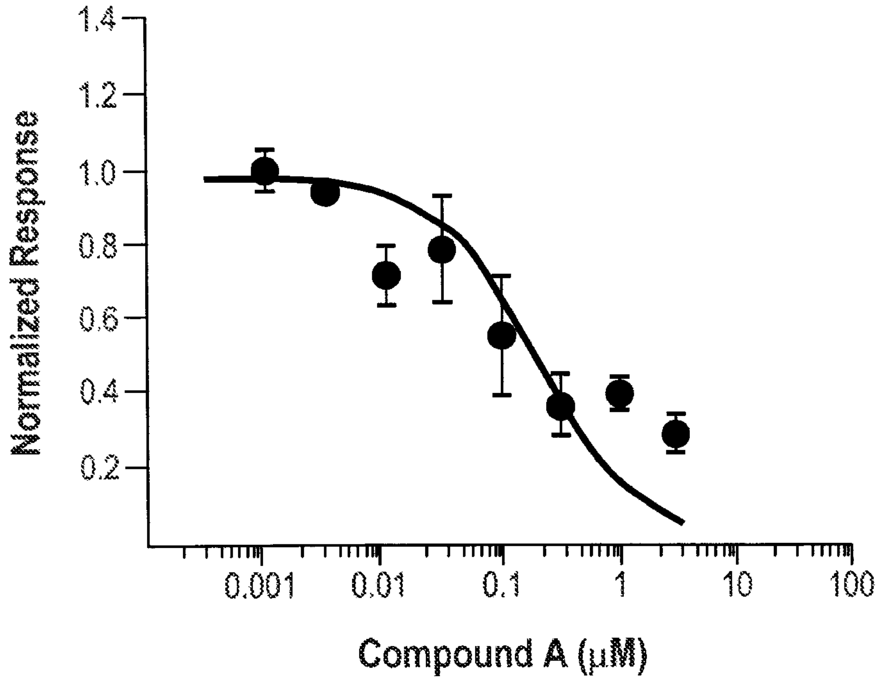
1.4
1.2
1.0
0.8
0.6
0.4
0.2
Normalized Response
0.001
0.01
0.1
1
10
100
Compound A (μM)

Fig. 9B

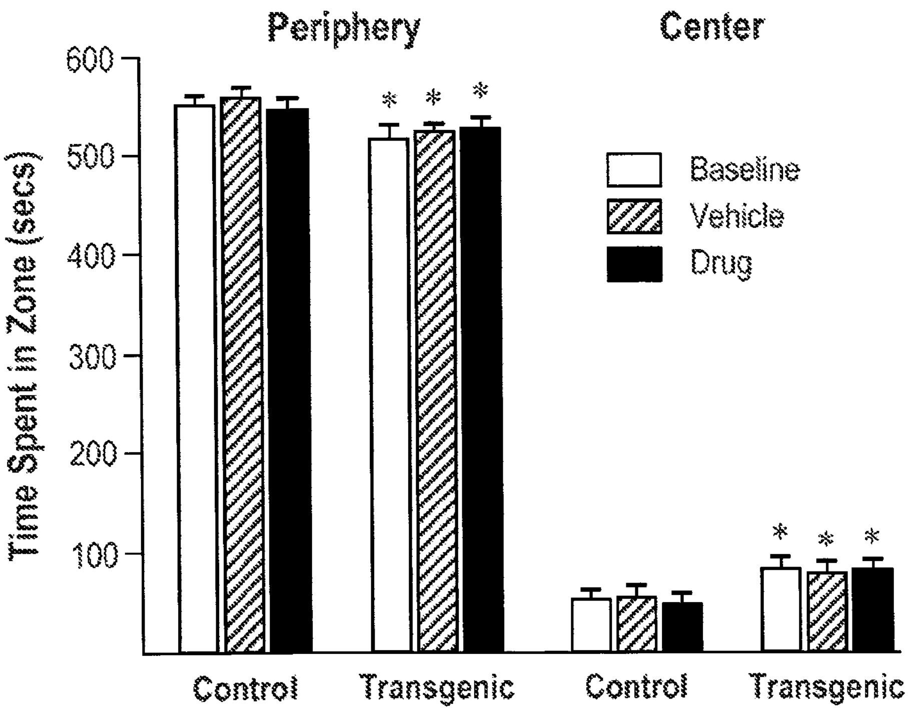


Fig. 10a

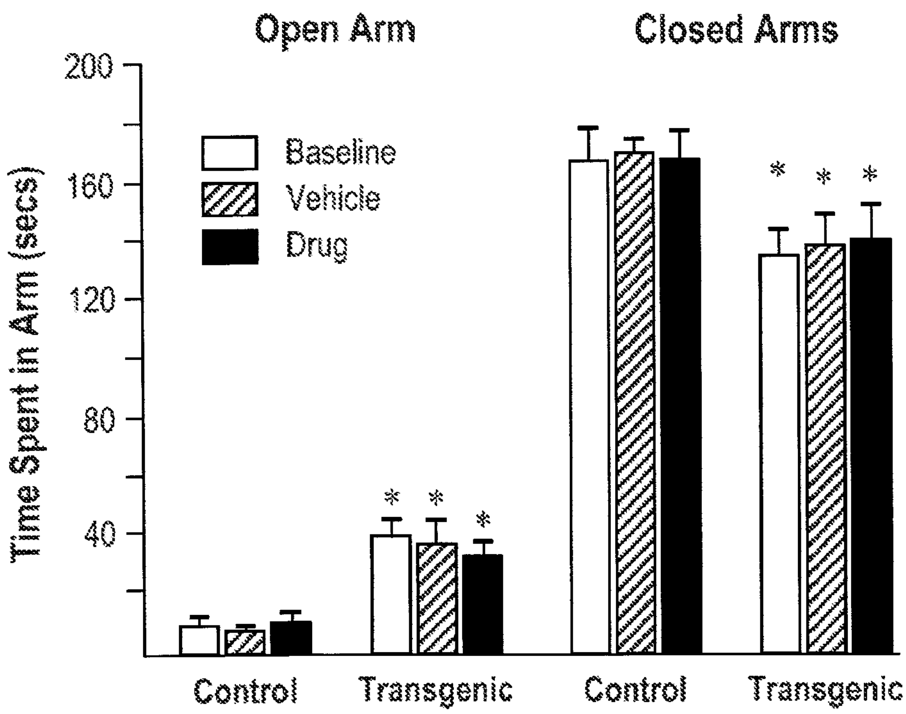


Fig. 10b

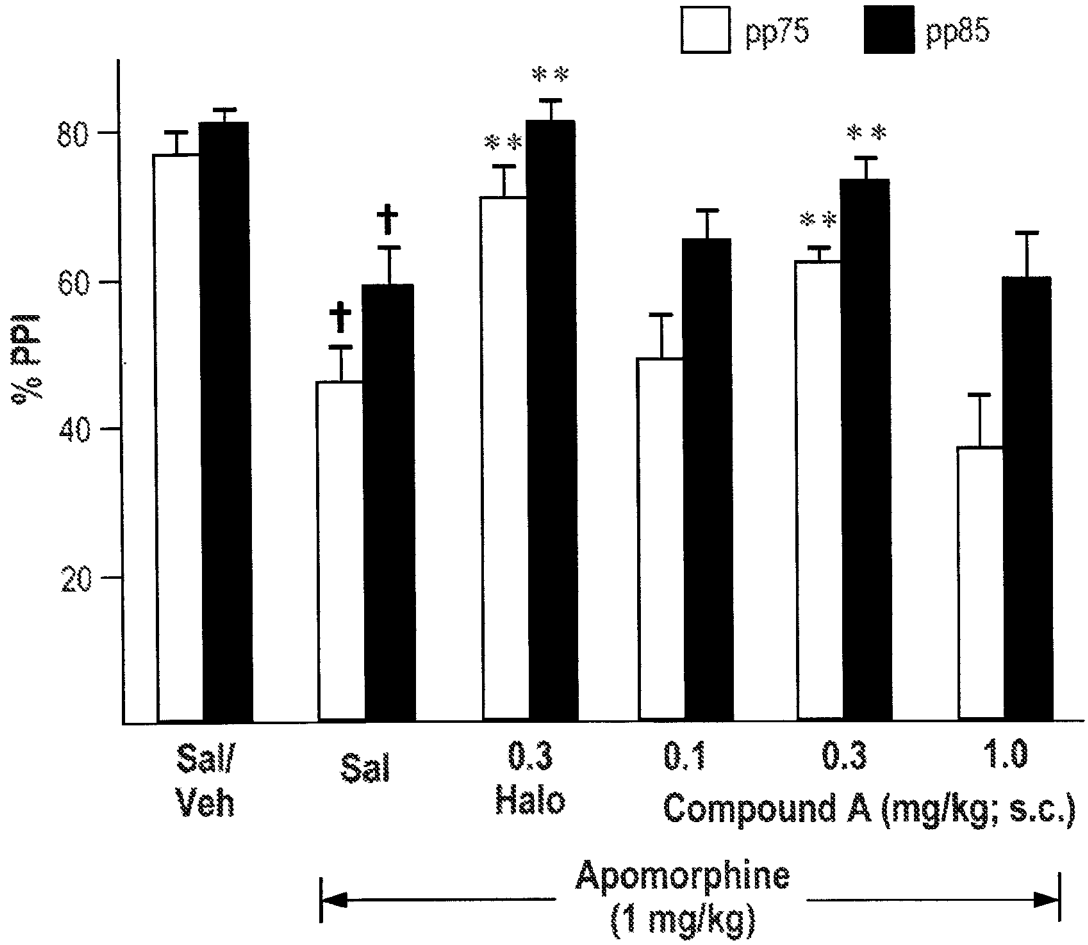
pp75
pp85
% PPI
80
60
40
20
* *
* *
* *
* *
†
†
Sal/
Veh
Sal
0.3
Halo
0.1
0.3
1.0
Compound A (mg/kg; s.c.)
Apomorphine
(1 mg/kg)

*Fig. 11*

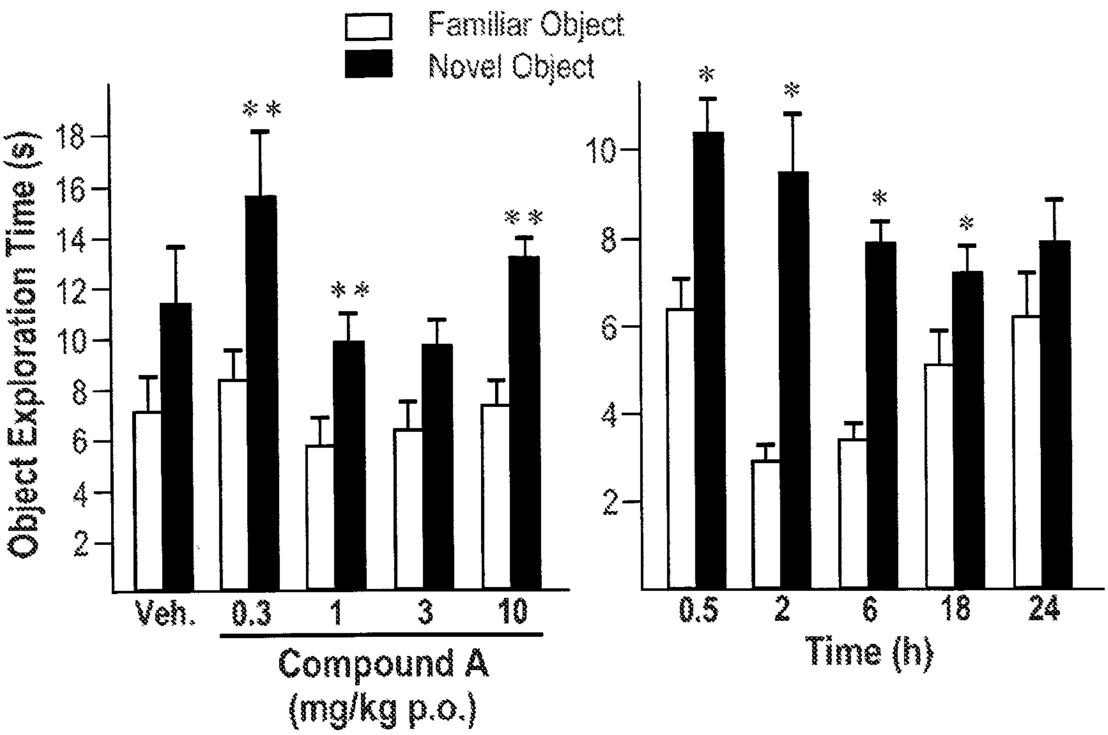


*Fig. 12a*

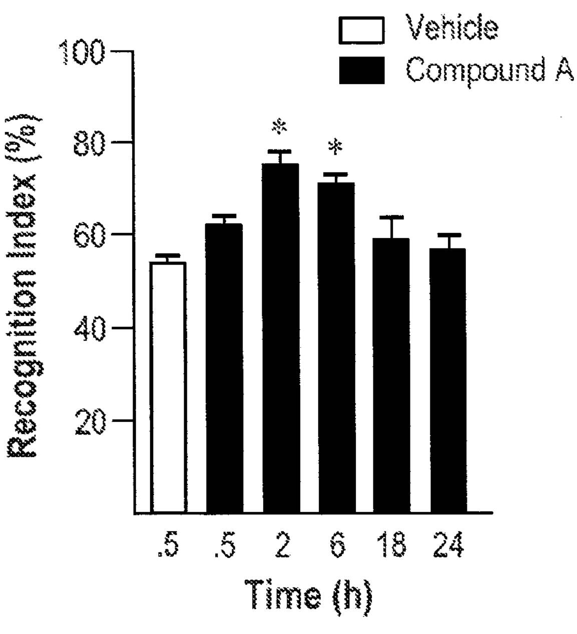


*Fig. 12b*

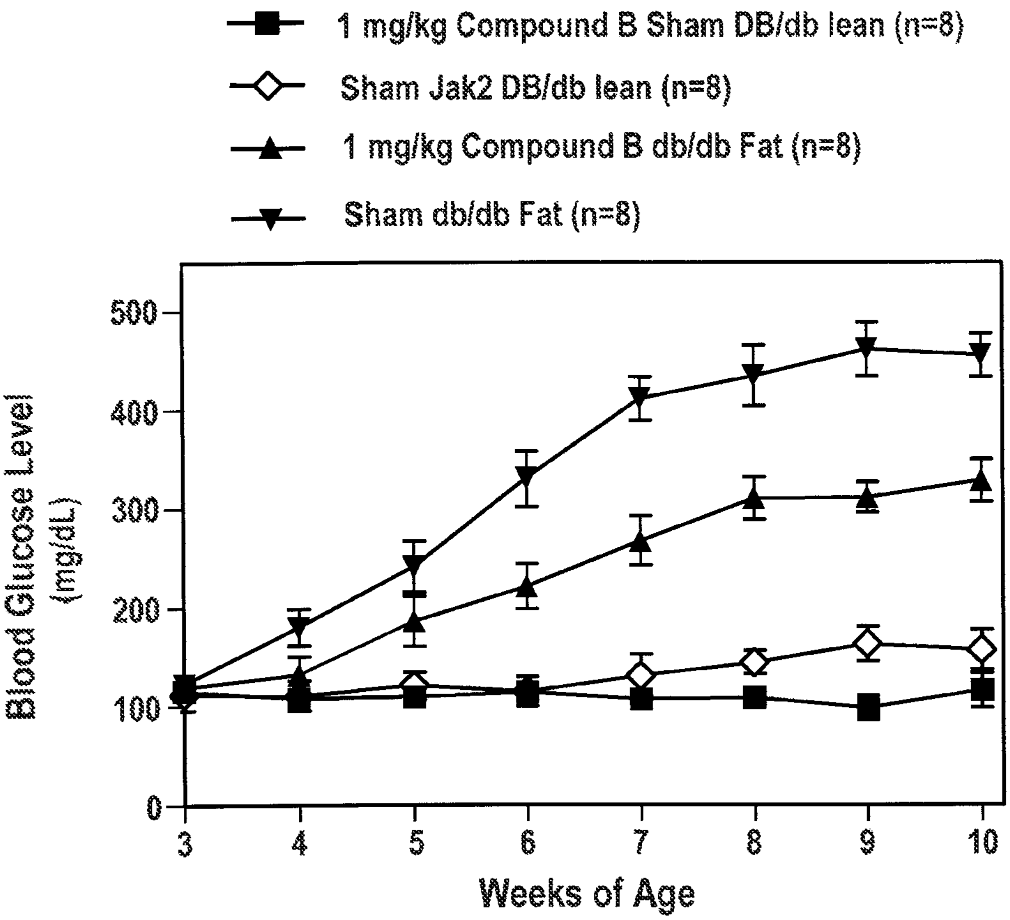
1 mg/kg Compound B Sham DB/db lean (n=8)
Sham Jak2 DB/db lean (n=8)
1 mg/kg Compound B db/db Fat (n=8)
Sham db/db Fat (n=8)
Blood Glucose Level (mg/dL)
500
400
300
200
100
0
3
4
5
6
7
8
9
10
Weeks of Age

*Fig. 13*

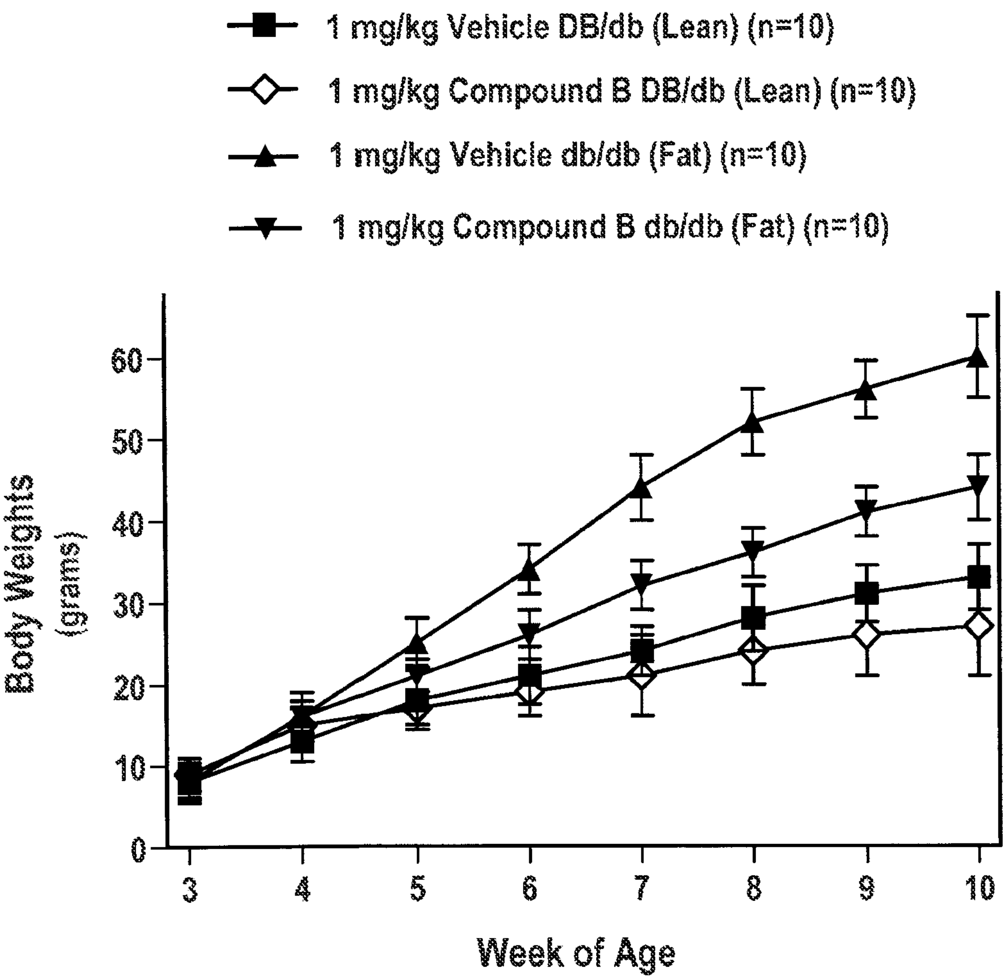
1 mg/kg Vehicle DB/db (Lean) (n=10)
1 mg/kg Compound B DB/db (Lean) (n=10)
1 mg/kg Vehicle db/db (Fat) (n=10)
1 mg/kg Compound B db/db (Fat) (n=10)
Body Weights (grams)
0
10
20
30
40
50
60
3
4
5
6
7
8
9
10
Week of Age

*Fig. 14*

Europäisches Patentamt
European Patent Office
Office européen des brevets

# EUROPEAN SEARCH REPORT

Application Number
EP 13 16 7089

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br>Y | WO 2004/099202 A1 (UPJOHN CO [US]; SELBO JOHN GORDON [US]; HAWLEY MICHAEL [US]; JIN QINGW) 18 November 2004 (2004-11-18)<br>* page 13, line 9 - page 14, line 24 *<br>----- | 1,2,6-14<br><br><br>3-5 | INV.<br>A61K45/06 |
| X<br><br><br>Y | WO 2004/052894 A1 (UPJOHN CO [US]; SELBO JON GORDON [US]; HEWITT BRADLEY DEE [US]; RAPPAT) 24 June 2004 (2004-06-24)<br>* page 21, line 7 - page 22, line 32 *<br>----- | 1,2,6-14<br><br><br>3-5 | |
| X<br><br><br>Y | WO 03/070731 A2 (UPJOHN CO [US]; WALKER DANIEL P [US]; PIOTROWSKI DAVID W [US]; JACOBSE) 28 August 2003 (2003-08-28)<br>* page 43, line 15 - page 44, line 33 *<br>----- | 1,2,6-14<br><br><br>3-5 | |
| X<br><br>Y | US 7 309 699 B2 (JI JIANGUO [US] ET AL) 18 December 2007 (2007-12-18)<br>* column 32, lines 19-27 *<br>----- | 1,2,6-14<br><br>3-5 | |
| Y,D | US 6 953 855 B2 (MAZUROV ANATOLY A [US] ET AL) 11 October 2005 (2005-10-11)<br>* the whole document *<br>----- | 3-5 | TECHNICAL FIELDS SEARCHED (IPC)<br>A61K |

The present search report has been drawn up for all claims

3

| Place of search | Date of completion of the search | Examiner |
|---|---|---|
| Munich | 31 July 2013 | Engl, Brigitte |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 16 7089

This annex lists the patent family members relating to the patent documents cited in the above-mentioned European search report.
The members are as contained in the European Patent Office EDP file on
The European Patent Office is in no way liable for these particulars which are merely given for the purpose of information.

31-07-2013

| Patent document cited in search report | Publication date | Patent family member(s) | Publication date |
|---|---|---|---|
| WO 2004099202 A1 | 18-11-2004 | US 2005059698 A1 | 17-03-2005 |
| | | WO 2004099202 A1 | 18-11-2004 |
| WO 2004052894 A1 | 24-06-2004 | AR 042295 A1 | 15-06-2005 |
| | | AU 2003302911 A1 | 30-06-2004 |
| | | BR 0317019 A | 25-10-2005 |
| | | CA 2506529 A1 | 24-06-2004 |
| | | CN 1720248 A | 11-01-2006 |
| | | CR 7859 A | 08-07-2005 |
| | | EC SP055834 A | 11-08-2005 |
| | | EP 1572700 A1 | 14-09-2005 |
| | | HR P20050494 A2 | 31-10-2005 |
| | | IS 7844 A | 12-05-2005 |
| | | JP 2006510664 A | 30-03-2006 |
| | | KR 20050087826 A | 31-08-2005 |
| | | MA 27604 A1 | 01-11-2005 |
| | | MX PA05005943 A | 18-08-2005 |
| | | OA 12968 A | 13-10-2006 |
| | | US 2005165047 A1 | 28-07-2005 |
| | | WO 2004052894 A1 | 24-06-2004 |
| | | ZA 200503988 A | 27-09-2006 |
| WO 03070731 A2 | 28-08-2003 | AU 2003217275 A1 | 09-09-2003 |
| | | BR 0307782 A | 04-01-2005 |
| | | CA 2476624 A1 | 28-08-2003 |
| | | EP 1476448 A2 | 17-11-2004 |
| | | JP 2005523287 A | 04-08-2005 |
| | | MX PA04008152 A | 08-09-2005 |
| | | US 2003232853 A1 | 18-12-2003 |
| | | US 2004224976 A1 | 11-11-2004 |
| | | WO 03070731 A2 | 28-08-2003 |
| US 7309699 B2 | 18-12-2007 | US 2005159597 A1 | 21-07-2005 |
| | | US 2007275975 A1 | 29-11-2007 |
| US 6953855 B2 | 11-10-2005 | AT 381563 T | 15-01-2008 |
| | | AU 2004215386 A1 | 10-09-2004 |
| | | BR PI0407708 A | 14-02-2006 |
| | | CA 2514135 A1 | 10-09-2004 |
| | | CN 1751041 A | 22-03-2006 |
| | | CN 101220026 A | 16-07-2008 |
| | | DE 602004010787 T2 | 24-12-2008 |
| | | DK 1594869 T3 | 07-04-2008 |
| | | EP 1594869 A2 | 16-11-2005 |
| | | ES 2300749 T3 | 16-06-2008 |
| | | HK 1083627 A1 | 13-06-2008 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 13 16 7089

This annex lists the patent family members relating to the patent documents cited in the above-mentioned European search report.
The members are as contained in the European Patent Office EDP file on
The European Patent Office is in no way liable for these particulars which are merely given for the purpose of information.

31-07-2013

| Patent document cited in search report | Publication date | Patent family member(s) | Publication date |
|---|---|---|---|
| | | IL 169797 A | 30-04-2012 |
| | | IL 208461 A | 28-06-2012 |
| | | IL 208462 A | 28-06-2012 |
| | | JP 4828407 B2 | 30-11-2011 |
| | | JP 5211183 B2 | 12-06-2013 |
| | | JP 2006518746 A | 17-08-2006 |
| | | JP 2011137009 A | 14-07-2011 |
| | | JP 2011137010 A | 14-07-2011 |
| | | KR 20060002770 A | 09-01-2006 |
| | | KR 20100130239 A | 10-12-2010 |
| | | KR 20100130240 A | 10-12-2010 |
| | | MX PA05008926 A | 05-10-2005 |
| | | NO 332757 B1 | 07-01-2013 |
| | | NO 332759 B1 | 07-01-2013 |
| | | NO 332760 B1 | 07-01-2013 |
| | | NZ 541794 A | 28-03-2008 |
| | | NZ 565177 A | 26-09-2008 |
| | | NZ 565178 A | 31-05-2009 |
| | | PT 1594869 E | 14-01-2008 |
| | | US 2004002513 A1 | 01-01-2004 |
| | | US 2005255040 A1 | 17-11-2005 |
| | | US 2006247270 A1 | 02-11-2006 |
| | | US 2008138287 A1 | 12-06-2008 |
| | | US 2010152232 A1 | 17-06-2010 |
| | | US 2010152233 A1 | 17-06-2010 |
| | | US 2010240696 A1 | 23-09-2010 |
| | | US 2011071187 A1 | 24-03-2011 |
| | | US 2011071188 A1 | 24-03-2011 |
| | | US 2011071189 A1 | 24-03-2011 |
| | | US 2012136024 A1 | 31-05-2012 |
| | | WO 2004076449 A2 | 10-09-2004 |
| | | ZA 200506515 A | 28-06-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9962505 A
- WO 9903859 A
- WO 9730998 A
- WO 0136417 A
- WO 0215662 A
- WO 0216355 A
- WO 0216356 A
- WO 0216357 A
- WO 0216358 A
- WO 0217358 A
- US 5712270 A, Sabb
- WO 0200652 A
- WO 02051841 A
- US 6953855 B
- US 157119 A
- US 11458231 A
- US 60971654 A
- US 5597919 A, Dull
- US 5616716 A, Dull
- US 5663356 A, Ruecroft
- US 11157119 B
- US 11458231 B
- US 60971654 B


### Non-patent literature cited in the description


- **SCHMITT.** *Current Med. Chem.,* 2000, vol. 7, 749
- **MAZUROV et al.** *Curr. Med. Chem.,* 2006, vol. 13, 1567-1584
- **STEVENS et al.** *Psychopharm,* 1998, vol. 136, 320
- **DOLLE et al.** *J. Labelled Comp. Radiopharm.,* 2001, vol. 44, 785
- **MACOR et al.** *Bioorg. Med. Chem. Lett.,* 2001, vol. 11, 319
- **CAULFIELD.** *Pharmacol. Ther.,* 1993, vol. 58, 319
- **BROADLEY ; KELLY.** *Molecules,* 2001, vol. 6, 142
- **LEONARD et al.** *Schizophrenia Bulletin,* 1996, vol. 22 (3), 431
- **FREEDMAN et al.** *Biological Psychiatry,* 1995, vol. 38 (1), 22
- American Psychiatric Association: Diagnostic and Statistical Manual of Mental Disorders. American Psychiatric Association, 2000
- **WONG AHC ; VAN TOL HHM.** Schizophrenia: from phenomenology to neurobiology. *Neurosci. Biobehav. Rev.,* 2003, vol. 27, 269-306
- **S, ADLER LE ; BENHAMMOU K et al.** Smoking and mental illness. *Pharmacol. Biochem. Behav.,* 2001, vol. 70, 561-570
- **FREEDMAN F ; HALL M ; ADLER LE ; LEONARD S.** Evidence in postmortem brain tissue for decreased numbers of hippocampal nicotinic receptors in schizophrenia. *Biol. Psychiatry,* 1995, vol. 38, 22-33
- **GUAN ZZ ; ZHANG X ; BLENNW K et al.** Decreased protein level of nicotinic acetylcholine receptor $\alpha7$ subunit in the frontal cortex from schizophrenic brain. *NeuroReport,* 1999, vol. 10, 1779-1782
- **BREESE CR ; ADAMS C ; LOGEL J et al.** Comparison of the regional expression of nicotinic acetylcholine receptor alpha7 mRNA and [1251]-alpha-bungarotoxin binding in human postmortem brain. *J. Comp. Neurol.,* 1997, vol. 387, 385-398
- **MANSVELDER,HD ; MCGEHEE DS.** Long-term potentiation of excitatory inputs to brain reward areas by nicotine. *Neuron,* 2000, vol. 27, 349-357
- **EGEA J ; ROSA AO ; SOBRADO M ; GANDIA L ; LOPEZ MG ; GARCIA AG.** Neuroprotection afforded by nicotine against oxygen and glucose deprivation in hippocampal slices is lost in alpha7 nicotinic receptor knockout mice. *Neuroscience,* 2007, vol. 145, 866-872
- **LEONARD S ; FREEDMAN R.** Genetics of Chromosome 15q13-q14 in Schizophrenia. *Biol. Psychiatry,* 2006, vol. 60, 115-122
- **WALKER E ; KESTLER L ; BOLLINI A ; HOCHMAN KM.** Schizophrenia: Etiology and course. *Annu. Rev. Psychol.,* 2004, vol. 55, 401-430
- **FENTON WS ; STOVER EL ; INSEL TR.** Breaking the log-jam in treatment development for cognition in schizophrenia: NIMH perspective. *Psychopharmacology,* 2003, vol. 169, 365-366
- **MEYER EM ; DE FIEBRE DM ; HUNTER BE ; SIMPKINS CE ; FRAUWORTH N ; DE FIEBRE NE.** Effects of anabaseine-related analogs on rat brain nicotinic receptor binding and on avoidance behaviors. *Drug Dev. Res.,* 1994, vol. 31, 127-134

- **ARENDASH GW ; SENGSTOCK GJ ; SANBERG PR ; KEM WR.** Improved learning and memory in aged rats with chronic administration of nicotinic receptor agonist GTS-21. *Brain Res.,* 1995, vol. 674, 252-259
- **KAZMI A ; KEM WR.** Nicotinic cholinergic system involvement in eyeblink classical conditioning in rabbits. *Behav. Neurosci.,* 1994, vol. 108, 486-493
- **BRIGGS CA ; ANDERSON DJ ; BRIONI JD et al.** Functional characterization of a novel neuronal nicotinic acetylcholine receptor ligand GTS-21 in vitro and in vivo. *Pharmacol. Biochem. Behav.,* 1997, vol. 57, 231-241
- **SIMOSKY JK ; STEVENS KE ; KEM WR ; FREEDMAN R.** Intragastric DMXB-A, an alpha7 nicotinic agonist, improves deficient sensory inhibition in DBA/2 mice. *Biol. Psychiatry,* 2001, vol. 50, 493-500
- **HURST RS ; HAJÓS M ; RAGGENBASS M et al.** A novel positive allosteric modulator of the $\alpha7$ neuronal nicotinic acetylcholine receptor: in vitro and in vivo characterization. *J. Neurosci.,* 2005, vol. 25, 4396-4405
- **HAJÓS M ; HURST RS ; HOFFMANN WE et al.** The selective $\alpha7$ nicotinic acetylcholine receptor agonist PNU-282987 [N-[(3R)-1-azabicyclo[2.2.2]oct-3-yl]-4-chlorobenzamide hydrochloride] enhances GABAergic synaptic activity in brain slices and restores auditory gating deficits in anesthetized rats. *JPET,* 2005, vol. 312, 1213-1222
- **BETTANY JH ; LEVIN ED.** Ventral hippocampal alpha 7 nicotinic receptor blockade and chronic nicotine effects on memory performance in the radial-arm maze. *Pharmacol. Biochem. Behav.,* 2001, vol. 70, 467-474
- **ADDY NA ; NAKIJAMA A ; LEVIN ED.** Nicotinic mechanisms of memory: effects of acute local DhbetaE and MLA infusions in the basolateral amygdala. *Brain Res. Cogn. Brain Res.,* 2003, vol. 16, 51-57
- **ADLER LE ; OLINCY A ; WALDO MC et al.** Schizophrenia, sensory gating and nicotinic receptors. *Schizophr. Bull.,* 1998, vol. 24, 189-202
- **ROSS RG ; OLINCY A ; HARRIS JG et al.** Anticipatory saccades during smooth pursuit eye movements and familial transmission of schizophrenia. *Biol. Psychiatry,* 1998, vol. 44, 690-697
- **CILIA J ; CLUDERAY JE ; ROBBINS MJ et al.** Reversal of isolation-rearing-induced PPI deficits by an $\alpha7$ nicotinic receptor agonist. *Psychopharmacology,* 2005, vol. 182, 214-219
- **VAN KAMPEN M ; SELBACH K ; SCHNEIDER R ; SCHIEGEL E ; BOESS F ; SCHREIBER R.** AR-R 17779 improves social recognition in rats by activation of nicotinic $\alpha7$ receptors. *Psychopharmacology,* 2004, vol. 172, 375-383
- **LEVIN ED ; BETTEGOWDA C ; BLOSSER J ; GORDON J.** AR-R 17779, an $\alpha7$ nicotinic agonist, improves learning and memory in rats. *Behav. Pharmacol.,* 1999, vol. 10, 675-680
- **PICHAT P ; BERGIS OE ; TERRANOVA JP et al.** SSR180711A, a novel selective $\alpha7$ nicotinic receptor partial agonist. III. Effects in models predictive of therapeutic activity on cognitive symptoms of schizophrenia. *Soc. Neurosci. Abstr.,* 2004, vol. 34, 583
- **BERGIS OE ; PICHAT P ; SANTAMARIA R et al.** SSR180711A, a novel selective $\alpha7$ nicotinic receptor partial agonist. II. Effects in models predictive of therapeutic activity on cognitive symptoms of Alzheimer's disease. *Soc. Neurosci. Abstr.,* 2004, vol. 34, 583
- **OLINCY A ; HARRIS JG ; JOHNSON LL et al.** Proof-of-concept trial of an $\alpha7$ nicotinic agonist in schizophrenia. *Arch. Gen. Psychiatry,* 2006, vol. 63, 630-638
- **KLEJBOR et al.** *J Neurochem,* 2006, vol. 97 (5), 1243-58
- **VOLLENWEIDER F.X. et al.** *Biol Psychiat,* 2006, vol. 60, 597-603
- **KUMARI V ; SHARMA T.** *Psychopharmacology,* 2002, vol. 162, 97-101
- **DEAN B.** *Aus. NZ J. Psychiat,* 2000, vol. 34, 560-569
- **ABI-DARGHAM, A. et al.** SPECT imaging of dopamine transporters in human brain with iodine-123-fluoroalkyl analogs of beta-CIT. *J Nucl Med,* 1996, vol. 37 (7), 1129-33
- **ABI-DARGHAM, A. et al.** Striatal amphetamine-induced dopamine release in patients with schizotypal personality disorder studied with single photon emission computed tomography and [1231]iodobenzamide. *Biol Psychiatry,* 2004, vol. 55 (10), 1001-6
- **ACKER, B.A. et al.** Discovery of N-[(3R,5R)-1-azabicyclo[3.2.1]oct-3-yl]furo[2,3-c]pyridine-5-carboxamide as an agonist of the alpha7 nicotinic acetylcholine receptor: in vitro and in vivo activity. *Bioorg Med Chem Lett,* 2008, vol. 18, 3611-3615
- **ADLER L. et al.** Schizophrenia, sensory gating, and nicotinic receptors. *Schizophr Bull,* 1998, vol. 24, 189-202
- **AMERICAN DIABETES ASSOCIATION et al.** Consensus development conference on antipsychotic drugs and obesity and diabetes. *Diabetes Care,* 2004, vol. 27, 596-601
- **AMAIZ-COT JJ et al.** Allosteric modulation of alpha 7 nicotinic receptors selectively depolarizes hippocampal interneurons, enhancing spontaneous GABAergic transmission. *Eur J Neurosci.,* 2008, vol. 27, 1097-1110
- **ASTLES PC et al.** Recent progress in the development of subtype selective nicotinic acetylcholine receptor ligands. *Curr Drug Targets CNS Neurol Disord,* 2002, vol. 1, 337-348

- **AVILA MT et al.** Effects of Nicotine on Leading Saccades during Smooth Pursuit Eye Movements in Smokers and Nonsmokers with Schizophrenia. *Neuropsychopharmacol,* 2003, vol. 28, 2184-2191
- **BANERJEE, S. A. et al.** 5' flanking sequences of the rat tyrosine hydroxylase gene target accurate tissue-specific, developmental, and transsynaptic expression in transgenic mice. *J Neurosci,* 2003, vol. 12 (11), 4460-7
- **BARNES TRE et al.** Pharmacological strategies for relapse prevention in schizophrenia. *Psychiatry,* 2007, vol. 6, 351-356
- **BECKSTEAD, R. M. et al.** Efferent connections of the substantia nigra and ventral tegmental area in the rat. *Brain Res,* 1979, vol. 175 (2), 191-217
- **BEHRENDT, RP.** Dysregulation of thalamic sensory 'transmission' in schizophrenia: neurochemical vulnerability to hallucinations. *J Psychopharmacol,* 2006, vol. 20, 356-72
- **BELLUARDO N et al.** Neurotrophic effects of central nicotinic receptor activation. *J Neural Transm Suppl,* 2000, vol. 60, 227-245
- **BIALOWAS, J. et al.** The relationship between catecholamine levels in the hypothalamus and amygdala under influence of glucose overloading in hungry and sated rats. *Pol J Pharmacol Pharm,* 1979, vol. 31 (4), 325-35
- **BITNER RS et al.** Broad-spectrum efficacy across cognitive domains by alpha7 nicotinic acetylcholine receptor agonism correlates with activation of ERK1/2 and CREB phosphorylation pathways. *J Neurosci,* 2007, vol. 27, 10578-10587
- **BLUMENTHAL EM et al.** Detection of Functional Nicotinic Receptors Blocked by □-Bungarotoxin on PC12 Cells and Dependence of Their Expression on Post-Translational Events. *J Neurosci,* 1997, vol. 17, 6094-6104
- **BOESS FG et al.** The novel alpha7 nicotinic acetylcholine receptor agonist N-[(3R)-1-azabicyclo[2.2.2]oct-3-yl]-7-[2-(methoxy)phenyl]-1-benzofuran-2-carboxamide improves working and recognition memory in rodents. *J Pharmacol Exp Ther,* 2007, vol. 321, 716-725
- **BOGERTS, B. et al.** A morphometric study of the dopamine-containing cell groups in the mesencephalon of normals, Parkinson patients, and schizophrenics. *Biol Psychiatry,* 1983, vol. 18 (9), 951-69
- **BRAFF DL et al.** Sensorimotor gating and schizophrenia: human and animal model studies. *Arch Gen Psych,* 1990, vol. 47, 181-188
- **BRODERICK, P.A. et al.** I. Serotonin (5-HT) within dopamine reward circuits signals open-field behavior. II. Basis for 5-HT--DA interaction in cocaine dysfunctional behavior. *Neurosci Biobehav Rev,* 1997, vol. 21 (3), 227-60
- **BUNNEY, E. B. et al.** Electrophysiological effects of cocaethylene, cocaine, and ethanol on dopaminergic neurons of the ventral tegmental area. *J Pharmacol Exp Ther,* 2001, vol. 297 (2), 696-703
- **CABIB, S. et al.** Behavioral and mesocorticolimbic dopamine responses to non aggressive social interactions depend on previous social experiences and on the opponent's sex. *Behav Brain Res,* 2000, vol. 112 (1-2), 13-22
- **CANITANO, R.** Clinical experience with Topiramate to counteract neuroleptic induced weight gain in 10 individuals with autistic spectrum disorders. *Brain Dev,* 2005, vol. 27, 228-232
- **CARLSSON, A.** Does dopamine play a role in schizophrenia?. *Psychol Med,* 1977, vol. 7 (4), 58397
- **CHENG Y et al.** Relationship between inhibition constant (Ki) and concentration of inhibitor which causes 50 per cent inhibition (I50) of an enzymatic-reaction. *Biochem Pharmacol,* 1973, vol. 22, 3099-3108
- **CORBETT R et al.** Antipsychotic agents antagonize non-competitive N-methyl-D-aspartate antagonist-induced behaviors. *Psychopharmacol,* 1995, vol. 120, 67-74
- **CORSO, T. D. et al.** Transfection of tyrosine kinase deleted FGF receptor-1 into rat brain substantia nigra reduces the number of tyrosine hydroxylase expressing neurons and decreases concentration levels of striatal dopamine. *Brain Res Mol Brain Res,* 2005, vol. 139 (2), 361-6
- **DALACK GW et al.** Nicotine dependence in schizophrenia: clinical phenomena and laboratory findings. *Am J Psych,* 1998, vol. 155, 1490-1501
- **DANI JA et al.** Nicotinic acetylcholine receptors and nicotinic cholinergic mechanisms of the central nervous system. *Annu Rev Pharmacol Toxicol,* 2007, vol. 47, 699-729
- **DAVIES AR et al.** Characterisation of the binding of [3H]methyllycaconitine: a new radioligand for labelling alpha 7-type neuronal nicotinic acetylcholine receptor. *Neuropharmacol,* 1999, vol. 38, 679-690
- **DAVIS, K. L. et al.** Dopamine in schizophrenia: a review and reconceptualization. *Am J Psychiatry,* 1991, vol. 148 (11), 1474-86
- **DE LUCA V et al.** Evidence of Association between Smoking and $\alpha$7 Nicotinic Receptor Subunit Gene in Schizophrenia Patients. *Neuropsychopharmacol,* 2004, vol. 29, 1522-1526
- **DICKINSON JA et al.** Presynaptic alpha 7- and beta 2-containing nicotinic acetylcholine receptors modulate excitatory amino acid release from rat prefrontal cortex nerve terminals via distinct cellular mechanisms. *Mol Pharmacol,* 2008, vol. 74, 348-359

- **DOHERTY, M. D. et al.** Ultrastructural localization of the serotonin 2A receptor in dopaminergic neurons in the ventral tegmental area. *Brain Res,* 2000, vol. 864 (2), 176-85
- **DURANY N et al.** Human post-mortem striatal alpha4beta2 nicotinic acetylcholine receptor density in schizophrenia and Parkinson's syndrome. *Neurosci Lett,* 2000, vol. 287, 109-112
- **ENNACEUR A et al.** A new one-trial test for neurobiological studies of memory in rats. I. Behavioral data. *Behav Brain Res,* 1988, vol. 100, 85-92
- **FANG, X. et al.** Control of CREB-binding protein signaling by nuclear fibroblast growth factor receptor-1: a novel mechanism of gene regulation. *J Biol Chem,* 2005, vol. 280 (31), 28451-62
- **FARDE, L. et al.** Quantitative analysis of D2 dopamine receptor binding in the living human brain by PET. *Science,* 1986, vol. 231, 258-261
- **FREEDMAN R et al.** The alpha7-nicotinic acetylcholine receptor and the pathology of hippocampal interneurons in schizophrenia. *J Chem Neuroanat,* 2000, vol. 20, 299-306
- **FREEDMAN R et al.** Initial phase 2 trial of a nicotinic agonist in schizophrenia. *Am J Psych,* 2008, vol. 165, 1040-1047
- **GAUGHRAN, F. et al.** Hippocampal FGF-2 and FGFR1 mRNA expression in major depression, schizophrenia and bipolar disorder. *Brain Res Bull,* 2006, vol. 70 (3), 221-7
- **GEYER MA et al.** Measurement of startle response, prepulse inhibition and habituation. *Current Protocols Neurosci,* 1998, vol. 8, 7.1-7.15
- **GRAHAM, KA et al.** Double-blind, placebo-controlled investigation of amantadine for weight loss in subjects who gained weight with olanzapine. *Am J Psychiatry,* 2005, vol. 162, 1744-1746
- **HAJÓS M et al.** The selective alpha7 nicotinic acetylcholine receptor agonist PNU-282987 [N-[(3R)-1-Azabicyclo[2.2.2]oct-3-yl]-4-chlorobenzamide hydrochloride] enhances GABAergic synaptic activity in brain slices and restores auditory gating deficits in anesthetized rats. *J Pharmacol Exp Ther,* 2005, vol. 312, 1213-1222
- **HARRIS JG et al.** Effects of nicotine on cognitive deficits in schizophrenia. *Neuropsychophannacol,* 2004, vol. 29, 1378-1385
- **HARRISON, P. J.** The neuropathology of schizophrenia. A critical review of the data and their interpretation. *Brain,* 1999, vol. 122, 593-624
- **HASHIMOTO K et al.** $\alpha$7 Nicotinic Receptor Agonists as Potential Therapeutic Drugs for Schizophrenia. *Curr Med Chem - Central Nervous System Agents,* 2005, vol. 5, 171-184
- **HASHIMOTO, R. et al.** Impact of the DISC1 Ser704Cys polymorphism on risk for major depression, brain morphology and ERK signaling. *Hum Mol Genet,* 2006, vol. 15 (20), 3024-33
- **HENDERSON, DC et al.** Glucose metabolism in patients with schizophrenia treated with atypical antipsychotic agents. *Arch Gen Psychiatry,* 2005, vol. 62, 19-28
- **HENDERSON, DC et al.** A double-blind, placebo-controlled trial of sibutramine for olanzapine-associated weight gain. *Am J Psychiatry,* 2005, vol. 162, 954-962
- **HIETALA, J. et al.** Depressive symptoms and presynaptic dopamine function in neuroleptic-naive schizophrenia. *Schizophr Res,* 1999, vol. 35 (1), 41-50
- **HIETALA, J. et al.** Presynaptic dopamine function in striatum of neuroleptic-naive schizophrenic patients. *Lancet,* 1995, vol. 346 (8983), 1130-1
- **HOLDEN C.** Deconstructing Schizophrenia: Large-scale family studies and new drug probes focus on cognitive deficits that may lie at the heart of the disease. *Science,* 2003, vol. 299, 333-335
- **HORBINSKI, C. et al.** Bone morphogenetic protein-7 stimulates initial dendritic growth in sympathetic neurons through an intracellular fibroblast growth factor signaling pathway. *J Neurochem,* 2002, vol. 80 (1), 54-63
- **HU, Z. et al.** Differentiation of the midbrain dopaminergic pathways during mouse development. *J Comp Neurol,* 2004, vol. 476 (3), 301-11
- **HURST RS et al.** A novel positive allosteric modulator of the alpha7 neuronal nicotinic acetylcholine receptor: in vitro and in vivo characterization. *J Neurosci,* 2005, vol. 25, 4396-4405
- **IKEMOTO, K. et al.** Human midbrain dopamine neurons express serotonin 2A receptor: an immunohistochemical demonstration. *Brain Res,* 2000, vol. 853 (2), 377-80
- **KASPER, S. et al.** Dopamine- and serotonin-receptors in schizophrenia: results of imaging-studies and implications for pharmacotherapy in schizophrenia. *EurArch Psychiatry Clin Neurosci,* 1999, vol. 249 (4), 83-9
- **KITAGAWA H et al.** Safety, pharmacokinetics, and effects on cognitive function of multiple doses of GTS-21 in healthy, male volunteers. *Neuropsychopharmacol,* 2003, vol. 28, 542-551
- **KLEIN, DJ et al.** A randomized, double-blind, placebo-controlled trial of metformin treatment of weight gain associated with initiation of atypical antipsychotic therapy in children and adolescents. *Am J Psychiatry,* 2006, vol. 163, 2072-2079
- **KLEJBOR, I. et al.** Fibroblast growth factor receptor signaling affects development and function of dopamine neurons - inhibition results in a schizophrenia-like syndrome in transgenic mice. *J Neurochem,* 2006, vol. 97 (5), 1243-58
- **KOLLER et al.** Olanzapine-associated diabetes mellitus. *Pharmacotherapy,* 2002, vol. 22, 841-852

- **KWON JS et al.** Gamma frequency-range abnormalities to auditory stimulation in schizophrenia. *Arch Gen Psych,* 1999, vol. 56, 1001-1005
- **LARUELLE, M. et al.** Single photon emission computerized tomography imaging of amphetamine-induced dopamine release in drug-free schizophrenic subjects. *Proc Natl Acad Sci U S A,* 1996, vol. 93 (17), 9235-40
- **LAUDER, J. M.** Neurotransmitters as growth regulatory signals: role of receptors and second messengers. *Trends Neurosci,* 1993, vol. 16 (6), 233-40
- **LENA C et al.** Role of Ca2+ ions in nicotinic facilitation of GABA release in mouse thalamus. *J Neurosci,* 1997, vol. 17, 576-585
- **LEONARD S et al.** Genetics of chromosome 15q13-q14 in schizophrenia. *Biol. Psych,* 2006, vol. 60, 115-122
- **LEONARD S et al.** Association of promoter variants in the alpha7 nicotinic acetylcholine receptor subunit gene with an inhibitory deficit found in schizophrenia. *Arch Gen Psych,* 2002, vol. 59, 1085-1096
- **LEVIN ED et al.** AR-R17779, and alpha7 nicotinic agonist, improves learning and memory in rats. *Behav Pharmacol,* 1999, vol. 10, 675-680
- **LEVIN ED et al.** Nicotine-haloperidol interactions and cognitive performance in schizophrenics. *Neuropsychopharmacol,* 1996, vol. 15, 429-436
- **LIPPIELLO PM et al.** The binding of L-[3H]nicotine to a single class of high affinity sites in rat brain membranes. *Mol Pharmacol,* 1986, vol. 29, 448-454
- **LIU Q et al.** Dissecting the signaling pathway of nicotine-mediated neuroprotection in a mouse Alzheimer disease model. *FASEB J,* 2007, vol. 21, 61-73
- **LUDEWIG K et al.** Impaired sensorimotor gating in schizophrenia with deficit and with nondeficit syndrome. *Swiss Med Wkly,* 2002, vol. 132, 159-165
- **MANSVELDER HD et al.** Long-term potentiation of excitatory inputs to brain reward areas by nicotine. *Neuron,* 2000, vol. 27, 349-357
- **MARTIN L et al.** Alpha-7 nicotinic receptor agonists: potential new candidates for the treatment of schizophrenia. *Psychopharmacol,* 2004, vol. 174, 54-64
- **MELTZER, H. Y. et al.** Classification of typical and atypical antipsychotic drugs on the basis of dopamine D-1, D-2 and serotonin2 pKi values. *J Pharmacal Exp Ther,* 1989, vol. 251 (1), 238-46
- **MEYER-LINDENBERG, A. et al.** Reduced prefrontal activity predicts exaggerated striatal dopaminergic function in schizophrenia. *Nat Neurosci,* 2002, vol. 5 (3), 267-71
- **MILLAR, J. K. et al.** DISC1 and PDE4B are interacting genetic factors in schizophrenia that regulate cAMP signaling. *Science,* 2005, vol. 310 (5751), 1187-91
- **MOFFETT J et al.** Increased tyrosine phosphorylation and novel cis-acting element mediate activation of the fibroblast growth factor-2 (FGF-2) gene by nicotinic acetylcholine receptor. New mechanism for trans-synaptic regulation of cellular development and plasticity. *Brain Res Mol Brain Res,* 1998, vol. 55, 293-305
- **MUDO G et al.** Nicotinic receptor agonists as neuroprotective / neurotrophic drugs. *Prog Mol Mechanisms,* 2007, vol. 114, 135-147
- **MUDO G et al.** Acute intermittent nicotine treatment induces fibroblast growth factor-2 in the subventricular zone of the adult rat brain and enhances neuronal precursor cell proliferation. *Neurosci,* 2007, vol. 145, 470-483
- **MURPHY PC et al.** Brain-stem modulation of the response properties of cells in the cat's perigeniculate nucleus. *Vis Neurosci,* 1994, vol. 11, 781-791
- **NEWHOUSE PA et al.** Effects of nicotinic stimulation on cognitive performance. *Curr Opin Pharmacol,* 2004, vol. 4, 36-46
- **NILSSON L.K. et al.** Subchronic treatment with kynurenine and probenecid: effects on prepulse inhibition and firing of midbrain dopamine neurons. *J. Neural Trans.,* 2006, vol. 113, 557-571
- **OHTANI, N. et al.** Dopamine modulates cell cycle in the lateral ganglionic eminence. *J Neurosci,* 2003, vol. 23 (7), 2840-50
- **O'NEILL HC et al.** DMXB, an alpha7 nicotinic agonist, normalizes auditory gating in isolation-reared rats. *Psychopharmacol,* 2003, vol. 169, 332-339
- **OVALLE, S. et al.** Fibroblast growth factor-2 is selectively modulated in the rat brain by E-5842, a preferential sigma-1 receptor ligand and putative atypical antipsychotic. *Eur J Neurosci,* 2001, vol. 13 (5), 909-15
- **PAPKE RL et al.** Comparative pharmacology of rat and human alpha7 nAChR conducted with net charge analysis. *Br J Pharmacol,* 2002, vol. 137, 49-61
- **PETRONIS, A.** The origin of schizophrenia: genetic thesis, epigenetic antithesis, and resolving synthesis. *Biol Psychiatry,* 2004, vol. 55 (10), 965-70
- *SA,* 1996, vol. 93 (17), 9235-40
- **PICHAT P et al.** SSR180711, a novel selective alpha7 nicotinic receptor partial agonist: (II) efficacy in experimental models predictive of activity against cognitive symptoms of schizophrenia. *Neuropsychopharmacol,* 2007, vol. 32, 17-34
- **QUARTA D et al.** Drug discrimination and neurochemical studies in alpha7 null mutant mice: tests for the role of nicotinic alpha7 receptors in dopamine release. *Psychopharmacol,* 30 August 2008

- **QUIK M et al.** Localization of nicotinic receptor subunit mRNAs in monkey brain by in situ hybridization. *J Comp Neurol,* 2000, vol. 425, 58-69
- **SCHIMMEL, J. J. et al.** 4.5 kb of the rat tyrosine hydroxylase 5' flanking sequence directs tissue specific expression during development and contains consensus sites for multiple transcription factors. *Brain Res Mol Brain Res,* 1999, vol. 74 (12), 1-14
- **SCHREIBER R et al.** Effects of alpha 4/beta 2- and alpha 7-nicotine acetylcholine receptor agonists on prepulse inhibition of the acoustic startle response in rats and mice. *Psychopharmacol,* 2002, vol. 159, 248-257
- **SEMYAK, MJ.** Association of diabetes mellitus with use of atypical neuroleptics in the treatment of schizophrenia. *Am J Psychiatry,* 2002, vol. 159, 561-566
- **SHARMA, R. P.** Schizophrenia, epigenetics and ligand-activated nuclear receptors: a framework for chromatin therapeutics. *Schizophr Res,* 2005, vol. 72 (2-3), 79-90
- **SHARP FR et al.** Psychosis: pathological activation of limbic thalamocortical circuits by psychomimetics and schizophrenia?. *Trends Neurosci,* 2001, vol. 24, 330-334
- **SIMOSKY JK et al.** Clozapine improves deficient inhibitory auditory processing in DBA/2 mice, via a nicotinic cholinergic mechanism. *Psychopharmacol,* 2003, vol. 165, 386-396
- **SINGHAL SK et al.** Antipsychotic clozapine inhibits the function of alpha7-nicotinic acetylcholine receptors. *Neuropharmacol,* 2007, vol. 52, 387-394
- **SNYDER, S. H.** The dopamine hypothesis of schizophrenia: focus on the dopamine receptor. *Am J Psychiatry,* 1976, vol. 133 (2), 197-202
- **STACHOWIAK MK et al.** Integrative Nuclear Signaling in Cell Development-A Role for FGF Receptor-1. *DNA Cell Biol,* 2007, vol. 26, 811-826
- **STACHOWIAK, E. K. et al.** cAMP-induced differentiation of human neuronal progenitor cells is mediated by nuclear fibroblast growth factor receptor-1 (FGFR1. *J Neurochem,* 2003, vol. 84 (6), 1296-312
- **STACHOWIAK, M. K. et al.** Apparent sprouting of striatal serotonergic terminals after dopamine-depleting brain lesions in neonatal rats. *Brain Res,* 1984, vol. 291 (1), 164-7
- **STACHOWIAK, M. K. et al.** Integrative Nuclear Signaling in Cell Development-A Role for FGF Receptor-1. *DNA Cell Biol,* 2007, vol. 26 (12), 811-26
- Antipsychotic agents. **STAHL SM.** Essential Pharmacology: Neuroscientific Basis and Practical Applications. Cambridge University Press, 2002, 401-458
- **SWERDLOW, N. R. et al.** Using an animal model of deficient sensorimotor gating to study the pathophysiology and new treatments of schizophrenia. *Schizophr Bull,* 1998, vol. 24 (2), 285301
- **SWERDLOW, N. R. et al.** Assessing the validity of an animal model of deficient sensorimotor gating in schizophrenic patients. *Arch Gen Psychiatry,* 1994, vol. 51 (2), 139-54
- **TIETJE KR et al.** Preclinical characterization of A-582941: a novel alpha 7 neuronal nicotinic receptor agonist with broad spectrum cognition-enhancing properties. *CNS Neurosci Ther,* 2008, vol. 14, 65-82
- **TIMMERMANN DB et al.** An allosteric modulator of the alpha7 nicotinic acetylcholine receptor possessing cognition-enhancing properties in vivo. *J Pharmacol Exp Ther,* 2007, vol. 323, 294-307
- **VAN KAMPEN M et al.** AR-R 17779 improves social recognition in rats by activation of nicotinic alpha7 receptors. *Psychopharmacol,* 2004, vol. 172, 375-383
- **WALDO MC et al.** Auditory sensory gating, hippocampal volume, and catecholamine metabolism in schizophrenics and their siblings. *Schizophrenia Res,* 1994, vol. 12, 93-106
- **WONG AH et al.** Schizophrenia: from phenomenology to neurobiology. *Neurosci Biobehav Rev,* 2003, vol. 27, 269-306
- **WONG, A. H. et al.** Identification of candidate genes for psychosis in rat models, and possible association between schizophrenia and the 14-3-3eta gene. *Mol Psychiatry,* 2003, vol. 8 (2), 156-66
- **YANG, Y. K. et al.** Associated alterations of striatal dopamine D2/D3 receptor and transporter binding in drug-naive patients with schizophrenia: a dual-isotope SPECT study. *Am J Psychiatry,* 2004, vol. 161 (8), 1496-8